# EUROPEAN PATENT SPECIFICATION

(11) **EP 3 704 133 B1**
(45) Date of publication and mention of the grant of the patent: **01.10.2025**
(21) Application number: 18874977.4
(22) Date of filing: 08.06.2018
(51) Int. Cl.: C07K 1/13, C12N 15/10, C12Q 1/68, G01N 33/68

(54) **REAGENTS AND METHODS FOR ELEMENTAL IMAGING MASS SPECTROMETRY OF BIOLOGICAL SAMPLES**
REAGENZIEN UND VERFAHREN FÜR DIE ELEMENTARE BILDGEBENDE MASSENSPEKTROMETRIE VON BIOLOGISCHEN PROBEN
RÉACTIFS ET PROCÉDÉS DE SPECTROMÉTRIE DE MASSE POUR L'IMAGERIE ÉLÉMENTAIRE D'ÉCHANTILLONS BIOLOGIQUES

(30) Priority: 03.11.2017 US 201762581584 P; 06.11.2017 US 201762582280 P; 19.03.2018 US 201862644903 P
(43) Date of publication of application: 09.09.2020
(73) Proprietor: Standard Biotools Canada Inc., Markham, ON L3R 4G5 (CA)
(72) Inventor: CHANG, Qing, Markham, Ontario L3R 4G5 (CA); ALLO, Bedilu, Markham, Ontario L3R 4G5 (CA); LOU, Xudong, Markham, Ontario L3R 4G5 (CA); BOUZEKRI, Alexander, Markham, Ontario L3R 4G5 (CA); ORNATSKY, Olga, Markham, Ontario L3R 4G5 (CA)
(74) Representative: Carpmaels & Ransford LLP
(86) International application number: PCT/US2018/036622
(87) International publication number: WO 2019/089088

(56) References cited:
- WO-A1-2007/137418
- WO-A1-2011/089393
- WO-A1-2017/004203
- WO-A2-2011/038158
- WO-A2-2016/145416
- US-A1- 2008 050 731
- US-A1- 2014 234 898
- US-A1- 2017 008 950
- PATTERSON ET AL: "Finding the right (bioorthogonal) chemistry", ACS CHEMICAL BIOLOGY, vol. 9, 2014, pages 592 - 605, XP002785552
- SHIEH ET AL.: "Design Strategies for Bioorthogonal Smart Probes", ORGANIC & BIOMOLECULAR CHOMICTRY, vol. 12, no. 46, 14 December 2014 (2014-12-14), pages 9307 - 9320, XP055364074
- MARTELL ET AL.: "Applications of Copper-Catalyzed Click Chemistry in Activity-Based Protein Profiling", MOLECULES, vol. 19, no. 2, 27 January 2014 (2014-01-27), pages 1378 - 1393, XP055612888

## Description

### BACKGROUND

Imaging of biological samples, such as cell smears or tissue sections, is often limited to a few distinguishable channels. For example, the multiplexity of traditional fluorescence microscopy is limited by the spectral overlap of fluorophore emissions. The recent development of imaging mass spectrometry of samples stained with element labelled antibodies allows for many more proteins to be imaged simultaneously, as each protein is associated with a unique element or isotope through an antibody intermediate. Imaging of a plurality of proteins allows for characterization of diverse cell types within the same sample based on protein expression. However, additional characteristics of the sample, such as intracellular and extracellular structures, may not be easily visualized by antibody staining. Fluorescence and light microscopy, which uses histochemical stains to visualize these structures, are often unable to visualize metal-containing drugs and/or accumulated toxic heavy metals. Many mass spectrometry methods are able to detect gross levels of metals in biological sample, but are unable to detect these metals at subcellular resolution or in combination with antibody or histochemical stains.

In contrast to metals which are detected due to their accumulation due to environmental exposure or because they are components of drugs, elemental labelled reagents (such as antibodies) must be synthesised by taking the e.g. antibody and conjugating it to the elemental label. Current techniques however are limited by their applicability to specific types of proteins, preventing the development of a truly modular system for generating reagents. WO2017/004203A1 relates to methods and compositions for simultaneously detecting RNA and protein in a biological sample by mass spectrometry. WO2007/137418A1 relates to a new class of tagged biomolecules that have been specifically designed to operate in conjunction with elemental analysis detection, to provide high sensitivity multiplexed biomarker determinations. Patterson, et al., ACS Chemical Biology, 2014, 9, 592-605 relates to bioorthogonal chemistries used to tag diverse classes of biomolecules in cells and other complex environments.

### TECHNICAL FIELD

The present disclosure relates to reagents and their use for elemental imaging mass spectrometry of biological samples.

### SUMMARY

The invention is defined by the appended claims. In particular, the invention provides a mass-tagged SBP, comprising an SBP and a mass tag, wherein the SBP and the mass tag are joined by a covalent linker, wherein the linker is formed at least in part by a strain-promoted click chemistry reaction product, and wherein the mass tag comprises a polymer comprising a plurality of metal chelating moieties loaded with labeling atoms of the same metal isotope.

Aspects of the present disclosure include methods, reagent, kits and biological samples for imaging of mass (e.g. metal-) tagged reagents by elemental mass spectrometry, as described herein. The reagents and methods described herein relate generally to applications in imaging mass spectrometry of biological samples. Certain reagents described herein include histochemical stains, metal-containing drugs, and toxic heavy metals detectable by elemental imaging mass spectrometry, and specific binding pair members (SBPs) which are conjugated to mass tags by a new coupling chemistry termed click chemistry, which the inventors have discovered to be particularly amenable to application within the realm of mass-tagging SBPs. Certain methods described herein include imaging of a combination of mass (e.g. metal-) tagged antibodies, histochemical stains, metal-containing drugs, and/or toxic heavy metals by elemental mass spectrometry. In certain aspects, imaging is performed at subcellular resolution allowing for the distribution of metal-tags/mass tags to be related.

The present disclosure is based on the discovery that click chemistry is compatible with the reaction of molecules, such as biological macromolecules, with mass tags. Such mass-tagged molecules are of use in labelling samples with the mass tags, in much the same way as a traditional immunohistochemistry (IHC) methodology would be performed with fluorescently labelled reagents.

It is frequently useful to attach two components, such as an SBP and a label. To be of use, the linkage chemistry should be modular, so that it can be adapted to a variety of different scenarios, both with respect to the label and the SBP. The linkage chemistry, particularly where live cells are used, should be non-toxic, both in terms of the reagents and products including byproducts.

Current methods for attaching mass tags to biomolecules are limited by the chemistry of the existing reaction. In particular, prior to the present disclosure, conjugation was dependent on the presence of a sulfhydryl group in the biomolecule (e.g. cysteine in a protein). In some instances, no sulfhydryl is available, and in such a case, a molecule could not be labelled with a mass tag. Examples of such proteins include wheat germ agglutinin (which binds to N-acetyl-D-glucosamine and sialic acid sugar moieties) and phalloidin (a peptide that binds to actin), neither of which comprise sulfhydryl groups. Alternatively, in order to generate the sulfhydryl, it may be necessary to (at least partially) reduce the protein, breaking existing disulfide bonds. When this occurs, the structure of the reduced protein may be altered such that the protein (e.g. an antibody) can lose affinity and/or specificity for the intended target, and so preventing the protein-mass tag conjugate from acting as a reliable strain.

Accordingly, there is a need for a way of linking mass tags to molecules which does not require the presence of a sulfhydryl group on the molecule. The inventors address this need by providing a method of linking SBPs and mass tags using click chemistry. Click chemistry is a concept introduced in Barry Sharpless in the late 1990s. Its central concept is a high thermodynamic driving force that drives a reaction quickly and irreversibly to high yield of a single reaction product, with high reaction specificity (in some cases, with both regio- and stereo-specificity). The reactions typically produce few, if any, toxic byproducts, and many are amenable to performance at physiological conditions, that is to say in an aqueous buffer, at approximately neutral pH and tolerant of typical buffer salts, with the resultant product also being stable in these conditions. Those that do not strictly meet these criteria are nonetheless characterized by their high yield, simplicity and ability to be performed in benign or easily removed solvents. Of additional importance is that the molecular reactions are bioorthogonal, which is to say they proceed without interacting with any of the functionalities typically seen in biological systems, such as amines, amides, carbonyls, acids etc.

Of interest in the present disclosure is the conjugation of a member of a specific binding pair (as discussed below), termed herein an SBP, with a mass tag (*i.e.* a tag comprising a component that when ionised products an ion of a characteristic mass that can be detected, e.g. at least one labelling atom). Accordingly, certain aspects of the present disclosure provide a method of attaching an SBP to a mass tag, comprising the steps of:
(i) providing the SBP and the mass tag; and
(ii) conjugating the SBP to the mass tag wherein at least one step in the conjugation comprises a strained promoted click chemistry reaction.

Certain aspects of the present disclosure therefore also provide a mass-tagged SBP, comprising an SBP and a mass tag, wherein the SBP and the mass tag are joined by a covalent linker wherein the linker is formed at least in part by a strained promoted click chemistry reaction product.

The panel of reagents enabled by the inventive concept disclosed herein means that many different kinds of reagents can now be labelled with mass tags using the same kind of chemistry. Accordingly, certain aspects of the present disclosure also provide kits for labelling SBPs, and also kits of mass-tagged SBPs generated by the click chemistry disclosed herein, optionally kits comprising of mass-tagged SBPs and also other labelling atom containing reagents, for instance DNA intercalators. Likewise, certain aspects of the present disclosure also provide a method of labelling a sample using a mass-tagged SBP of the disclosure, optionally are methods of labelling a sample using multiple such mass-tagged SBPs, for example wherein the mass-tagged SBPs include SBPs of different types, for instance an antibody SBP (including multiple antibody SBPs), a nucleic acid SBP (including multiple nucleic acid SBPs), a lectin (including multiple lectins), a sugar (including multiple sugars) and a DNA intercalator (including multiple DNA intercalators). Similarly, certain aspects of the present disclosure include use of a mass-tagged SBP of the disclosure for labelling a sample, such as the use of multiple such mass-tagged SBPs for labelling a sample, for example wherein the mass-tagged SBPs include SBPs of different types, for instance an antibody SBP (including multiple antibody SBPs), a nucleic acid SBP (including multiple nucleic acid SBPs), a lectin (including multiple lectins), a sugar (including multiple sugars) and a DNA intercalator (including multiple DNA intercalators). Accordingly, certain aspects of the present disclosure also provide a sample labelled according to the disclosure, such as a sample labelled with a mass-tagged SBP of the disclosure, optionally a sample labelled with multiple such mass-tagged SBPs, for example wherein the mass-tagged SBPs include SBPs of different types, for instance an antibody SBP (including multiple antibody SBPs), a nucleic acid SBP (including multiple nucleic acid SBPs), a lectin (including multiple lectins), a sugar (including multiple sugars) and a DNA intercalator (including multiple DNA intercalators).

Certain aspects of the present disclosure also provide a method of performing mass cytometry, for instance imaging mass cytometry, on a sample of the disclosure, prepared in line with the above.

Certain aspects of the present disclosure also provide a mass cytometry apparatus, such as an imaging mass cytometry apparatus comprising a sample according to the disclosure.

### BRIEF DESCRIPTION OF THE FIGURES

FIGURE 1. Ruthenium Red staining of mucosal stroma.
FIGURE 2. FFPE section of mouse gut was stained with Trichrome stain (A) and IdU stain (B).
FIGURE 3. Postfixation OsO4 imaging by LA-ICP-MS of a tissue section prepared for electron microscopy.
FIGURE 4. Elemental imaging mass spectrometry of cisplatin in human pancreatic tumor xenograft to immunodeficient mice.
FIGURE 5. Cisplatin effects on tumor proliferation, DNA damage and cisplatin distribution in the tumor.
FIGURE 6. Cisplatin effects and distribution in the small intestine.
FIGURE 7. Histological features of skin identified by IMC, and distribution of platinum following treatment with cisplatin.
FIGURE 8. Cisplatin distribution in kidney and liver.
FIGURE 9. Kinetics of cisplatin distribution measured using monoisotopic cisplatin.
FIGURE 10. Cisplatin effects on tumor proliferation, DNA damage and 195Pt distribution in OCIP23.
FIGURE 11. Cisplatin effects on cell proliferation and platinum uptake determined by mass cytometry.
FIGURE 12. Platinum distribution in cryostat and FFPE sections.
FIGURE 13. Platinum distribution in large intestine.
FIGURE 14. Platinum distribution in non-tumor bearing mouse skin.
FIGURE 15. Platinum distribution in non-tumor bearing mouse kidney
FIGURE 16. Definiens Developer image analysis.
FIGURE 17. ¹H NMR spectrum of TCO-end polymer-based mass tag in D₂O.

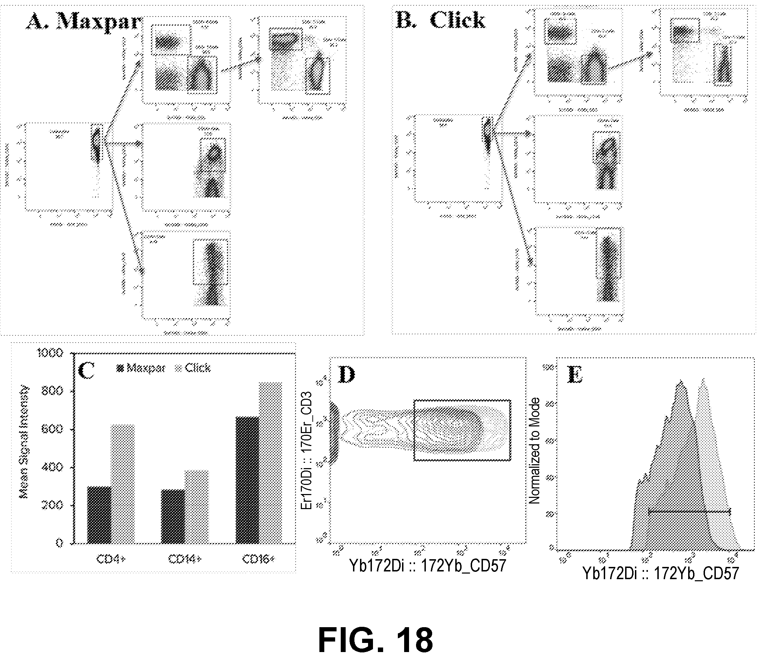

FIGURE 18. (A) Human PBMCs were stained with Maxpar Human PBMCs Basic II Phenotyping 172 145 Panel Kit and anti-human CD57 (HCD57)- ¹⁷²Yb Ab. (B) Click chemistry conjugated CD4- Nd, CD14-¹⁶⁰ Gd, and CD16-¹⁶⁵ Ho Abs at 1-2 µg/mL and CD57(HCD57)- Yb (IgM class) replaced respective Abs in the basic phenotyping panel. (A) and (B) show the gating strategy for live single cells. (C) Bar graph comparing mean signal intensity [normalized to EQ^{™} Four Element Calibration Beads (Fluidigm)] for Maxpar labeled Abs (red) versus click chemistry-conjugated Abs (green). (D) Contour plot and (E) signal distribution histogram for CD57+CD4-CD3+ cells.

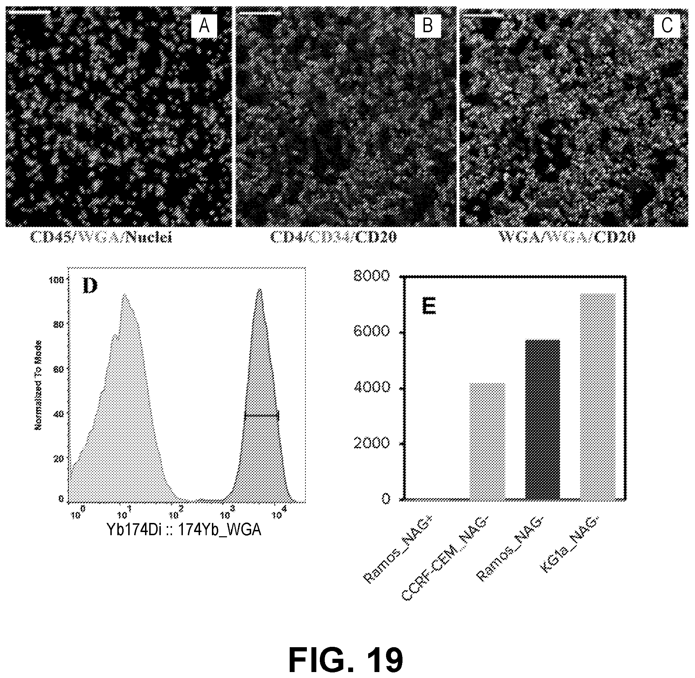

FIGURE 19: (A) Representative IMC pseudo-color image of human Ramos cells stained with WGA-¹⁷⁴Yb, CD45-¹⁵⁴Sm, and ^{191/193}Ir DNA intercalator. (B) and (C) show representative IMC images of mixed human KG1a, CCRF-CEM, and Ramos cells. Cells are stained with ¹⁴⁷Sm-CD20, ¹⁴⁸Nd-CD34, and ¹⁴⁵Nd-CD4 Abs prior to lectin staining. WGA-¹⁷⁴Yb treated with N-acetylglucosamine (NAG) is used to stain Ramos cells as a negative control. Untreated WGA-¹⁷⁴Yb is used to stain human KG1a and CCRF-CEM cells (D) Representative CyTOF^{®} histograms of Ramos cells stained with NAG-treated WGA-¹⁷⁴Yb (left distribution) with and WGA-¹⁷⁴Yb (right distribution) without NAG treatment. (E) Mean signal intensity (normalized to EQ beads) values of human KG1a, CCRF-CEM, and Ramos (NAG+/-) cells stained with WGA-¹⁷⁴Yb as analyzed by CyTOF. Scale bar = 100 µm.
FIGURE 20: Two-dimensional viSNE representation of human whole blood cell populations showing the locations of major cell populations (A) and distribution of WGA intensity (B). Human whole blood populations were modeled based on the surface staining of 29 targets. (C) Quantitation of metal-tagged WGA mean intensities for gated cell populations in human whole blood for size discrimination.
FIGURE 21: Pseudo-colour IMC images of HeLa cells. Lysosomes are visualized in red using 151Eu-CD107a, actin filaments in green with phalloidin-165Ho, and nuclei in blue with DNA intercalator-Ir staining. (B) Pseudo-colour IMC images of FFPE sections of mouse colon. Actin filaments are visualized in red using click chemistry (azide-DBCO) conjugated phalloidin-165Ho, nuclei in green with DNA intercalator-Ir staining, and the mucus of goblet cells in blue with click chemistry conjugated 174Yb-WGA (DBCO-Azide). Scale bar = 100 µm.

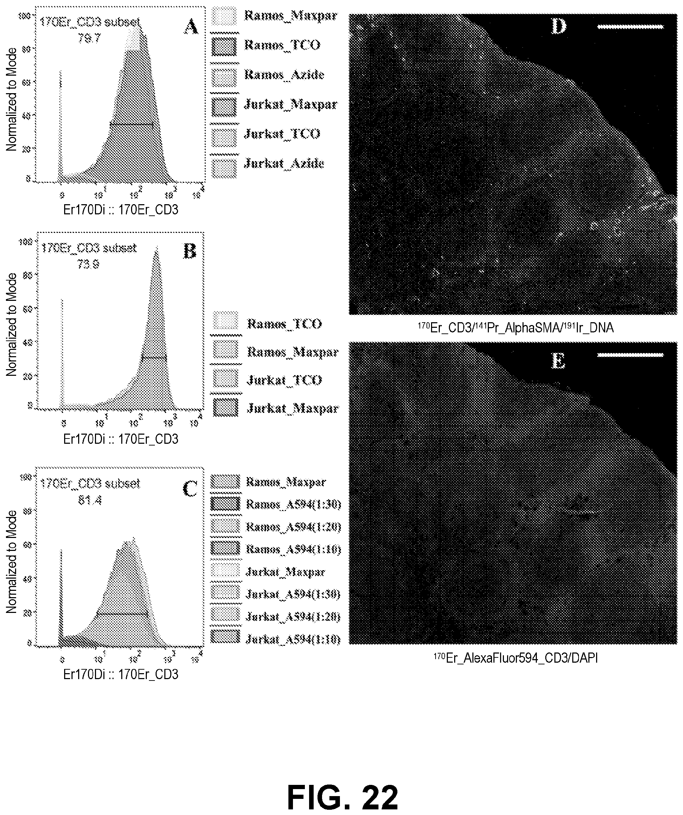

FIGURE 22: Human Jurkat and Ramos cells stained with Maxpar and click chemistry-conjugated anti-CD3(UCHT1) (2.5 µg/mL) mAbs. (A) Representative histograms show a comparison between Maxpar and click chemistry-conjugated (DBCO-Azide and Tetrazine-TCO) LEAF anti-CD3(UCHT1) mAbs. (B) show a comparison between Maxpar and click chemistry-conjugated (Tetrazine-TCO) purified anti-CD3(UCHT1) mAbs. (C) show a comparison between Maxpar and click chemistry-conjugated (TCO-Tetrazine) dual-tagged (¹⁷⁰Er and AlexaFluor594) purified anti-CD3(UCHT1) mAbs. Representative IMC (D) and Immunofluorescence (IF) (E) images of frozen tonsillar tissue sections labeled with dual-tagged CD3-¹⁷⁰Er-AlexaFluor594 mAb prepared via a two-step (thiol-maleimide conjugation for fluorophore tagging and click chemistry for mass tagging) conjugation method. Intercalator-¹⁹¹Ir (IMC) and DAPI (IF) were used for nucleus staining. Scale bar = 500 µm.

### DETAILED DESCRIPTION

Mass cytometry, including imaging mass cytometry, relies on the staining of target species (also referred to as analytes herein and in the field) on or in a sample using SBPs that bind to specific analytes (proteins, nucleic acids, sugars, metabolites etc).

The SBPs attached to mass tags therefore immobilise the mass tags to the area where the SBP's target analyte is found on or in cells. In order for each mass-tagged SBP to be detected discretely, a different mass tag is required. As explained in the art (e.g. Giesen et al., 2014, Nature methods 11: 417-422; D138, Z102), one key advantage over mass cytometry, including imaging mass cytometry, over other analytical techniques is the ability for very high levels of multiplexing, due to the ability of mass detectors to resolve differences of in the masses of elemental ions just one atomic mass unit apart.

To enable the best use of the capacity for highly multiplexed analytical procedures, it is therefore desirable to have a palette of reagents, which can be combined using common steps to achieve the desired mass-tagged SBPs. For instance, a standardised protocol can enable conjugation of a nucleic acid to a mass tag, a protein to a mass tag, a peptide to a mass tag, a sugar to a mass tag etc. by performing the same or at least highly similar reactions for each class of molecule. Likewise, any SBP can be conjugated to a variety of different mass tags using the same chemistry. This simple modular approach enables the provision of kits for labelling SBPs that are more simple for the use to utilise because the same reaction conditions/length of reaction etc. can be used to label multiple different SBPs each with different mass tags in parallel (and moreover, as explained elsewhere herein, at ambient conditions).

### Mass-tagged SBPs of the present disclosure and methods of synthesising the reagents and the constituent parts thereof

Mass-tagged reagents of certain aspects of the present dislcosure comprise a number of modular components. The first is the SBP. The second is the mass tag. The mass tag and the SBP are joined by a linker, formed at least in part of by the conjugation of the mass tag and the SBP. The linkage between the SBP and the mass tag may also comprise a spacer. One key component of certain aspects of the present disclosure described herein is the reaction used to generate the linker. The reaction is a strain promoted click chemistry reaction. Strain promoted click chemistry provides particular advantages in the conjugation of mass tags and SBPs. This is because many click chemistry reactions rely on the presence of a metal catalyst. For instance, click chemistry reactions have been reported which are catalysed by copper or iron. Such metals may be incorporated into the components being reacted together (e.g. into metal-ligating groups of the mass tag). This is undesirable at least because occupancy of metal-ligating groups of the mass tag by the metal catalyst prevents the desired labelling atom(s) from occupying the metal-ligating groups. Accordingly, sensitivity is lost in the desired mass channel. Furthermore, background could be increased, if the metal catalysing the reaction were the labelling atom in another mass-tagged SBP used in a multiplex reaction. Furthermore, catalytic metals such as copper are toxic to living cells, and so if some e.g. non-chelated copper remained in the composition of the conjugated mass-tagged SBP this could interfere with subsequent experimental procedures, if those procedures involved living material.

Accordingly, certain aspects of the present disclosure provide a method of attaching an SBP to a mass tag, comprising the steps of:
(i) providing the SBP and the mass tag; and
(ii) conjugating the SBP to the mass tag wherein at least one step in the conjugation comprises a strain promoted click chemistry reaction.

The present disclosure therefore also provides a mass-tagged SBP, comprising an SBP and a mass tag, wherein the SBP and the mass tag are joined by a covalent linker wherein the linker is formed at least in part by a strain promoted click chemistry reaction product. The linker can be formed from just the reaction product of the click chemistry reaction, for instance the triazole formed from reaction alkyne with an azide. Alternatively the linker might be comprise further atoms, such as from resulting of conjugation of a click chemistry reagent onto the SBP or mass tag/metal-chelating moiety (e.g. NHS ester reaction with lysine, or maleimide sulfhydryl reaction; and optionally spacers, to attach the click chemistry reagent to the SBP and/or mass tag). Herein below, these are called linker components; a first linker component may be conjugated to the SBP before the click chemistry reaction, and a second linker component may be conjugated to the mass tag before the click chemistry reaction. Together the linker component if present (optionally including spacers), and the reaction product of the click chemistry reaction form the linker between the mass tag and the SBP.

### Exemplary click chemistry reactions

A variety of different metal-catalyst free reactions, such as strain promoted reactions, can be used according to certain aspects of the present disclosure.

### a. Alkyne reaction with azide

A first example is the reaction between a strained alkyne and an azide, such as a strain promoted azide-alkyne cycloaddition between a cyclooctyne derivative and an azide.

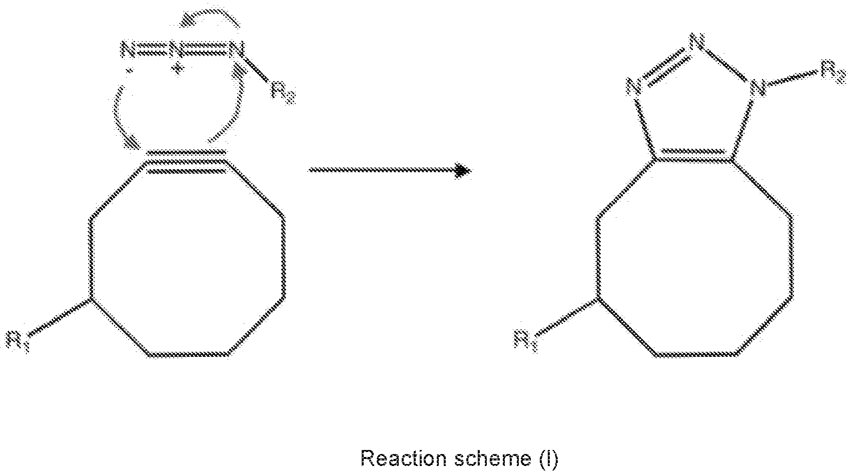

Here, reaction of the cyclooctyne and azide covalently links the R₁ and R₂ groups. Given the alkyne ring strain, the reaction of an organic azide with a cyclic alkyne, typically cyclooctyne, has become commonly known as strain-promoted azide-alkyne cycloaddition (SPAAC). In certain aspects of the present disclosure, R₁ may be the SBP and R₂ may be the mass tag. Alternatively, R₂ may be the SBP and R₁ may be the mass tag. Accordingly, in some embodiments of the present disclosure, the method comprises conjugating an SBP to a mass tag using a click chemistry reaction, wherein the click chemistry reaction is a reaction of an azide with an alkyne.

Reaction with of a cyclooctyne with an azide is characterised by relatively slow reaction kinetics, and requires large excesses of reagents, and long incubation times. Accordingly, cycloalkyne derivatives can be used which have greater reactivity, but without a compromise in reactivity. These include monofluorinated cyclooctyne (MOFO), difluorocyclooctyne (DIFO), dimethoxyazacyclooctyne (DIMAC), dibenzocyclooctyne (DIBO), dibenzoazacyclooctyne (DIBAC), biarylazacyclooctynone (BARAC), bicyclononyne (BCN), 2,3,6,7-tetramethoxy-DIBO (TMDIBO), sulfonylated DIBO (S-DIBO), carboxymethylmonobenzocyclooctyne (COMBO), pyrrolocyclooctyne (PYRROC). It is commonly understood that the enhanced reactivity of (di)benzoannulated cyclooctynes is caused by the increase in ring strain imparted by the multiple sp2-hybridized carbons. Many kinds of dibenzocyclootyne (DBCO) derivatives can be used in the present disclosure, such as those derivatised (directly or via a linker) with NHS esters, such as for conjugation to amines on the SBP (e.g. the N-terminus amine group, of the amine groups of the side chains of lysine, arginine, and histidine in proteins (e.g. antibodies and lectins), conjugation to amino modified oligonucleotide probes (as commercially available from IDT (IL, USA), Sigma Aldrich (MO, USA), Bio-Synthesis, Inc. (TX, USA) *inter alia),* amino sugar derivatives etc.). Alternatively, the DBCO derivative may be derivatised (directly or via a linker) with a maleimide (e.g. dibenzocyclooctyne-maleimide; Sigma Aldrich Catalog number 760668, or Dibenzocyclooctyne-PEG4-maleimide; Sigma Aldrich Catalog number 760676). The maleimide functionality can be used to couple the DBCO to a sulfhydryl group of the mass tag.

Accordingly, in some embodiments the alkyne is a cyclic alkyne, for instance wherein the cyclic alkyne is part of an 8-membered ring. The cyclic alkyne may be strained. Sometimes, the cyclic alkyne is part of a multi-ring structure that comprises 3 or more rings, optionally wherein the multi ring structure comprises at least two benzene rings. In some embodiments, the cyclic alkyne is dibenzocyclooctyne (DBCO).

The azide likewise can be coupled to the SBP (directly or via a linker) with NHS esters, such as azido-dPEG₈-NHS ester, azido-dPEG₁₂-NHS ester etc. (available from Sigma Aldrich; cat nos. QBD10503 and QBD10505, respectively). Via the NHS ester, the azide can be coupled to amines on the SBP (e.g. the N-terminus amine group, of the amine groups of the side chains of lysine, arginine, and histidine in proteins (e.g. antibodies and lectins), conjugation to amino modified oligonucleotide probes (as commercially available from IDT (IL, USA), Sigma Aldrich (MO, USA), Bio-Synthesis, Inc. (TX, USA) *inter alia),* amino sugar derivatives etc.). Alternatively, azide modified oligonucleotides/sugars can be synthesised directly (i.e. without need to attach the azide functionality via a separate NHS ester reaction to an amino modified). The azide component can also be coupled to the mass tag. For instance, azo initiators of polymerisation may be used in conventional polymerisation reactions. Azide terminated polymethacrylate is also available from Sigma Aldrich.

Accordingly, certain aspects of the present disclosure provide a series of methods of producing a mass-tagged SBP, comprising conjugating the mass tag and the SBP via a SPAAC reaction. In some embodiments, the method comprises the steps of providing an alkyne functionalised SBP and an azide functionalised mass tag, and reacting the alkyne functionalised SBP with the azide functionalised mass tag, such as wherein the alkyne functionalised SBP is functionalised with a strained cycloalkyne, for example DBCO. In some embodiments, the method comprises the steps of providing an azide functionalised SBP and an alkyne functionalised mass tag, and reacting the azide functionalised SBP and the alkyne functionalised mass tag, such as wherein the alkyne functionalised mass tag is functionalised with a strained cycloalkyne, for example DBCO. As noted below, a spacer may be presented between the SBP and the alkyne or azide and/or the mass tag and azide or alkyne.

Certain aspects of the present disclosure also provide a mass-tagged SBP, wherein the SBP and the mass tag are joined by a linker which comprises the reaction product of an alkyne and an azide, such as a strained cycloalkyne and an azide, for example DBCO and an azide. In some instances the reaction product is the product of a metal free click chemistry reaction, such as a copper free click chemistry reaction. Accordingly, certain aspects of the present disclosure provide a mass-tagged SBP wherein the SBP and the mass tag are joined by a linker comprising a triazole. The triazole group in the linker may be part of a multi-ring structure. In certain aspects, the multi-ring structure may comprise 4 or more rings. For example, the multi-ring structure may be a 3 member ring, a 4 member ring, a 5 member ring, a 6 member ring, 7 member ring, an 8 member ring, a 9 member ring, a 10 member ring, and so forth. In certain aspects, the multi-ring structure may comprise two or more benzene rings. Specifically, the multi-ring structure may comprise a dibenzocyclooctene group. The triazole group and the dibenzocyclooctene group may be in any orientation. For example, the triazole group may be separated from the SBP by the dibenzocyclooctene group (thus meaning the dibenzocyclooctene group would be separated from the mass tag by the triazole group). Alternatively, the dibenzocyclooctene group may be separated from the SBP by the triazole group (thus meaning the triazole group would be separated from the mass tag by the dibenzocyclooctene group).

As explained below, the mass tag comprises one or more labelling atoms, which allow presence identification of the target of the SBP in a simple manner in a mass detector. However, it is not the case that the SBP will be conjugated to a mass tag with the labelling elements already in the tag. Sometimes, SBP will be conjugated to a metal chelating moiety before one or more metal labelling atoms are loaded onto the metal-chelating moiety to form a mass tag. As explained below in more detail, the metal chelating moiety may be a single metal-chelating group or it may be a polymer to which a metal-chelating group has been attached to two or more subunits.

Accordingly, certain aspects of the present disclosure provide a series of methods of producing an SBP conjugated to a metal-chelating moiety, comprising conjugating the metal-chelating moiety and the SBP via a SPAAC reaction. In some embodiments, the method comprises the steps of providing an alkyne functionalised SBP and an azide functionalised metal-chelating moiety, and reacting the alkyne functionalised SBP with the azide functionalised metal-chelating moiety, such as wherein the alkyne functionalised SBP is functionalised with a strained cycloalkyne, for example DBCO. In some embodiments, the method comprises the steps of providing an azide functionalised SBP and an alkyne functionalised metal-chelating moiety, and reacting the azide functionalised SBP and the alkyne functionalised metal-chelating moiety, such as wherein the alkyne functionalised metal-chelating moiety is functionalised with a strained cycloalkyne, for example DBCO. As noted below, a spacer may be presented between the SBP and the alkyne or azide and/or the metal-chelating moiety and azide or alkyne.

The SPAAC reaction may be performed under physiological conditions, and is compatible with macro-biomolecules such as proteins (including, antibodies, ligands, receptors, nucleic acids etc., as described herein below in the section relating to SBPs on page 40). Physiological conditions may include an isotonic solution or biological buffer such as saline, phosphate buffered saline (PBS), and the like. Physiological conditions may alternatively or additionally include a temperature ranging from 1°C to 42°C, 4°C to 37°C, 4°C to 25°C, 10°C to 37°C, 25°C to 37°C, and so forth. Physiological conditions may include a neutral pH, a pH of 5.5 to 8.5, a pH of 6 to 8, a pH of 6.5 to 7.5, and so forth. Because the reaction between DBCO and azide may be a slow process, a relatively long incubation time may be preferable. For example, the conjugation of step b) may be conducted for 4 to 48 hours at 4 °C, 10 to 24 hours at 4 °C, 18 to 20 hours at 4 °C, 10 minutes to 10 hours at room temperature, 30 minutes to 5 hours at room temperature, 30 minutes to 3 hours at room temperature, 5 minutes to 5 hours at 37 °C, 10 minutes to 2 hours at 37 °C, and so forth.

Certain aspects of the present disclosure also provide a method of making a mass-tagged SBP comprising performing a method according to the previous paragraph to produce an SBP conjugated to a metal-chelating moiety, and further comprising the step of loading metal on to the metal-chelating moiety, e.g. polymer.

Certain aspects of the present disclosure also provide a SBP-metal-chelating moiety conjugate, wherein the SBP and the metal-chelating moiety are joined by a linker which comprises the reaction product of an alkyne and an azide, such as a strained cycloalkyne and an azide, for example DBCO and an azide. In some instances the reaction product is the product of a metal free click chemistry reaction, such as a copper free click chemistry reaction. Accordingly, certain aspects of the present disclosure provide a SBP-metal-chelating moiety conjugate wherein the SBP and the metal-chelating moiety are joined by a linker comprising a triazole. The triazole group in the linker may be part of a multi-ring structure. In certain aspects, the multi-ring structure may comprise 4 or more rings. For example, the multi-ring structure may be a 3 member ring, a 4 member ring, a 5 member ring, a 6 member ring, 7 member ring, an 8 member ring, a 9 member ring, a 10 member ring, and so forth. In certain aspects, the multi-ring structure may comprise two or more benzene rings. Specifically, the multi-ring structure may comprise a dibenzocyclooctene group. The triazole group and the dibenzocyclooctene group may be in any orientation. For example, the triazole group may be separated from the SBP by the dibenzocyclooctene group (thus meaning the dibenzocyclooctene group would be separated from the metal-chelating moiety by the triazole group). Alternatively, the dibenzocyclooctene group may be separated from the SBP by the triazole group (thus meaning the triazole group would be separated from the metal-chelating moiety by the dibenzocyclooctene group).

The mass-tagged SBP comprising a triazole group may be stable in solution. For example, the SBP-mass tag may be stable in solution for up to a week, a month, 6 months, a year, 2 years, 5 years and so forth. The SBP-mass tag may be stable in solution at -20°C (e.g., wherein the solution comprises glycerol), below freezing, 4 °C, 10 °C, or at room temperature. Where the SBP is an antibody, stability may be measured by antibody affinity.

In some instances, the click chemistry reaction proceeds in the absence of a metal catalyst, in particular wherein the click chemistry reaction proceeds in the absence of copper or iron. In some instances, the click chemistry reaction is performed under physiological conditions, optionally wherein the click chemistry reaction is performed at a pH from 6 to 8, such as wherein the click chemistry reaction is performed in a buffer, for example wherein the buffer is isotonic.

Sometimes, the alkyne is attached to the SBP and the azide is attached to the mass tag or metal-chelating moiety. Sometimes, the azide is attached to the SBP and the alkyne is attached to mass tag or the metal-chelating moiety. Sometimes, where the alkyne is attached to the SBP, it is attached by a linker component (optionally via a spacer). Sometimes, where the alkyne is attached to the mass tag or metal-chelating moiety, it is attached by a linker component (optionally via a spacer). Sometimes, where the azide is attached to the SBP, it is attached by a linker component (optionally via a spacer). Sometimes, where the azide is attached to the mass tag or metal-chelating moiety, it is attached by a linker component (optionally via a spacer).

### b. Alkene reaction with tetrazine

A second example is the reaction between a strained alkene and a tetrazine, such as a strain promoted tetrazine-alkene cycloaddition between a trans-cyclooctene derivative and a tetrazine.

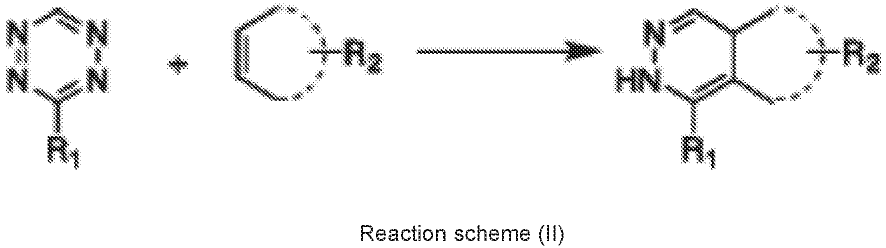

Here, reaction of the trans-cyclooctene and tetrazine covalently links the R₁ and R₂ groups. Given the alkyne ring strain, the reaction of an organic tetrazine with a cyclic alkene, typically trans-cyclooctene, in an inverse electron demand Diels-Alder cycloaddition (iEDDA). In certain aspects of the present disclosure, R₁ may be the SBP and R₂ may be the mass tag. Alternatively, R₂ may be the SBP and R₁ may be the mass tag.

Among three different possible tetrazine isomers, 1,2,4,5-tetrazine is used for the iEDDA reaction. The completion of the reaction releases N₂ gas as the only by-product, which makes the iEDDA reaction irreversible and more suitable for bio-labelling than conventional reversible Diels-Alder reactions.

Trans-cyclooctene (TCO) is currently one of the most reactive cyclic alkenes known as a reagent in this reaction. Many kinds of derivatives can be used in certain aspects of the present disclosure, such as those derivatised (directly or via a linker) with NHS esters, such as for conjugation to amines on the SBP (e.g. lysine). Alternatively, the TCO derivative may be derivatised (directly or via a linker) with a maleimide (e.g. the N-terminus amine group, of the amine groups of the side chains of lysine, arginine, and histidine in proteins (e.g. antibodies and lectins), conjugation to amino modified oligonucleotide probes (as commercially available from IDT (IL, USA), Sigma Aldrich (MO, USA), Bio-Synthesis, Inc. (TX, USA) *inter alia),* amino sugar derivatives etc.). The maleimide functionality can be used to couple the TCO to a sulfhydryl group of the mass tag. *trans*-bicyclo[6.1.0]nonene derivatives can also be used, with the substitution occurring on the cyclopropyl ring. Although less rapid than TCO, methylcyclopropene, bicyclo[6.1.0]nonyne, cyclooctyne and norbornene can also be reacted with tetrazines.

The tetrazine likewise can be coupled to the SBP (directly or via a linker) with NHS esters (such as available from Sigma Aldrich). Via the NHS ester, the tetrazine can be coupled to amines on the SBP (e.g. the N-terminus amine group, of the amine groups of the side chains of lysine, arginine, and histidine in proteins (e.g. antibodies and lectins), conjugation to amino modified oligonucleotide probes (as commercially available from IDT (IL, USA), Sigma Aldrich (MO, USA), Bio-Synthesis, Inc. (TX, USA) *inter alia),* amino sugar derivatives etc.).The tetrazine component can also be coupled to the mass tag, e.g. where the component also comprises a maleimide functionality. There are two main types of tetrazines that are widely applied: 6-methyl-substituted tetrazines and 6-hydrogen-substituted tetrazines. Methyl-substituted tetrazines exhibit a high stability even when dissolved in aqueous media, while still offering faster reaction kinetics with TCO derivatives than any other bioorthogonal reaction pairs (approx. 1000 M⁻¹ s⁻¹). Moreover, they tolerate a wide array of reaction conditions. This makes them the prime choice for applications like protein labelling. Hydrogen-substituted tetrazines, on the other hand, show lower stability and less tolerance to harsh reaction conditions, but offer extremely fast reaction kinetics (up to 30000 M⁻¹ s⁻¹) for applications like in vivo imaging. The tetrazine can be 3-(benzylamino)-tetrazine.

Accordingly, certain aspects of the present disclosure provide a series of methods of producing a mass-tagged SBP, comprising conjugating the mass tag and the SBP via an inverse electron-demand Diels-Alder cycloaddition reaction between a strained alkene and a tetrazine (which is followed by a retro-Diels-Alder reaction under elimination of N₂). In some embodiments, the method comprises the steps of providing an alkene functionalised SBP and a tetrazine functionalised mass tag, and reacting the alkene functionalised SBP with the tetrazine functionalised mass tag, such as wherein the alkene functionalised SBP is functionalised with a strained cycloalkene, for example TCO. In some embodiments, the method comprises the steps of providing an tetrazine functionalised SBP and an alkene functionalised mass tag, and reacting the tetrazine functionalised SBP and the alkene functionalised mass tag, such as wherein the alkene functionalised mass tag is functionalised with a strained cycloalkene, for example TCO. As noted below, a spacer may be presented between the SBP and the alkene or tetrazine and/or the mass tag and tetrazine or alkene.

Certain aspects of the present disclosure also provide a mass-tagged SBP, wherein the SBP and the mass tag are joined by a linker which comprises the reaction product of an alkene and a tetrazine, such as a strained cycloalkene and a tetrazine, for example TCO and a tetrazine. In some instances the reaction product is the product of a metal free click chemistry reaction, such as a copper free click chemistry reaction. Accordingly, certain aspects of the present disclosure provide a mass-tagged SBP wherein the SBP and the mass tag are joined by a linker comprising a pyridazine. The pyridazine group in the linker may be part of a multi-ring structure. In certain aspects, the multi-ring structure may comprise 2 or more rings. For example, the multi-ring structure may be a 3 member ring, a 4 member ring, a 5 member ring, a 6 member ring, 7 member ring, an 8 member ring, a 9 member ring, a 10 member ring, and so forth. In certain aspects, the multi-ring structure may comprise a 6 member ring and an 8 member ring. Specifically, the multi-ring structure may comprise a cycylooctane group. The pyridazine group and the cyclooctane group may be in any orientation. For example, the pyridazine group may be separated from the SBP by the cyclooctane group (thus meaning the cyclooctane group would be separated from the mass tag by the pyridazine group). Alternatively, the cyclooctane group may be separated from the SBP by the pyridazine group (thus meaning the pyridazine group would be separated from the mass tag by the cyclooctane group).

As explained below, the mass tag comprises one or more labelling atoms, which allow presence identification of the target of the SBP in a simple manner in a mass detector. However, it is not the case that the SBP will be conjugated to a mass tag with the labelling elements already in the tag. Sometimes, SBP will be conjugated to a metal-chelating moiety before one or more labelling atoms are loaded onto the metal-chelating moiety. As explained below in more detail, the metal-chelating moiety may be a single metal-chelating group or it may be a polymer to which a metal-chelating group has been attached to two or more subunits.

Accordingly, certain aspects of the present disclosure provide a series of methods of producing an SBP conjugated to a metal-chelating moiety, comprising conjugating the metal-chelating moiety and the SBP via an inverse electron-demand Diels-Alder cycloaddition (iEDDA) reaction between a strained alkene and a tetrazine (which is followed by a retro-Diels-Alder reaction under elimination of N₂). In some embodiments, the method comprises the steps of providing an alkene functionalised SBP and a tetrazine functionalised metal-chelating moiety, and reacting the alkene functionalised SBP with the tetrazine functionalised metal-chelating moiety, such as wherein the alkene functionalised SBP is functionalised with a strained cycloalkene, for example TCO. In some embodiments, the method comprises the steps of providing a tetrazine functionalised SBP and an alkene functionalised metal-chelating moiety, and reacting the tetrazine functionalised SBP and the alkene functionalised metal-chelating moiety, such as wherein the alkene functionalised metal-chelating moiety is functionalised with a strained cycloalkene, for example TCO. As noted below, a spacer may be presented between the SBP and the alkene or tetrazine and/or the metal-chelating moiety and tetrazine or alkene.

The iEDDA reaction may be performed under physiological conditions, and is compatible with macro-biomolecules such as proteins (including, antibodies, ligands, receptors, nucleic acids etc., as described herein below in the section relating to SBPs on page40). Physiological conditions may include an isotonic solution or biological buffer such as saline, phosphate buffered saline (PBS), and the like. Physiological conditions may alternatively or additionally include a temperature ranging from 1°C to 42°C, 4°C to 37°C, 4°C to 25°C, 10°C to 37°C, 25°C to 37°C, and so forth. Physiological conditions may include a neutral pH, a pH of 5.5 to 8.5, a pH of 6 to 8, a pH of 6.5 to 7.5, and so forth. Because the reaction between TCO and tetrazine is a rapid process, advantageously short incubation times are possible. For example, the conjugation of step b) may be conducted for 1 to 4 hours at 4 °C, 1 to 60 minutes at room temperature, 1 minutes to 10 minutes at 37 °C, and so forth.

Certain aspects of the present disclosure also provide a method of making a mass-tagged SBP comprising performing a method according to the previous paragraph to produce an SBP conjugated to a metal-chelating moiety, and further comprising the step of binding metal to the polymer.

Certain aspects of the present disclosure also provide a SBP-metal-chelating moiety conjugate, wherein the SBP and the metal-chelating moiety are joined by a linker which comprises the reaction product of an alkene and a tetrazine, such as a strained cycloalkene and a tetrazine, for example TCO and a tetrazine. In some instances the reaction product is the product of a metal free click chemistry reaction, such as a copper free click chemistry reaction. Accordingly, certain aspects of the present disclosure provide a SBP-metal-chelating moiety conjugate wherein the SBP and the metal-chelating moiety are joined by a linker comprising a pyridazine. The pyridazine group in the linker may be part of a multi-ring structure. In certain aspects, the multi-ring structure may comprise 2 or more rings. For example, the multi-ring structure may be a 3 member ring, a 4 member ring, a 5 member ring, a 6 member ring, 7 member ring, an 8 member ring, a 9 member ring, a 10 member ring, and so forth. In certain aspects, the multi-ring structure may comprise a 6 member ring and an 8 member ring. Specifically, the multi-ring structure may comprise a cycylooctane group. The pyridazine group and the cyclooctane group may be in any orientation. For example, the pyridazine group may be separated from the SBP by the cyclooctane group (thus meaning the cyclooctane group would be separated from the metal-chelating moiety by the pyridazine group). Alternatively, the cyclooctane group may be separated from the SBP by the pyridazine group (thus meaning the pyridazine group would be separated from the metal-chelating moiety by the cyclooctane group).

The mass-tagged SBP comprising a pyridazine group may be stable in solution. For example, the SBP-mass tag may be stable in solution for up to a week, a month, 6 months, a year, 2 years, 5 years and so forth. In certain cases, stability may be increased by lyophilization. The SBP-mass tag may be stable in solution at -20°C (e.g., wherein the solution comprises glycerol), below freezing, 4 °C, 10 °C, or at room temperature. Where the SBP is an antibody, stability may be measured by antibody affinity.

In some instances, the click chemistry reaction proceeds in the absence of a metal catalyst, in particular wherein the click chemistry reaction proceeds in the absence of copper or iron. In some instances, the click chemistry reaction is performed under physiological conditions, optionally wherein the click chemistry reaction is performed at a pH from 6 to 8, such as wherein the click chemistry reaction is performed in a buffer, for example wherein the buffer is isotonic.

Sometimes, the alkene is attached to the SBP and the tetrazine is attached to the mass tag or metal-chelating moiety. Sometimes, the tetrazine is attached to the SBP and the alkene is attached to mass tag or the metal-chelating moiety. Sometimes, where the alkene is attached to the SBP, it is attached by a linker component (optionally via a spacer). Sometimes, where the alkene is attached to the mass tag or metal-chelating moiety, it is attached by a linker component (optionally via a spacer). Sometimes, where the tetrazine is attached to the SBP, it is attached by a linker component (optionally via a spacer). Sometimes, where the tetrazine is attached to the mass tag or metal-chelating moiety, it is attached by a linker component (optionally via a spacer).

### c. Alkyne reaction with nitrone

A third example is the reaction between a strained alkyne and a nitrone, such as a strain promoted nitrone-alkyne cycloaddition between a cyclooctyne derivative and a nitrone.

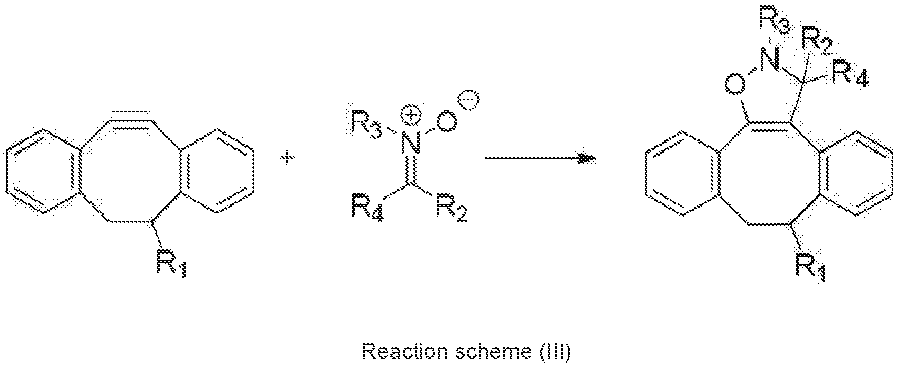

Here, reaction of the cyclooctyne and nitrone covalently links the R₁ and R₂₋R₄ groups. Given the alkyne ring strain, the reaction of an organic nitrone with a cyclic alkyne, typically cyclooctyne, has become commonly known as strain-promoted alkyne-nitrone cycloaddition (SPANC). In certain aspects of the present disclosure, R₁ may be the SBP and one of R₂₋R₄, typically R₂, may be the mass tag. Alternatively, one of R₂₋R₄, typically R₂, may be the SBP and R₁ may be the mass tag.

It is commonly understood that the enhanced reactivity of (di)benzoannulated cyclooctynes is caused by the increase in ring strain imparted by the multiple sp2-hybridized carbons. Many kinds of dibenzocyclootyne (DBCO) derivatives can be used in the present disclosure, such as those derivatised (directly or via a linker) with NHS esters, such as for conjugation to amines on the SBP (e.g. the N-terminus amine group, of the amine groups of the side chains of lysine, arginine, and histidine in proteins (e.g. antibodies and lectins), conjugation to amino modified oligonucleotide probes (as commercially available from IDT (IL, USA), Sigma Aldrich (MO, USA), Bio-Synthesis, Inc. (TX, USA) *inter alia),* amino sugar derivatives etc.). Alternatively, the DBCO derivative may be derivatised (directly or via a spacer) with a maleimide (e.g. dibenzocyclooctyne-maleimide; Sigma Aldrich Catalog number 760668, or Dibenzocyclooctyne-PEG4-maleimide; Sigma Aldrich Catalog number 760676). The maleimide functionality can be used to couple the DBCO to a sulfhydryl group of the mass tag.

The nitrone likewise can be coupled to the SBP (directly or via a spacer) with NHS esters). Via the NHS ester, the nitrone can be coupled to amines on the SBP (e.g. the N-terminus amine group, of the amine groups of the side chains of lysine, arginine, and histidine in proteins (e.g. antibodies and lectins), conjugation to amino modified oligonucleotide probes (as commercially available from IDT (IL, USA), Sigma Aldrich (MO, USA), Bio-Synthesis, Inc. (TX, USA) *inter alia*), amino sugar derivatives etc.). The nitrone component can also be coupled to the mass tag.

Accordingly, certain aspects of the present disclosure provide a series of methods of producing a mass-tagged SBP, comprising conjugating the mass tag and the SBP via a SPANC reaction. In some embodiments, the method comprises the steps of providing an alkyne functionalised SBP and a nitrone functionalised mass tag, and reacting the alkyne functionalised SBP with the nitrone functionalised mass tag, such as wherein the alkyne functionalised SBP is functionalised with a strained cycloalkyne, for example DBCO. In some embodiments, the method comprises the steps of providing a nitrone functionalised SBP and an alkyne functionalised mass tag, and reacting the nitrone functionalised SBP and the alkyne functionalised mass tag, such as wherein the alkyne functionalised mass tag is functionalised with a strained cycloalkyne, for example DBCO. As noted below, a spacer may be presented between the SBP and the alkyne or nitrone and/or the mass tag and nitrone or alkyne.

Certain aspects of the present disclosure also provide a mass-tagged SBP, wherein the SBP and the mass tag are joined by a linker which comprises the reaction product of an alkyne and a nitrone, such as a strained cycloalkyne and a nitrone, for example DBCO and a nitrone. In some instances the reaction product is the product of a metal free click chemistry reaction, such as a copper free click chemistry reaction. Accordingly, certain aspects of the present disclosure provide a mass-tagged SBP wherein the SBP and the mass tag are joined by a linker comprising an isoxazole. The isoxazole group in the linker may be part of a multi-ring structure. In certain aspects, the multi-ring structure may comprise 4 or more rings. For example, the multi-ring structure may be a 3 member ring, a 4 member ring, a 5 member ring, a 6 member ring, 7 member ring, an 8 member ring, a 9 member ring, a 10 member ring, and so forth. In certain aspects, the multi-ring structure may comprise two or more benzene rings. Specifically, the multi-ring structure may comprise a dibenzocyclooctene group. The isoxazole group and the dibenzocyclooctene group may be in any orientation. For example, the isoxazole group may be separated from the SBP by the dibenzocyclooctene group (thus meaning the dibenzocyclooctene group would be separated from the mass tag by the isoxazole group). Alternatively, the dibenzocyclooctene group may be separated from the SBP by the isoxazole group (thus meaning the isoxazole group would be separated from the mass tag by the dibenzocyclooctene group).

As explained below, the mass tag comprises one or more labelling atoms, which allow presence identification of the target of the SBP in a simple manner in a mass detector. However, it is not the case that the SBP will be conjugated to a mass tag with the labelling elements already in the tag. Sometimes, SBP will be conjugated to a metal-chelating moiety before one or more labelling atoms are loaded onto the metal-chelating moiety to form a mass tag. As explained below in more detail, the metal-chelating moiety may be a single metal-chelating group or it may be a polymer to which a metal-chelating group has been attached to each of two or more subunits.

Accordingly, certain aspects of the present disclosure provide a series of methods of producing an SBP conjugated to a metal-chelating moiety, comprising conjugating the metal-chelating moiety and the SBP via a SPANC reaction. In some embodiments, the method comprises the steps of providing an alkyne functionalised SBP and a nitrone functionalised metal-chelating moiety, and reacting the alkyne functionalised SBP with the nitrone functionalised metal-chelating moiety, such as wherein the alkyne functionalised SBP is functionalised with a strained cycloalkyne, for example DBCO. In some embodiments, the method comprises the steps of providing a nitrone functionalised SBP and an alkyne functionalised metal-chelating moiety, and reacting the nitrone functionalised SBP and the alkyne functionalised metal-chelating moiety, such as wherein the alkyne functionalised metal-chelating moiety is functionalised with a strained cycloalkyne, for example DBCO. As noted below, a spacer may be presented between the SBP and the alkyne or nitrone and/or the metal-chelating moiety and nitrone or alkyne.

The SPAAC reaction may be performed under physiological conditions, and is compatible with macro-biomolecules such as proteins (including, antibodies, ligands, receptors, nucleic acids etc., as described herein below in the section relating to SBPs on page 40). Physiological conditions may include an isotonic solution or biological buffer such as saline, phosphate buffered saline (PBS), and the like. Physiological conditions may alternatively or additionally include a temperature ranging from 1°C to 42°C, 4°C to 37°C, 4°C to 25°C, 10°C to 37°C, 25°C to 37°C, and so forth. Physiological conditions may include a neutral pH, a pH of 5.5 to 8.5, a pH of 6 to 8, a pH of 6.5 to 7.5, and so forth. Because the reaction between DBCO and nitrone may be a slow process, a relatively long incubation time may be preferable. For example, the conjugation of step b) may be conducted for 4 to 48 hours at 4 °C, 10 to 24 hours at 4 °C, 18 to 20 hours at 4 °C, 10 minutes to 10 hours at room temperature, 30 minutes to 5 hours at room temperature, 30 minutes to 3 hours at room temperature, 5 minutes to 5 hours at 37°C, 10 minutes to 2 hours at 37 °C, and so forth.

Certain aspects of the present disclosure also provide a method of making a mass-tagged SBP comprising performing a method according to the previous paragraph to produce an SBP conjugated to a metal-chelating moiety, and further comprising the step of binding metal to the polymer.

Certain aspects of the present disclosure also provide a SBP-metal-chelating moiety conjugate, wherein the SBP and the metal-chelating moiety are joined by a linker which comprises the reaction product of an alkyne and a nitrone, such as a strained cycloalkyne and a nitrone, for example DBCO and a nitrone. In some instances the reaction product is the product of a metal free click chemistry reaction, such as a copper free click chemistry reaction. Accordingly, certain aspects of the present disclosure provide a SBP-metal-chelating moiety conjugate wherein the SBP and the metal-chelating moiety are joined by a linker comprising an isoxazole. The isoxazole group in the linker may be part of a multi-ring structure. In certain aspects, the multi-ring structure may comprise 4 or more rings. For example, the multi-ring structure may be a 3 member ring, a 4 member ring, a 5 member ring, a 6 member ring, 7 member ring, an 8 member ring, a 9 member ring, a 10 member ring, and so forth. In certain aspects, the multi-ring structure may comprise two or more benzene rings. Specifically, the multi-ring structure may comprise a dibenzocyclooctene group. The isoxazole group and the dibenzocyclooctene group may be in any orientation. For example, the isoxazole group may be separated from the SBP by the dibenzocyclooctene group (thus meaning the dibenzocyclooctene group would be separated from the metal-chelating moiety by the isoxazole group). Alternatively, the dibenzocyclooctene group may be separated from the SBP by the isoxazole group (thus meaning the isoxazole group would be separated from the metal-chelating moiety by the dibenzocyclooctene group).

The mass-tagged SBP comprising an isoxazole group may be stable in solution. For example, the SBP-mass tag may be stable in solution for up to a week, a month, 6 months, a year, 2 years, 5 years and so forth. The SBP-mass tag may be stable in solution at -20°C (e.g., wherein the solution comprises glycerol), below freezing, 4 °C, 10 °C, or at room temperature. Where the SBP is an antibody, stability may be measured by antibody affinity.

In some instances, the click chemistry reaction proceeds in the absence of a metal catalyst, in particular wherein the click chemistry reaction proceeds in the absence of copper or iron. In some instances, the click chemistry reaction is performed under physiological conditions, optionally wherein the click chemistry reaction is performed at a pH from 6 to 8, such as wherein the click chemistry reaction is performed in a buffer, for example wherein the buffer is isotonic.

Sometimes, the alkyne is attached to the SBP and the nitrone is attached to the mass tag or metal-chelating moiety. Sometimes, the nitrone is attached to the SBP and the alkyne is attached to mass tag or the metal-chelating moiety. Sometimes, where the alkyne is attached to the SBP, it is attached by a linker component (optionally via a spacer). Sometimes, where the alkyne is attached to the mass tag or metal-chelating moiety, it is attached by a linker component (optionally via a spacer). Sometimes, where the nitrone is attached to the SBP, it is attached by a linker component (optionally via a spacer). Sometimes, where the nitrone is attached to the mass tag or metal-chelating moiety, it is attached by a linker component (optionally via a spacer).

### General reaction schemes for preparation of mass-tagged SBPs

As will be apparent, a variety of different orders of the method steps can be performed to synthesis a mass-tagged SBP. Several exemplary processes are set out below:
(a) (i) Synthesise metal-chelating moiety comprising a click chemistry reagent functionality; (ii) load metals onto metal-chelating moiety to form a mass tag; (iii) react mass tag with a first linker component comprising a click chemistry functional group (e.g. azide or DBCO; tetrazine or TCO, etc.); (iv) react SBP with a second linker component comprising a complementary click chemistry functional group (e.g. DBCO or azide; TCO or tetrazine; etc.); (v) react the SBP-linker component with the mass tag linker component to form a mass-tagged SBP, wherein the mass tag and the SBP are covalently joined by a linker (formed from the two linker components). As will be appreciated by the skilled person that the main thrust of this exemplary method is the fact that the metals are loaded onto the metal-chelating moiety to form a mass tag before that is reacted with the linker and then the SBP. Temporally, it may be possible that step (iv), for instance, comes before steps (i), (ii) or (iii), provided that (i) comes before (ii), (ii), before (iii) and (i), (ii), (iii) and (iv) before (v). Likewise, it will be appreciated, if the SBP and/or metal-chelating moiety/mass tag already contain the appropriate functionalities, it will not be necessary to add the functionality on via reaction with a linker component.
(b) (i) Synthesise metal-chelating moiety comprising a click chemistry reagent functionality; (ii) react metal-chelating moiety with a first linker component comprising a click chemistry functional group (e.g. azide or DBCO; tetrazine or TCO; etc.); (iii) load metals onto metal-chelating moiety to form a mass tag; (iv) react SBP with a further linker component comprising a complementary click chemistry functional group (e.g. DBCO or azide; TCO or tetrazine; etc.); (v) react the SBP-linker component with the mass tag linker component to form a mass-tagged SBP, wherein the mass tag and the SBP are covalently joined by a linker (formed from the two linker components). As will be appreciated by the skilled person that the main thrust of this exemplary method is the fact that the metal-chelating moiety is attached to the linker component providing the click chemistry functional group before metals are loaded onto the metal-chelating moiety to form a mass tag before that is reacted with the SBP. Temporally, it may be possible that step (iv), for instance, comes before steps (i), (ii) or (iii), provided that (i) comes before (ii), (ii), before (iii) and (i), (ii), (iii) and (iv) before (v). Likewise, it will be appreciated, if the SBP and/or metal-chelating moiety/mass tag already contain the appropriate functionalities, it will not be necessary to add the functionality on via reaction with a linker component.
(c) (i) Synthesise metal-chelating moiety comprising a click chemistry reagent functionality; (ii) react metal-chelating moiety with a first linker component comprising a click chemistry functional group (e.g. azide or DBCO; tetrazine or TCO; etc.); (iii) react SBP with a further linker component comprising a complementary click chemistry functional group (e.g. DBCO or azide; TCO or tetrazine; etc.); (iv) react the SBP-linker component with the metal-chelating moiety linker component to form an SBP-metal-chelating moiety conjugate, wherein the mass tag and the SBP are covalently joined by a linker (formed from the two linker components); (v) load metals onto metal-chelating moiety to form a mass tag. As will be appreciated by the skilled person that the main thrust of this exemplary method is the fact that the metals are loaded onto the metal-chelating moiety to form a mass tag after the metal-chelating moiety has been reacted with the SBP to form the SBP-metal-chelating moiety conjugate. Likewise, it will be appreciated, if the SBP and/or metal-chelating moiety/mass tag already contain the appropriate functionalities, it will not be necessary to add the functionality on via reaction with a linker component.
(d) (i) Synthesise metal-chelating moiety comprising a click chemistry reagent functionality; (ii) load metals onto metal-chelating moiety to form a mass tag; (iii) react mass tag with a first linker component comprising a click chemistry functional group (e.g. azide or DBCO; tetrazine or TCO; etc.); (iv) react mass tag-linker component with a further linker component comprising a complementary click chemistry functional group (e.g. DBCO or azide; TCO or tetrazine; etc.) and an NHS ester to form a mass tag-linker; (v) react the mass tag-linker with an SBP to form a mass-tagged SBP, wherein the mass tag and the SBP are covalently joined by a linker (formed from the two linker components). As will be appreciated, if the SBP and/or metal-chelating moiety/mass tag already contain the appropriate functionalities, it will not be necessary to add the functionality on via reaction with a linker component. Likewise, it will be appreciated that this method can be easily adapted such that the SBP is linked to a first linker component, that SBP-linker component linked to a second linker component to form an SNP linker before conjugation of the SBP-linker with the mass tag.
(e) (i) Synthesise metal-chelating moiety comprising a click chemistry reagent functionality; (ii) react metal-chelating moiety with a first linker component comprising a click chemistry functional group (e.g. azide or DBCO; tetrazine or TCO; etc.); (iii) load metals onto metal-chelating moiety to form a mass tag; (iv) react mass tag-linker component with a further linker component comprising a complementary click chemistry functional group (e.g. DBCO or azide; TCO or tetrazine; etc.) and an NHS ester to form a mass tag-linker; (v) react the mass tag-linker with an SBP to form a SBP-metal-chelating moiety conjugate, wherein the mass tag and the SBP are covalently joined by a linker (formed from the two linker components). As will be appreciated, if the SBP and/or metal-chelating moiety/mass tag already contain the appropriate functionalities, it will not be necessary to add the functionality on via reaction with a linker component.
(f) (i) Synthesise metal-chelating moiety comprising a click chemistry reagent functionality; (ii) react metal-chelating moiety with a first linker component comprising a click chemistry functional group (e.g. azide or DBCO; tetrazine or TCO; etc.); (iii) react metal-chelating moiety-linker component with a further linker component comprising a complementary click chemistry functional group (e.g. DBCO or azide; TCO or tetrazine; etc.) and an NHS ester to form a metal-chelating moiety-linker; (iv) react the SBP component with the metal-chelating moiety-linker to form a mass-tagged SBP, wherein the mass tag and the SBP are covalently joined by a linker (formed from the two linker components); (v) load metals onto metal-chelating moiety to form a mass tag. As will be appreciated, if the SBP and/or metal-chelating moiety/mass tag already contain the appropriate functionalities, it will not be necessary to add the functionality on via reaction with a linker component. Likewise, it will be appreciated that this method can be easily adapted such that the SBP is linked to a first linker component, that SBP-linker component linked to a second linker component to form an SNP linker before conjugation of the SBP-linker with the metal-chelating moiety, and ultimately the loading of metals onto the metal-chelating moiety.

In the various reaction steps of the exemplary methods discussed hereinabove in this subsection, excess reaction components may be removed by centrifugal filtration with a molecular weight cutoff chosen as appropriate for the product desired to be retained and the excess reactant desired to be removed. For instance, when conjugating a linker component to an IgG antibody, a cutoff of 50 kiloDaltons (kDa), such as 100kDa or more, could be chosen, as the IgG has a higher MW than this, but the linker would typically have an MW significantly lower. Such spin filters may be used to separate labelled SBP from label, if the label itself has an MW signigicantly lower than the cut off (e.g. polymer mass tags of a mass range of a one to a few tens of thousand MW). In some instances, multiple rounds of centrifugal filtration followed by topping up the volume of the solution being filtered may be necessitated to remove the impurity. The number of rounds required can be assessed by detecting whether any of the unreacted reagent appears in the flowthrough of the centrifugal filtration process, as standard in the art.

Smaller proteins will naturally be much harder to separate from the label/linker components by relatively facile centrifugal filtration, and in this case more complicated chromatography techniques may need to be performed, such as size exclusion chromatography. Where the mass of the SBP and the mass of the mass-tagged SBP do not differ significantly, the purification of the labelled from the unlabelled SBP at the end of the reaction can be more difficult. Accordingly, it is here when one of the major advantages of click chemistry reactions as discussed herein become apparent: the high yields and efficiencies of the click chemistry steps ensures that unreacted SBP is kept to the minimum possible.

Alternatively, unreacted functional groups on the SBP (or SBP-linker component or SBP-linker as discussed above) might be reacted with a further bifunctional linker comprising e.g. a biotin as one of the functionalities. Here therefore, and SBP that has not been attached to the mass tag may be pulled out of the solution by incubation with an avidin (or derivative thereof) coated solid support, such as an avidin-coated bead.

Metal-catalyst free catalysis refers only to the presence of metals acting as catalysts at the stage of the reaction, not the absence of metal *per se.* As noted in the preceding paragraphs, it may be possible to load the mass tag component used to form the mass-tagged SBPs of certain aspects of the disclosure before reaction with the SBP, and so the reaction mixture may contain metal atoms bound within the mass tag component (e.g. chelated to pendant groups branching out form a polymer backbone). When bound in the mass tag, such metals would not act as a catalyst even though they could potentially have an effect as a free ion; thus the reaction would is still metal-catalyst free.

Certain aspects of the present disclosure therefore also provide a mass-tagged SBP, comprising an SBP and a mass tag, wherein the SBP and the mass tag are joined by a covalent linker wherein the linker is formed at least in part by a click chemistry reaction product.

### Mass tags

The mass tag used in the certain aspects of the present disclosure can take a number of forms. Typically, the tag comprises at least one labelling atom. A labelling atom is discussed herein below.

Accordingly, in its simplest form, the mass tag may comprise a metal-chelating moiety which is a metal-chelating group with a metal labelling atom co-ordinated in the ligand. In some instances, detecting only a single metal atom per mass tag may be sufficient. However, in other instances, it may be desirable of each mass tag to contain more than one labelling atom. This can be achieved in a number of ways, as discussed below.

A first means to generate a mass tag that can contain more than one labelling atom is the use of a polymer comprising metal-chelating ligands attached to more than one subunit of the polymer. The number of metal-chelating groups capable of binding at least one metal atom in the polymer can be between approximately 1 and 10,000, such as 5-100, 10-250, 250-5,000, 500-2,500, or 500-1,000. At least one metal atom can be bound to at least one of the metal-chelating groups. The polymer can have a degree of polymerization of between approximately 1 and 10,000, such as 5-100, 10-250, 250-5,000, 500-2,500, or 500-1,000. Accordingly, a polymer based mass tag can comprise between approximately 1 and 10,000, such as 5-100, 10-250, 250-5,000, 500-2,500, or 500-1,000 labelling atoms.

The polymer can be selected from the group consisting of linear polymers, copolymers, branched polymers, graft copolymers, block polymers, star polymers, and hyperbranched polymers. The backbone of the polymer can be derived from substituted polyacrylamide, polymethacrylate, or polymethacrylamide and can be a substituted derivative of a homopolymer or copolymer of acrylamides, methacrylamides, acrylate esters, methacrylate esters, acrylic acid or methacrylic acid. The polymer can be synthesised from the group consisting of reversible addition fragmentation polymerization (RAFT), atom transfer radical polymerization (ATRP) and anionic polymerization. The step of providing the polymer can comprise synthesis of the polymer from compounds selected from the group consisting of N-alkyl acrylamides, N,N-dialkyl acrylamides, N-aryl acrylamides, N-alkyl methacrylamides, N,N-dialkyl methacrylamides, Naryl methacrylamides, methacrylate esters, acrylate esters and functional equivalents thereof.

The polymer can be water soluble. This moiety is not limited by chemical content. However, it simplifies analysis if the skeleton has a relatively reproducible size (for example, length, number of tag atoms, reproducible dendrimer character, etc.). The requirements for stability, solubility, and non-toxicity are also taken into consideration. Thus, the preparation and characterization of a functional water soluble polymer by a synthetic strategy that places many functional groups along the backbone plus a different reactive group (the linking group), that can be used to attach the polymer to a molecule (for example, an SBP), through a linker and optionally a spacer. The size of the polymer is controllable by controlling the polymerisation reaction. Typically the size of the polymer will be chosen so as the radiation of gyration of the polymer is as small as possible, such as between 2 and 11 nanometres. The length of an IgG antibody, an exemplary SBP, is approximately 10 nanometres, and therefore an excessively large polymer tag in relation to the size of the SBP may sterically interfere with SBP binding to its target.

The metal-chelating group that is capable of binding at least one metal atom can comprise at least four acetic acid groups. For instance, the metal-chelating group can be a diethylenetriaminepentaacetate (DTPA) group or a 1,4,7,10-tetraazacyclododecane-1,4,7,10-tetraacetic acid (DOTA) group. Alternative groups include Ethylenediaminetetraacetic acid (EDTA) and ethylene glycol-bis(β-aminoethyl ether)-N,N,N',N'-tetraacetic acid (EGTA)

The metal-chelating group can be attached to the polymer through an ester or through an amide. Examples of suitable metal-chelating polymers include the X8 and DM3 polymers available from Fluidigm Canada, Inc.

The polymer can be water soluble. Because of their hydrolytic stability, N-alkyl acrylamides, N-alkyl methacrylamides, and methacrylate esters or functional equivalents can be used. A degree of polymerization (DP) of approximately 1 to 1000 (1 to 2000 backbone atoms) encompasses most of the polymers of interest. Larger polymers are in the scope of the present disclosure with the same functionality and are possible as would be understood by practitioners skilled in the art. Typically the degree of polymerization will be between 1 and 10,000, such as 5-100, 10-250, 250-5,000, 500-2,500, or 500-1,000. The polymers may be amenable to synthesis by a route that leads to a relatively narrow polydispersity. The polymer may be synthesized by atom transfer radical polymerization (ATRP) or reversible addition-fragmentation (RAFT) polymerization, which should lead to values of Mw (weight average molecular weight)/Mn (number average molecular weight) in the range of 1.1 to 1.2. An alternative strategy involving anionic polymerization, where polymers with Mw/Mn of approximately 1.02 to 1.05 are obtainable. Both methods permit control over end groups, through a choice of initiating or terminating agents. This allows synthesizing polymers to which the linker can be attached. A strategy of preparing polymers containing functional pendant groups in the repeat unit to which the liganded transition metal unit (for example a Ln unit) can be attached in a later step can be adopted. This embodiment has several advantages. It avoids complications that might arise from carrying out polymerizations of ligand containing monomers.

To minimize charge repulsion between pendant groups, the target ligands for (M³⁺) should confer a net charge of -1 on the chelate.

Polymers that be used in certain aspects of the present disclosure include:
- random copolymer poly(DMA-co-NAS): The synthesis of a 75/25 mole ratio random copolymer of N-acryloxysuccinimide (NAS) with N,N-dimethyl acrylamide (DMA) by RAFT with high conversion, excellent molar mass control in the range of 5000 to 130,000, and with Mw/Mn ≈ 1.1 is reported in Relógio et al. (2004) (Polymer, 45, 8639-49). The active NHS ester is reacted with a metal-chelating group bearing a reactive amino group to yield the metal-chelating copolymer synthesised by RAFT polymerization.
- poly(NMAS): NMAS can be polymerised by ATRP, obtaining polymers with a mean molar mass ranging from 12 to 40 KDa with Mw/Mn of approximately 1.1 (see e.g. Godwin et al., 2001; Angew. Chem.Int.Ed, 40: 594-97).
- poly(MAA): polymethacrylic acid (PMAA) can be prepared by anionic polymerization of its t-butyl or trimethylsilyl (TMS) ester.
- poly(DMAEMA): poly(dimethylaminoethyl methacrylate) (PDMAEMA) can be prepared by ATRP (see Wang et al, 2004, J.Am.Chem.Soc, 126, 7784-85). This is a well-known polymer that is conveniently prepared with mean Mn values ranging from 2 to 35 KDa with Mw/Mn of approximately 1.2 This polymer can also be synthesized by anionic polymerization with a narrower size distribution.
- polyacrylamide, or polymethacrylamide.

The metal-chelating groups can be attached to the polymer by methods known to those skilled in the art, for example, the pendant group may be attached through an ester or through an amide. For instance, to a methylacrylate based polymer, the metal-chelating group can be attached to the polymer backbone first by reaction of the polymer with ethylenediamine in methanol, followed by subsequent reaction of DTPA anhydride under alkaline conditions in a carbonate buffer.

A second means is to generate nanoparticles which can act as mass tags. A first pathway to generating such mass tags is the use of nanoscale particles of the metal which have been coated in a polymer. Here, the metal is sequestered and shielded from the environment by the polymer, and does not react when the polymer shell can be made to react e.g. by functional groups incorporated into the polymer shell. The functional groups can be reacted with linker components (optionally incorporating a spacer) to attach click chemistry reagents, so allowing this type of mass tag to plug in to the synthetics strategies discussed above in a simple, modular fashion.

Grafting-to and grafting-from are the two principle mechanism for generating polymer brushes around a nanoparticle. In grafting to, the polymers are synthesised separately, and so synthesis is not constrained by the need to keep the nanoparticle colloidally stable. Here reversible addition-fragmentation chain transfer (RAFT) synthesis has excelled due to a large variety of monomers and easy functionalization. The chain transfer agent (CTA) can be readily used as functional group itself, a functionalized CTA can be used or the polymer chains can be postfunctionalized. A chemical reaction or physisorption is used to attach the polymers to the nanoparticle. One drawback of grafting-to is the usually lower grafting density, due to the steric repulsion of the coiled polymer chains during attachment to the particle surface. All grafting-to methods suffer from the drawback that a rigorous workup is necessary to remove the excess of free ligand from the functionalized nanocomposite particle. This is typically achieved by selective precipitation and centrifugation. In the grafting-from approach molecules, like initiators for atomic transfer radical polymerization (ATRP) or CTAs for (RAFT) polymerizations, are immobilized on the particle surface. The drawbacks of this method are the development of new initiator coupling reactions. Moreover, contrary to grafting-to, the particles have to be colloidally stable under the polymerization conditions.

An additional means of generating a mass tag is via the use of doped beads. Chelated lanthanide (or other metal) ions can be employed in nanoemulsion polymerization to create polymer particles with the chelated lanthanide ions embedded in the polymer. The chelating groups are chosen, as is known to those skilled in the art, in such a way that the metal chelate will have negligible solubility in water but reasonable solubility in the monomer for nanoemulsion polymerization. Typical monomers that one can employ are styrene, methylstyrene, various acrylates and methacrylates, among others as is known to those skilled in the art. For mechanical robustness, the metal-tagged particles have a glass transition temperature (Tg) above room temperature. In some instances, core-shell particles are used, in which the metal-containing particles prepared by nanoemulsion polymerization are used as seed particles for a seeded emulsion polymerization to control the nature of the surface functionality. Surface functionality can be introduced through the choice of appropriate monomers for this secondstage polymerization. Additionally, acrylate (and possible methacrylate) polymers are advantageous over polystyrene particles because the ester groups can bind to or stabilize the unsatisfied ligand sites on the lanthanide complexes. An exemplary method for making such doped beads is: (a) combining at least one labelling atom-containing complex in a solvent mixture comprising at least one organic monomer (such as styrene and/or methyl methacrylate in one embodiment) in which the at least one labelling atom-containing complex is soluble and at least one different solvent in which said organic monomer and said at least one labelling atom-containing complex are less soluble, (b) emulsifying the mixture of step (a) for a period of time sufficient to provide a uniform emulsion; (c) initiating polymerization and continuing reaction until a substantial portion of monomer is converted to polymer; and (d) incubating the product of step (c) for a period of time sufficient to obtain a latex suspension of polymeric particles with the at least one labelling atom-containing complex incorporated in or on the particles therein, wherein said at least one labelling atom-containing complex is selected such that upon interrogation of the polymeric mass tag, a distinct mass signal is obtained from said at least one labelling atom. By the use of two or more complexes comprising different labelling atoms, doped beads can be made comprising two or more different labelling atoms. Furthermore, controlling the ration of the complexes comprising different labelling atoms, allows the production of doped beads with different ratios of the labelling atoms. By use of multiple labelling atoms, and in different ratios, the number of distinctively identifiable mass tags is increased. In core-shell nanobeads, this may be achieved by incorporating a first labelling atom-containing complex into the core, and a second labelling atom-containing complex into the shell.

A further means is the generation of a polymer that include the labelling atom in the backbone of the polymer rather than as a co-ordinated metal ligand. For instance, Carerra and Seferos (Macromolecules 2015, 48, 297-308) disclose the inclusion of tellurium into the backbone of a polymer. Other polymers incorporating atoms capable as functioning as labelling atoms tin-, antimony- and bismuth-incorporating polymers. Such molecules are discussed *inter alia* in Priegert et al., 2016 (Chem. Soc. Rev., 45, 922-953).

Thus the mass tag can comprise at least two components: the labelling atoms, and a polymer, which either chelates, contains or is doped with the labelling atom. In addition, the mass tag comprises an attachment group (when not-conjugated to the SBP), which forms part of the chemical linkage between the mass tag and the SBP following reaction of the two components, in a click chemistry reaction in line with the discussion above.

### Metal-binding proteins and peptides

Biological systems (bacteria and eukaryotic cells) express metal-binding proteins and peptides, which are involved in a wide range of important biological process. Accordingly, such proteins and peptides can be used as mass tags as discussed herein as the metals in these molecules can be detected by a mass detector (the metal they contain is used as a labelling atom as discussed herein). The metal-containing protein or peptide can be attached to the SBP via the same techniques other mass tags described herein, using naturally occurring reactive groups on the protein or peptide or following appropriate functionalisation of the metal-binding protein or peptide if desired. Where both SBP and mass tag are proteins, then typically the SBP will be reacted an NHS ester-based reagent, reactive with amine functionalities and providing one half of the reactive groups in a click chemistry reaction (e.g. tetrazine). The metal binding protein or peptide would then be reacted with a second NHS-based reagent, reactive with amine functionalities and providing the other half of the reactive groups in a click chemistry reaction (e.g. strained cyclooctene). An exemplary range of such metal-binding proteins and peptides is set out below. Accordingly, in some embodiments disclosed herein, the mass tag is a metal-binding protein or peptide.

### Background to metal-binding proteins and peptides

Metal ions can be essential for the correct tertiary structure of a metal-binding protein or peptide. Some metal-binding proteins or peptides are enzymes. Metal ions may be bound to an enzyme's active site and be essential for correct enzymatic activity. Some metal-binding proteins and peptides bind to individual metal ions, whereas others bind to clusters of metal ions. Metal-binding proteins or peptides are responsible for catalyzing processes such as photosynthesis and respiration. Metal-binding proteins or peptides are also involved in processes such as heavy metal sequestration, accumulation and toxicity reduction.

Metal-binding sites in metal-binding proteins and peptides are varied in their coordination numbers and geometries, their metal preferences and their ligands. Despite the variation in metal-binding proteins and peptides, some common features have been identified, for example metal-binding sites are generally centred in regions of hydrophobicity (PNAS, 1990, Vol. 87, pp. 5648-5652). Typically, the metal ions are coordinated by donor groups. Examples of donor groups include nitrogen, oxygen or sulphur centres in the amino acids of the protein or peptide. Donor groups may be in the side chain of amino acid residues or in the backbone of the protein or peptide.

A variety of different metals are present in proteins in nature, for instance zinc is present in a series of transcription factors and carbonic anhydrase. Molybdenum is present in nitrogenase (which fixes atmospheric nitrogen). Copper is present in various proteins, as is calcium, particularly in proteins associated with signalling.

### Exemplary metal-binding proteins and peptides of use

Given the range of different functions in which metal-binding proteins are involved, metals in metal binding proteins are common in biological samples. Accordingly, when metal-binding proteins and peptides are used as mass tags according to certain aspects of the present disclosure, as understood by the skilled person, an assessment will need to be made to determine the prevalent naturally occurring metals in the tissue/cell to be analysed (e.g. tissue and cells derived from liver will be rich in iron). A metal binding protein or peptide should thus be chosen as the mass tag which comprises a metal that is distinguishable over the naturally occurring metals (e.g. different element or isotope).

Where the metal-binding protein used as a mass tag is an enzyme, typically, enzymatic activity by the enzyme is not desired. Accordingly, the mass-tagged SBP comprising the metal-binding protein will be used under conditions which prevent or limit enzymatic acivity. Alternatively, the metal-binding protein may be a mutated version of a naturally occurring enzyme, mutated so that it still binds metal but is not catalytically active.

Metal-binding proteins which have a greater range of use will be those capable of binding to metals which are not typically found in tissues, for instance the proteins which normally bind to heavy metals in the case of metal poisoning, which can be loaded with these metals prior to or following conjugation of a metal binding protein or peptide (as a mass tag) to an SBP. Accordingly, in some embodiments the metal binding protein or peptide is bound to a heavy metal.

Metallothioneins are a family of sulphydryl-rich, low molecular weight proteins that are ubiquitous in animal tissues. Metallothioneins can be characterized by their unusually high cysteine content (typically 30%) and lack of aromatic amino acids. However, there is little structural homology between metallothioneins. Metallothioneins have important functions in cell growth and differentiation, as well as an important role as anti-oxidants (World J. Gastroenterol., 2007, 13(7): 993-996). Metallothioneins bind to a wide range of metals, including cadmium, zinc, copper, cobalt, silver and mercury. They have also been shown to bind to several other metals in vitro, including: Ag(I), Au(I), Bi(III), Co(II), Fe(II), Hg(II), Ni(II), and Pt(II). In particular metallothioneins have a strong affinity to cadmium (J. Biol. Chem., 1985, 260(9): 5342-5350). Thus metallothioneins are particularly useful as mass tags because of the wide range of different metals labelling atoms that can be bound, which would be understood by the skilled person to be selectable so that the labelling atom in a metallothionein mass tag can be detected against the background of metals naturally present in the particular sample to be analysed. Accordingly, in some embodiments, the metal-binding protein or peptide is a metallothionein.

Phytochelatins are found in plants. Phytochelatins are a family of proteins which consist of the amino acids Glu, Cys and Gly. The Glu and Cys residues are linked through a gammacarboxylamide bond. The phytochelatin family is made up of polymers with different repetitions of the Glu-Cys dipeptide, although there are some structural variants. Phytochelatins are structurally related to Glutathione. Phytochelatins are metal ion chelators and have an important role in heavy metal detoxification (Plant Physiol., 2000, 123(3):825-32). Accordingly, in some embodiments, the metal-binding protein or peptide is a phytochelatin.

The metal vanadium is bound by only a few protein species, such as vanabin proteins from the blood cells of ascidians and tunicates. Accordingly, it may be used to label e.g. mammalian cells due to its absence from such cells in significant quantities.As set out above, the metal ion specificity of naturally occurring metal-binding proteins and peptides varies greatly. Some metal-binding proteins and peptides bind to metal ions non-specifically, whereas others only bind to specific metal ions. Short synthetic metal-binding peptides have been made that only bind to one type of heavy metal, for example mercury (Biopolymers, 2002, 64(4):187-97). Accordingly, in some embodiments, the metal-binding peptide is a non-naturally occurring peptide. In some embodiments, multiple metal-binding peptides of particular specificities can be joined together in a single polypeptide chain. In this way, multiple different elements/isotopes can be bound by a single polypeptide, thus enabling the generation of combinatorial labels; increasing the number of different unique tags that can be produced using metal-binding peptides. Further reagents of this kind may be derived from proteins comprising clusters of different metals.

In certain embodiments, the metal(s) bound by the one or more metal-binding proteins or peptides is (are) of greater than 80 amu, greater than 90 amu, or greater than 100 amu. In particular, the use of metal binding proteins or peptides binding metals of above 80 amu is used when the sample is being analysed by an apparatus using an ICP maintained in argon gas as the means for ionising sample material for detection. In some embodiments, the metal binding protein or peptide comprises a metal of mass greater than 80 amu which is not a lanthanide.

A metal binding protein may be bound to metals of a particular isotope. As such, the same metal binding protein bound different isotopes of its metal may be used to tag different SBPs (e.g., different antibodies).

Kits according to certain aspects of the present disclosure, such as defined elsewhere herein at page 61, may include a plurality of metal binding proteins, each attached to (or functionalized so as to be attachable to) an SBP such as a particular antibody. Certain kits may have the same metal binding protein attached to different SBPs, wherein each metal binding protein attached to a different SBP is bound to a different isotope of the same metal (e.g., at least at 80%, 90%, 95%, or 99% isotopic purity). The kit may further comprise metal binding proteins bound to different metals (or isotopes therof), each attached to (or functionalized to attach to) an SBP such as a particular antibody.

A kit comprising at least one metal binding protein attached to (or functionalized to attach to) an SBP may further comprise at least one polymer (e.g., linear or branched) comprising metal in binding pendant groups (e.g., metal atoms bound to DTPA or DOTA chelation groups) or comprising metal in the backbone of the polymer. The polymer may similarly be attached to (or functionalized so as to be attachable to) an SBP such as an antibody. In certain aspects, the polymer may comprise one or more lanthanides, while the metal binding protein my bind to a metal other than a lanthanide. In certain aspects, both the at least one metal binding protein and the at least one polymer are attached to (or functionalized so as to be attachable to) an SBP by the same conjugation chemistry (such as one of the click chemistry reactions described herein).

Embodiments of the disclosure include methods of staining a biological sample with a plurality of metal binding protein tagged SBPs in addition contacting the sample with one or more additional reagents. The additional reagents may include one or more of a metallodrug, metal containing histochemical stain, and non-protein metal containing polymer tagged SBPs as described herein. The additional reagents may each comprise a metal of different isotopic mass from each other and from the metal binding protein tagged SBPs. Certain aspects of the present disclosure also provide samples stained with a SBP conjugated to a metal-binding protein.

Thus the disclosure also relates to new uses of metal-binding proteins or peptides. Certain aspects of the present disclosure provide the use of a metal-binding protein or peptide in the labelling of a sample for mass cytometry, including imaging mass cytometry. Certain aspects of the present disclosure also provide use of a mass-tagged SBP comprising a metal-binding protein or peptide in the labelling of a sample for mass cytometry, including imaging mass cytometry. Certain aspects of the present disclosure also provide the use of a metal-binding protein or peptide in the production of a mass-tagged SBP comprising a metal-binding protein or peptide. Specific exemplary methods of manufacturing such mass-tagged SBPs comprising a metal-binding protein or peptide using click chemistry are set out on page 24 above.

### Labelling atom

Labelling atoms that can be used with the disclosure include any species that are detectable by mass spectrometry (MS) or optical emission spectrometry (OES) and that are substantially absent from the unlabelled tissue sample. Thus, for instance, ¹²C atoms would be unsuitable as labelling atoms because they are naturally abundant, whereas ¹¹C could in theory be used for MS because it is an artificial isotope which does not occur naturally. Often the labelling atom is a metal. In preferred embodiments, however, the labelling atoms are transition metals, such as the rare earth metals (the 15 lanthanides, plus scandium and yttrium). These 17 elements (which can be distinguished by OES and MS) provide many different isotopes which can be easily distinguished (by MS). A wide variety of these elements are available in the form of enriched isotopes e.g. samarium has 6 stable isotopes, and neodymium has 7 stable isotopes, all of which are available in enriched form. The 15 lanthanide elements provide at least 37 isotopes that have non-redundantly unique masses. Examples of elements that are suitable for use as labelling atoms include Lanthanum (La), Cerium (Ce), Praseodymium (Pr), Neodymium (Nd), Promethium (Pm), Samarium (Sm), Europium (Eu), Gadolinium, (Gd), Terbium (Tb), Dysprosium (Dy), Holmium (Ho), Erbium (Er), Thulium (Tm), Ytterbium (Yb), Lutetium (Lu), Scandium (Sc), and Yttrium (Y). In addition to rare earth metals, other metal atoms are suitable for detection e.g. gold (Au), platinum (Pt), iridium (Ir), rhodium (Rh), bismuth (Bi), *etc.* The use of radioactive isotopes is not preferred as they are less convenient to handle and are unstable e.g. Pm is not a preferred labelling atom among the lanthanides.

In order to facilitate time-of-flight (TOF) analysis (as discussed herein) it is helpful to use labelling atoms with an atomic mass within the range 80-250 e.g. within the range 80-210, or within the range 100-200. This range includes all of the lanthanides, but excludes Sc and Y. The range of 100-200 permits a theoretical 101-plex analysis by using different labelling atoms, while taking advantage of the high spectral scan rate of TOF MS. As mentioned above, by choosing labelling atoms whose masses lie in a window above those seen in an unlabelled sample (e.g. within the range of 100-200), TOF detection can be used to provide rapid imaging at biologically significant levels.

Various numbers of labelling atoms can be attached to a single SBP member dependent upon the mass tag used (and so the number of labelling atoms per mass tag) and the number of mass tags that are attached to each SBP). Greater sensitivity can be achieved when more labelling atoms are attached to any SBP member. For example, greater than 10, 20, 30, 40, 50, 60, 70, 80, 90 or 100 labelling atoms can be attached to a SBP member, such as up to 10,000, for instance as 5-100, 10-250, 250-5,000, 500-2,500, or 500-1,000 labelling atoms. As noted above, monodisperse polymers containing multiple monomer units may be used, each containing a chelator such as diethylenetriaminepentaacetic acid (DTPA) or DOTA. DTPA, for example, binds 3+ lanthanide ions with a dissociation constant of around 10⁻⁶ M. These polymers can terminate in a thiol which can be used for attaching to a SBP via reaction of that with a maleimide to attach a click chemistry reactivity in line with those discussed above. Other functional groups can also be used for conjugation of these polymers e.g. amine-reactive groups such as N-hydroxy succinimide esters, or groups reactive against carboxyls or against an antibody's glycosylation. Any number of polymers may bind to each SBP. Specific examples of polymers that may be used include straight-chain ("X8") polymers or third-generation dendritic ("DN3") polymers, both available as MaxPar^{™} reagents. Use of metal nanoparticles can also be used to increase the number of atoms in a label, as also discussed above.

In some embodiments, all labelling atoms in a mass tag are of the same atomic mass. Alternatively, a mass tag can comprise labelling atoms of differing atomic mass. Accordingly, in some instances, a labelled sample may be labelled with a series of mass-tagged SBPs each of which comprises just a single type of labelling atom (wherein each SBP binds its cognate target and so each kind of mass tag is localised on the sample to a specific e.g. antigen). Alternatively, in some instance, a labelled sample may be labelled with a series of mass-tagged SBPs each of which comprises a mixture of labelling atoms. In some instances, the mass-tagged SBPs used to label the sample may comprise a mix of those with single labelling atom mass tags and mixes of labelling atoms in their mass tags.

### Spacer

As noted above, in some instances, the SBP is conjugated to a mass tag through a linker which comprises a spacer. There may be a spacer between the SBP and the click chemistry reagent (e.g. between the SBP and the strained cycloalkyne (or azide); strained cycloalkene (or tetrazine); etc.). There may be a spacer between the between the mass tag and the click chemistry reagent (e.g. between the mass tag and the azide (or strained cycloalkyne); tetrazine (or strained cycloalkene); etc.). In some instances there may be a spacer both between the SNP and the click chemistry reagent, and the click chemistry reagent and the mass tag.

The spacer might be a polyethylene glycol (PEG) spacer, a poly(N-vinylpyrolide) (PVP) spacer, a polyglycerol (PG) spacer, poly(N-(2-hydroxylpropyl)methacrylamide) spacer, or a polyoxazoline (POZ, such as polymethyloxazoline, polyethyloxazoline or polypropyloxazoline) or a C5-C20 non-cyclic alkyl spacer. For example, the spacer may be a PEG spacer with 3 or more, 4 or more, 5 or more, 6 or more, 7 or more, 8 or more, 9 or more, 10 or more, 11 or more, 12 or more, 15 or more of 20 or more EG (ethylene glycol) units. The PEG linker may have from 3 to 12 EG units, from 4 to 10, or may have 4, 5, 6, 7, 8, 9, or 10 EG units. The linker may include cystamine or derivatives thereof, may include one or more disulfide groups, or may be any other suitable linker known to one of skill in the art.

Spacers may be beneficial to minimize the steric effect of the mass tag on the SBP to which is conjugated. Hydrophilic spacers, such as PEG based spacers, may also act to improve the solubility of the mass-tagged SBP and act to prevent aggregation.

### SBPs

Mass cytometry, including imaging mass cytometry is based on the principle of specific binding between members of specific binding pairs. The mass tag is linked to a specific binding pair member, and this localises the mass tag to the target/analyte which is the other member of the pair. Specific binding does not require binding to just one molecular species to the exclusion of others, however. Rather it defines that the binding is not-nonspecific, i.e. not a random interaction. An example of an SBP that binds to multiple targets would therefore be an antibody which recognises an epitope that is common between a number of different proteins. Here, binding would be specific, and mediated by the CDRs of the antibody, but multiple different proteins would be detected by the antibody. The common epitopes may be naturally occurring, or the common epitope could be an artificial tag, such as a FLAG tag. Similarly, for nucleic acids, the a nucleic acid of defined sequence may not bind exclusively to a fully complementary sequence, but varying tolerances of mismatch can be introduced under the use of hybridisation conditions of a differing stringencies, as would be appreciated by one of skill in the art. Nonetheless, this hybridisation is not non-specific, because it is mediated by homology between the SBP nucleic acid and the target analyte. Similarly, ligands can bind specifically to multiple receptors, a facile example being TNFα which binds to both TNFR1 and TNFR2.

The SBP may comprise any of the following: a nucleic acid duplex; an antibody/antigen complex; a receptor/ligand pair; or an aptamer/target pair. Thus a labelling atom can be attached to a nucleic acid probe which is then contacted with a tissue sample so that the probe can hybridise to complementary nucleic acid(s) therein *e.g.* to form a DNA/DNA duplex, a DNA/RNA duplex, or a RNA/RNA duplex. Similarly, a labelling atom can be attached to an antibody which is then contacted with a tissue sample so that it can bind to its antigen. A labelling atom can be attached to a ligand which is then contacted with a tissue sample so that it can bind to its receptor. A labelling atom can be attached to an aptamer ligand which is then contacted with a tissue sample so that it can bind to its target. Thus, labelled SBP members can be used to detect a variety of targets in a sample, including DNA sequences, RNA sequences, proteins, sugars, lipids, or metabolites.

The mass-tagged SBP therefore can be a protein or peptide, or a polynucleotide or oligonucleotide.

Examples of protein SBPs include an antibody or antigen binding fragment thereof, a monoclonal antibody, a polyclonal antibody, a bispecific antibody, a multispecific antibody, an antibody fusion protein, scFv, antibody mimetic, avidin, streptavidin, neutravidin, biotin, or a combination thereof, wherein optionally the antibody mimetic comprises a nanobody, affibody, affilin, affimer, affitin, alphabody, anticalin, avimer, DARPin, Fynomer, kunitz domain peptide, monobody, or any combination thereof, a receptor, such as a receptor-Fc fusion, a ligand, such as a ligand-Fc fusion, a lectin, for example an agglutinin such as wheat germ agglutinin.

The peptide may be a linear peptide, or a cyclical peptide, such as a bicyclic peptide. One example of a peptide that can be used is Phalloidin.

A polynucleotide or oligonucleotide generally refers to a single- or double-stranded polymer of nucleotides containing deoxyribonucleotides or ribonucleotides that are linked by 3 '-5' phosphodiester bonds, as well as polynucleotide analogs. A nucleic acid molecule includes, but is not limited to, DNA, RNA, and cDNA. A polynucleotide analog may possess a backbone other than a standard phosphodiester linkage found in natural polynucleotides and, optionally, a modified sugar moiety or moieties other than ribose or deoxyribose. Polynucleotide analogs contain bases capable of hydrogen bonding by Watson-Crick base pairing to standard polynucleotide bases, where the analog backbone presents the bases in a manner to permit such hydrogen bonding in a sequence-specific fashion between the oligonucleotide analog molecule and bases in a standard polynucleotide. Examples of polynucleotide analogs include, but are not limited to xeno nucleic acid (XNA), bridged nucleic acid (BNA), glycol nucleic acid (GNA), peptide nucleic acids (PNAs), yPNAs, morpholino polynucleotides, locked nucleic acids (LNAs), threose nucleic acid (TNA), 2'-0-Methyl polynucleotides, 2'-0-alkyl ribosyl substituted polynucleotides, phosphorothioate polynucleotides, and boronophosphate polynucleotides. A polynucleotide analog may possess purine or pyrimidine analogs, including for example, 7-deaza purine analogs, 8-halopurine analogs, 5-halopyrimidine analogs, or universal base analogs that can pair with any base, including hypoxanthine, nitroazoles, isocarbostyril analogues, azole carboxamides, and aromatic triazole analogues, or base analogs with additional functionality, such as a biotin moiety for affinity binding.

### Antibody SBP members

In a typical embodiment, the labelled SBP member is an antibody. Labelling of the antibody can be achieved through conjugation of one or more labelling atom binding molecules to the antibody, by attachment of a mass tag using the click chemistry described herein. Antibodies which recognise cellular proteins that are useful for imaging are already widely available for IHC usage, and by using labelling atoms instead of current labelling techniques (e.g. fluorescence) these known antibodies can be readily adapted for use in methods disclosure herein, but with the benefit of increasing multiplexing capability. Antibodies can recognise targets on the cell surface or targets within a cell. Antibodies can recognise a variety of targets *e.g.* they can specifically recognise individual proteins, or can recognise multiple related proteins which share common epitopes, or can recognise specific post-translational modifications on proteins (e.g. to distinguish between tyrosine and phosphor-tyrosine on a protein of interest, to distinguish between lysine and acetyl-lysine, to detect ubiquitination, *etc*.). After binding to its target, labelling atom(s) conjugated to an antibody can be detected to reveal the location of that target in a sample.

The labelled SBP member will usually interact directly with a target SBP member in the sample. In some embodiments, however, it is possible for the labelled SBP member to interact with a target SBP member indirectly e.g. a primary antibody may bind to the target SBP member, and a labelled secondary antibody can then bind to the primary antibody, in the manner of a sandwich assay. Usually, however, the method relies on direct interactions, as this can be achieved more easily and permits higher multiplexing. In both cases, however, a sample is contacted with a SBP member which can bind to a target SBP member in the sample, and at a later stage label attached to the target SBP member is detected.

### Nucleic acid SBPs, and labelling methodology modifications

RNA is another biological molecule which the methods and apparatus disclosed herein are capable of detecting in a specific, sensitive and if desired quantitative manner. In the same manner as described above for the analysis of proteins, RNAs can be detected by the use of a SBP member labelled with an elemental tag that specifically binds to the RNA (e.g. an poly nucleotide or oligonucleotide of complementary sequence as discussed above, including a locked nucleic acid (LNA) molecule of complementary sequence, a peptide nucleic acid (PNA) molecule of complementary sequence, a plasmid DNA of complementary sequence, an amplified DNA of complementary sequence, a fragment of RNA of complementary sequence and a fragment of genomic DNA of complementary sequence). RNAs include not only the mature mRNA, but also the RNA processing intermediates and nascent pre-mRNA transcripts.

In certain embodiments, both RNA and protein are detected using methods of the disclosure.

To detect RNA, cells in biological samples as discussed herein may be prepared for analysis of RNA and protein content using the methods and apparatus described herein. In certain aspects, cells are fixed and permeabilized prior to the hybridization step. Cells may be provided as fixed and/or pemeabilized. Cells may be fixed by a crosslinking fixative, such as formaldehyde, glutaraldehyde. Alternatively or in addition, cells may be fixed using a precipitating fixative, such as ethanol, methanol or acetone. Cells may be permeabilized by a detergent, such as polyethylene glycol (e.g., Triton X-100), Polyoxyethylene (20) sorbitan monolaurate (Tween-20), Saponin (a group of amphipathic glycosides), or chemicals such as methanol or acetone. In certain cases, fixation and permeabilization may be performed with the same reagent or set of reagents. Fixation and permeabilization techniques are discussed by Jamur et al. in "Permeabilization of Cell Membranes" (Methods Mol. Biol., 2010).

Detection of target nucleic acids in the cell, or "in-situ hybridization" (ISH), has previously been performed using fluorophore-tagged oligonucleotide probes. As discussed herein, mass-tagged oligonucleotides, coupled with ionization and mass spectrometry, can be used to detect target nucleic acids in the cell. Methods of in-situ hybridization are known in the art (see Zenobi et al. "Single-Cell Metabolomics: Analytical and Biological Perspectives," Science vol. 342, no. 6163, 2013). Hybridization protocols are also described in US Pat. No. 5,225,326 and US Pub. No. 2010/0092972 and 2013/0164750.

Prior to hybridization, cells present in suspension or immobilized on a solid support may be fixed and permeabilized as discussed earlier. Permeabilization may allow a cell to retain target nucleic acids while permitting target hybridization nucleotides, amplification oligonucleotides, and/or mass-tagged oligonucleotides to enter the cell. The cell may be washed after any hybridization step, for example, after hybridization of target hybridization oligonucleotides to nucleic acid targets, after hybridization of amplification oligonucleotides, and/or after hybridization of mass-tagged oligonucleotides.

Cells can be in suspension for all or most of the steps of the method, for ease of handling. However, the methods are also applicable to cells in solid tissue samples (e.g., tissue sections) and/or cells immobilized on a solid support (e.g., a slide or other surface). Thus, sometimes, cells can be in suspension in the sample and during the hybridization steps. Other times, the cells are immobilized on a solid support during hybridization.

Target nucleic acids include any nucleic acid of interest and of sufficient abundance in the cell to be detected by the subject methods. Target nucleic acids may be RNAs, of which a plurality of copies exist within the cell. For example, 10 or more, 20 or more, 50 or more, 100 or more, 200 or more, 500 or more, or 1000 or more copies of the target RNA may be present in the cell. A target RNA may be a messenger NA (mRNA), ribosomal RNA (rRNA), transfer RNA (tRNA), small nuclear RNA (snRNA), small interfering RNA (siRNA), long noncoding RNA (IncRNA), or any other type of RNA known in the art. The target RNA may be 20 nucleotides or longer, 20 nucleotides or longer, 20 nucleotides or longer, 50 nucleotides or longer, 100 nucleotides or longer, 200 nucleotides or longer, 500 nucleotides or longer, 1000 nucleotides or longer, between 20 and 1000 nucleotides, between 20 and 500 nucleotides in length, between 40 and 200 nucleotides in length, and so forth.

In certain embodiments, a mass-tagged oligonucleotide may be hybridized directly to the target nucleic acid sequence. However, hybridization of additional oligonucleotides may allow for improved specificity and/or signal amplification.

In certain embodiments, two or more target hybridization oligonucleotides may be hybridized to proximal regions on the target nucleic acid, and may together provide a site for hybridization of an additional oligonucleotides in the hybridization scheme.

In certain embodiments, the mass-tagged oligonucleotide may be hybridized directly to the two or more target hybridization oligonucleotides. In other embodiments, one or more amplification oligonucleotides may be added, simultaneously or in succession, so as to hybridize the two or more target hybridization oligonucleotides and provide multiple hybridization sites to which the mass-tagged oligonucleotide can bind. The one or more amplification oligonucleotides, with or without the mass-tagged oligonucleotide, may be provided as a multimer capable of hybridizing to the two or more target hybridization oligonucleotides.

While the use of two or more target hybridization oligonucleotides improves specificity, the use of amplification oligonucleotides increases signal. Two target hybridization oligonucleotides are hybridized to a target RNA in the cell. Together, the two target hybridization oligonucleotides provide a hybridization site to which an amplification oligonucleotide can bind. Hybridization and/or subsequent washing of the amplification oligonucleotide may be performed at a temperature that allows hybridization to two proximal target hybridization oligonucleotides, but is above the melting temperature of the hybridization of the amplification oligonucleotide to just one target hybridization oligonucleotide. The first amplification oligonucleotide provides multiple hybridization sites, to which second amplification oligonucleotides can be bound, forming a branched pattern. Mass-tagged oligonucleotides are bound multiple hybridization sites provided by the second amplification nucleotides. Together, these amplification oligonucleotides (with or without mass-tagged oligonucleotides) are referred to herein as a "multimer". Thus the term "amplification oligonucleotide" includes oligonucleotides that provides multiple copies of the same binding site to which further oligonucleotides can anneal. By increasing the number of binding sites for other oligonucleotides, the final number of labels that can be found to a target is increased. Thus, multiple labelled oligonucleotides are hybridized, indirectly, to a single target RNA. This is enables the detection of low copy number RNAs, by increasing the number of detectable atoms of the element used per RNA.

One particular method for performing this amplification comprises using the RNAscope^{®} method from Advanced cell diagnostics, as discussed in more detail below. A further alternative is the use of a method that adapts the QuantiGene^{®} FlowRNA method (Affymetrix eBioscience). The assay is based on oligonucleotide pair probe design with branched DNA (bDNA) signal amplification. There are more than 4,000 probes in the catalog or custom sets can be requested at no additional charge. In line with the previous paragraph, the method works by hybridization of target hybridization oligonucleotides to the target, followed by the formation of a branched structure comprising first amplification oligonucleotides (termed preamplification oligonucleotides in the QuantiGene^{®} method) to form a stem to which multiple second amplification oligonucleotides can anneal (termed simply amplification oligonucleotides in the QuantiGene^{®} method). Multiple mass-tagged oligonucleotides can then bind.

Another means of amplification of the RNA signal relies on the rolling circle means of amplification (RCA). There are various means why which this amplification system can be introduced into the amplification process. In a first instance, a first nucleic acid is used as the hybridisation nucleic acid wherein the first nucleic acid is circular. The first nucleic acid can be single stranded or may be double-stranded. It comprises as sequence complementary to the target RNA. Following hybridisation of the first nucleic acid to the target RNA, a primer complementary to the first nucleic acid is hybridised to the first nucleic acid, and used for primer extension using a polymerase and nucleic acids, typically exogenously added to the sample. In some instances, however, when the first nucleic acid is added to sample, it may already have the primer for extension hybridised to it. As a result of the first nucleic acid being circular, once the primer extension has completed a full round of replication, the polymerase can displace the primer and extension continues (*i.e*. without 5'→3' exonuclase activity), producing linked further and further chained copies of the complement of the first nucleic acid, thereby amplifying that nucleic acid sequence. Oligonucleotides comprising an elemental tag (RNA or DNA, or LNA or PNA and the like) as discussed above) may therefore be hybridised to the chained copies of the complement of the first nucleic acid. The degree of amplification of the RNA signal can therefore be controlled by the length of time allotted for the step of amplification of the circular nucleic acid.

In another application of RCA, rather than the first, e.g., oligonucleotide that hybridises to the target RNA being circular, it may be linear, and comprise a first portion with a sequence complementary to its target and a second portion which is user-chosen. A circular RCA template with sequence homologous to this second portion may then be hybridised to this the first oligonucleotide, and RCA amplification carried out as above. The use of a first, e.g., oligonucleotide having a target specific portion and user-chosen portion is that the user-chosen portion can be selected so as to be common between a variety of different probes. This is reagent-efficient because the same subsequent amplification reagents can be used in a series of reactions detecting different targets. However, as understood by the skilled person, when employing this strategy, for individual detection of specific RNAs in a multiplexed reaction, each first nucleic acid hybridising to the target RNA will need to have a unique second sequence and in turn each circular nucleic acid should contain unique sequence that can be hybridised by the labelled oligonucleotide. In this manner, signal from each target RNA can be specifically amplified and detected.

Other configurations to bring about RCA analysis will be known to the skilled person, as set out, for instance, in . In some instances, to prevent the first, e.g., oligonucleotide dissociating from the target during the following amplification and hybridisation steps, the first, e.g., oligonucleotide may be fixed following hybridisation (such as by formaldehyde).

Further, hybridisation chain reaction (HCR) may be used to amplify the RNA signal (see, e.g., Choi et al., 2010, Nat. Biotech, 28:1208-1210). Choi explains that an HCR amplifier consists of two nucleic acid hairpin species that do not polymerise in the absence of an initiator. Each HCR hairpin consists of an input domain with an exposed single-stranded toehold and an output domain with a single-stranded toehold hidden in the folded hairpin. Hybridization of the initiator to the input domain of one of the two hairpins opens the hairpin to expose its output domain. Hybridization of this (previously hidden) output domain to the input domain of the second hairpin opens that hairpin to expose an output domain identical in sequence to the initiator. Regeneration of the initiator sequence provides the basis for a chain reaction of alternating first and second hairpin polymerization steps leading to formation of a nicked double-stranded 'polymer'. Either or both of the first and second hairpins can be labelled with an elemental tag in the application of the methods and apparatus disclosed herein. As the amplification procedure relies on output domains of specific sequence, various discrete amplification reactions using separate sets of hairpins can be performed independently in the same process. Thus this amplification also permits amplification in multiplex analyses of numerous RNA species. As Choi notes, HCR is an isothermal triggered self-assembly process. Hence, hairpins should penetrate the sample before undergoing triggered self-assembly in situ, suggesting the potential for deep sample penetration and high signal-to-background ratios

Hybridization may include contacting cells with one or more oligonucleotides, such as target hybridization oligonucleotides, amplification oligonucleotides, and/or mass-tagged oligonucleotides, and providing conditions under which hybridization can occur. Hybridization may be performed in a buffered solution, such as saline sodium-citrate (SCC) buffer, phosphate-buffered saline (PBS), saline-sodium phosphate-EDTA (SSPE) buffer, TNT buffer (having Tris-HCl, sodium chloride and Tween 20), or any other suitable buffer. Hybridization may be performed at a temperature around or below the melting temperature of the hybridization of the one or more oligonucleotides.

Specificity may be improved by performing one or more washes following hybridization, so as to remove unbound oligonucleotide. Increased stringency of the wash may improve specificity, but decrease overall signal. The stringency of a wash may be increased by increasing or decreasing the concentration of the wash buffer, increasing temperature, and/or increasing the duration of the wash. RNAse inhibitor may be used in any or all hybridization incubations and subsequent washes.

A first set of hybridization probes, including one or more target hybridizing oligonucleotides, amplification oligonucleotides and/or mass-tagged oligonucleotides, may be used to label a first target nucleic acid. Additional sets of hybridization probes may be used to label additional target nucleic acids. Each set of hybridization probes may be specific for a different target nucleic acid.

The additional sets of hybridization probes may be designed, hybridized and washed so as to reduce or prevent hybridization between oligonucleotides of different sets. In addition, the mass-tagged oligonucleotide of each set may provide a unique signal. As such, multiple sets of oligonucleotides may be used to detect 2, 3, 5, 10, 15, 20 or more distinct nucleic acid targets.

Sometimes, the different nucleic acids detected are splice variants of a single gene. The mass-tagged oligonucleotide can be designed to hybridize (directly or indirectly through other oligonucleotides as explained below) within the sequence of the exon, to detect all transcripts containing that exon, or may be designed to bridge the splice junctions to detect specific variants (for example, if a gene had three exons, and two splice variants - exons 1-2-3 and exons 1-3 - then the two could be distinguished: variant 1-2-3 could be detected specifically by hybridizing to exon 2, and variant 1-3 could be detected specifically by hybridizing across the exon 1-3 junction.

### SBP compositions

As set out above, the SBPs of certain aspects of the disclosure are produced via a click chemistry reaction. Strain promoted click chemistry reactions do not involve metal catalysts. Strain promoted click chemistry therefore provides particular advantages in the conjugation of mass tags and SBPs. This is because many click chemistry reactions rely on the presence of a metal catalyst. For instance, click chemistry reactions have been reported which are catalysed by copper or iron. Such metals may be incorporated into the components being reacted together (e.g. into metal-ligating groups of the mass tag). This is undesirable at least because occupancy of metal-ligating groups of the mass tag by the metal catalyst prevents the desired labelling atom(s) from occupying the metal-ligating groups. Accordingly, sensitivity is lost in the desired mass channel. Furthermore, background could be increased, if the metal catalysing the reaction were the labelling atom in another mass-tagged SBP used in a multiplex reaction. Furthermore, catalytic metals such as copper are toxic to living cells, and so if some e.g. non-chelated copper remained in the composition of the conjugated mass-tagged SBP this could interfere with subsequent experimental procedures, if those procedures involved living material.

Accordingly, certain aspects of the present disclosure provide a composition comprising a mass-tagged SBP of the present disclosure wherein the composition is free from at least one of copper and iron. In some instances, the composition is free from copper.

### Histochemical Stains

The histochemical stain reagents having one or more intrinsic metal atoms and methods of use described herein may be combined with other reagents and methods of use as described herein. For example, histochemical stains may be colocalized (e.g., at cellular or subcellular resolution) with metal containing drugs, metal-labelled antibodies, and/or accumulated heavy metals. In certain aspects, one or more histochemical stains may be used at lower concentrations (e.g., less than half, a quarter, a tenth, etc.) from what is used for other methods of imaging (e.g., fluorescence microscopy, light microscopy, or electron microscopy).

To visualize and identify structures, a broad spectrum of histological stains and indicators are available and well characterized. The metal-containing stains have a potential to influence the acceptance of the imaging mass cytometry by pathologists. Certain metal containing stains are well known to reveal cellular components, and are suitable for use in the subject disclosure. Additionally, well defined stains can be used in digital image analysis providing contrast for feature recognition algorithms. These features are strategically important for the development of imaging mass cytometry.

Often, morphological structure of a tissue section can be contrasted using affinity products such as antibodies. They are expensive and require additional labelling procedure using metal-containing tags, as compared to using histochemical stains. This approach was used in pioneering works on imaging mass cytometry using antibodies labelled with available lanthanide isotopes thus depleting mass (e.g. metal) tags for functional antibodies to answer a biological question.

The subject disclosure expands the catalog of available isotopes including such elements as Ag, Au, Ru, W, Mo, Hf, Zr. The disclosure relates to compounds such as Ruthenium Red used to identify mucinous stroma, Trichrome stain for identification of collagen fibers, osmium tetroxide as cell counterstain. Silver staining is used in karyotyping. Silver nitrate stains the nucleolar organization region (NOR)-associated protein, producing a dark region wherein the silver is deposited and denoting the activity of rRNA genes within the NOR. Adaptation to IMC may require that the protocols (e.g., oxidation with potassium permanganate and a silver concentration of 1% during) be modified for use lower concentrations of silver solution, e.g., less than 0.5%, 0.01%, or 0.05% silver solution.

Autometallographic amplification techniques have evolved into an important tool in histochemistry. A number of endogenous and toxic heavy metals form sulfide or selenide nanocrystals that can be autocatalytically amplified by reaction with Ag ions. The larger Ag nanocluster can then be readily visualized by IMC. At present, robust protocols for the silver amplified detection of Zn-S/Se nanocrystals have been established as well as detection of selenium through formation of silver-selenium nanocrystals. In addition, commercially available quantum dots (detection of Cd) are also autocatalytically active and may be used as histochemical labels.

Aspects of the subject disclosure may include histochemical stains and their use in imaging by elemental mass spectrometry. Any histochemical stain resolvable by elemental mass spectrometry may be used in the subject disclosure. In certain aspects, the histochemical stain includes one or more atoms of mass greater than a cut-off of the elemental mass spectrometer used to image the sample, such as greater than 60 amu, 80 amu, 100 amu, or 120 amu. For example, the histochemical stain may include a metal tag (e.g., metal atom) as described herein. The metal atom may be chelated to the histochemical stain, or covalently bound within the chemical structure of the histochemical stain. In certain aspects, the histochemical stain may be an organic molecule. Histochemical stains may be polar, hydrophobic (e.g., lipophilic), ionic or may comprise groups with different properties. In certain aspects, a histochemical stain may comprise more than one chemical.

Histochemical stains include small molecules of less than 2000, 1500, 1000, 800, 600, 400, or 200 amu. Histochemical stains may bind to the sample through covalent or non-covalent (e.g., ionic or hydrophobic) interactions. Histochemical stains may provide contrast to resolve the morphology of the biological sample, for example, to help identify individual cells, intracellular structures, and/or extracellular structures. Intracellular structures that may be resolved by histochemical stains include cell membrane, cytoplasm, nucleus, Golgi body, ER, mitochondria, and other cellular organelles. Histochemical stains may have an affinity for a type of biological molecule, such as nucleic acids, proteins, lipids, phospholipids or carbohydrates. In certain aspects, a histochemical stain may bind a molecule other than DNA. Suitable histochemical stains also include stains that bind extracellular structures (e.g., structures of the extracellular matrix), including stroma (e.g., mucosal stroma), basement membrane, interstitial stroma, proteins such as collage or elastin, proteoglycans, non-proteoglycan polysaccharides, extracellular vesicles, fibronectin, laminin, and so forth.

In certain aspects, histochemical stains and/or metabolic probes may indicate a state of a cell or tissue. For example, histochemical stains may include vital stains such as cisplatin, eosin, and propidium iodide. Other histochemical stains may stain for hypoxia, e.g., may only bind or deposit under hypoxic conditions. Probes such as lododeoxyuridine (IdU) or a derivative thereof, may stain for cell proliferation. In certain aspects, the histochemical stain may not indicate the state of the cell or tissue. Probes that detect cell state (e.g., viability, hypoxia and/or cell proliferation) but are administered in-vivo (e.g., to a living animal or cell culture) be used in any of the subject methods but do not qualify as histochemical stains.

Histochemical stains may have an affinity for a type of biological molecule, such as nucleic acids (e.g., DNA and/or RNA), proteins, lipids, phospholipids, carbohydrates (e.g., sugars such as mono-saccharides or di-saccharides or polyols; oligosaccharides; and/or polysaccharides such as starch or glycogen), glycoproteins, and/or glycolipids. In certain aspects the histochemical stain may be a counterstain.

The following are examples of specific histochemical stains and their use in the subject methods:
Ruthenium Red stain as a metal-containing stain for mucinous stroma detection may be used as follows: Immunostained tissue (e.g., de-paraffinized FFPE or cryosection) may be treated with 0.0001-0.5%, 0.001-0.05%, less than 0.1%, less than 0.05%, or around 0.0025% Ruthenium Red (e.g., for at least 5 minutes, at least 10 minutes, at least 30 minutes, or around 30min at 4-42° C, or around room temperature). The biological sample may be rinsed, for example with water or a buffered solution. Tissue may then be dried before imaging by elemental mass spectrometry.

A kit including Ruthenium Red may contain concentrated stock of filtered stain to be diluted prior to application to tissue section, or may contain Rutheinum Red at any of the above describe concentrations.

Phosphotungstic Acid (e.g., as a Trichrome stain) may be used as a metal-containing stain for collagen fibers. Tissue sections on slides (de-paraffinized FFPE or cryosection) may be fixed in Bouin's fluid (e.g., for at least 5 minutes, at least 10 minutes, at least 30 minutes, or around 30 minutes at 4-42° C or around room temperature). The sections may then be treated with 0.0001%-0.01 %, 0.0005%-0.005%, or around 0.001% Phosphotangstic Acid for (e.g., for at least 5 minutes, at least 10 minutes, at least 30 minutes, or around 15 minutes at 4-42° C or around room temperature). Sample may then be rinsed with water and/or buffered solution, and optionally dried, prior to imaging by elemental mass spectrometry. Triichrome stain may be used at a dilution (e.g., 5 fold, 10 fold, 20 fold, 50 fold or great dilution) compared to concentrations used for imaging by light (e.g., fluorescence) microscopy.

A phosphotungstic Acid (Trichrome) containing kit may contain concentrated stock of filtered stain to be diluted prior to application to tissue section, or may contain phototungstic acid at any of the above describe concentrations.

In some embodiments, the histochemical stain is an organic molecule. In some embodiments, the second metal is covalently bound. In some embodiments, the second metal is chelated. In some embodiments, the histochemical stain specifically binds cell membrane. In some embodiments, the histochemical stain is osmium tetroxide. In some embodiments, the histochemical stain is lipophilic. In some embodiments, the histochemical stain specifically binds an extracellular structure. In some embodiments, the histochemical stain specifically binds extracellular collagen. In some embodiments, the histochemical stain is a trichrome stain comprising phosphotungstic/phosphomolybdic acid. In some embodiments, trichrome stain is used after contacting the sample with the antibody, such as at a lower concentration than would be used for optical imaging, for instance wherein the concentration is a 50 fold dilution of trichrome stain or greater.

In some embodiments, the histochemical stain specifically binds stroma, such as wherein the histochemical stain specifically binds mucinous stroma, for instance wherein the histochemical stain is ruthenium red or a derivative thereof.

### Metal-containing Drugs

Metals in medicine is a new and exciting field in pharmacology. Little is known about the cellular structures that are involved in transiently storing metal ions prior to their incorporation into metalloproteins, nucleic acid metal complexes or metal-containing drugs or the fate of metal ions upon protein or drug degradation. An important first step towards unravelling the regulatory mechanisms involved in trace metal transport, storage, and distribution represents the identification and quantification of the metals, ideally in context of their native physiological environment in tissues, cells, or even at the level of individual organelles and subcellular compartments. Histological studies are typically carried out on thin sections of tissue or with cultured cells.

A number of metal-containing drugs are being used for treatment of various diseases, however not enough is known about their mechanism of action or biodistribution: cisplatin, ruthenium imidazole, metallocene-based anti-cancer agents with Mo, tungstenocenes with W, B-diketonate complexes with Hf or Zr, auranofin with Au, polyoxomolybdate drugs. Many metal complexes are used MRI contrast agents (Gd(III) chelates). Characterization of the uptake and biodistribution of metal-based anti-cancer drugs is of critical importance for understanding and minimizing the underlying toxicity.

The atomic masses of certain metals present in drugs fall into the range of mass cytometry. Specifically, cisplatin and others with Pt complexes (iproplatin, lobplatin) are extensively used as a chemotherapeutic drug for treating a wide range of cancers. The nephrotoxicity and myelotoxicity of platinum-based anti-cancer drugs is well known. With the methods and reagents described herein, their subcellular localization within tissue sections, and colocalization with mass- (e.g. metal-) tagged antibodies and/or histochemical stains can now be examined. Chemotherepeutic drugs may be toxic to certain cells, such as proliferating cells, through direct DNA damage, inhibition of DNA damage repair pathways, radioactivity, and so forth. In certain aspects, chemotherapeutic drugs may be targeted to tumor through an antibody intermediate.

In certain aspects, the metal containing drug is a chemotherapeutic drug. Subject methods may include administering the metal containing drug to a living animal, such as an animal research model or human patient as previously described, prior to obtaining the biological sample. The biological sample may be, for example, a biopsy of cancerous tissue or primary cells. Alternatively, the metal containing drug may be added directly to the biological sample, which may be an immortalized cell line or primary cells. When the animal is a human patient, the subject methods may include adjusting a treatment regimen that includes the metal containing drug, based on detecting the distribution of the metal containing drug.

The method step of detecting the metal containing drug may include subcellular imaging of the metal containing drug by elemental mass spectrometry, and may include detecting the retention of the metal containing drug in an intracellular structure (such as membrane, cytoplasm, nucleus, Golgi body, ER, mitochondria, and other cellular organelles) and/or extracellular structure (such as including stroma, mucosal stroma, basement membrane, interstitial stroma, proteins such as collage or elastin, proteoglycans, non-proteoglycan polysaccharides, extracellular vesicles, fibronectin, laminin, and so forth).

A histochemical stain and/or mass- (e.g. metal-) tagged antibody that resolves (e.g., binds to) one or more of the above structures may be colocalized with the metal containing drug to detected retention of the drug at specific intracellular or extracellular structures. For example, a chemotherapeutic drug such as cisplatin may be colocalized with a structure such as collagen. Alternatively or in addition, the localization of the drug may be related to presence of a marker of cell viability, cell proliferation, hypoxia, DNA damage response, or immune response.

In some embodiments, the metal containing drug comprises a non-endogenous metal, such as wherein the non-endogenous metal is platinum, palladium, cerium, cadmium, silver or gold. In certain aspects, the metal containing drug is one of cisplatin, ruthenium imidazole, metallocene-based anti-cancer agents with Mo, tungstenocenes with W, B-diketonate complexes with Hf or Zr, auranofin with Au, polyoxomolybdate drugs, N-myristoyltransferase-1 inhibitor (Tris(dibenzylideneacetone) dipalladium) with Pd, or a derivative thereof. For example the drug may comprise Pt, and may be, for example, cisplatin, carboplatin, oxaliplatin, iproplatin, lobaplatin or a derivative thereof. The metal containing drug may include a non-endogenous metal, such as platinum (Pt), ruthenium (Ru), molybdenum (Mo), tungsten (W), hafnium (Hf), zirconium (Zr), gold (Au), gadolinium (Gd), palladium (Pd) or an isotope thereof. Gold compounds (Auranofin, for example) and gold nanoparticle bioconjugates for photothermal therapy against cancer can be identified in tissue sections.

### Accumulated Heavy Metals

Exposure to heavy metals can occur though injection of food or water, contact through skin, or aerosol intake. Heavy metals may accumulate in soft tissues of the body, such that prolonged exposure has serious health effects. In certain aspect, the heavy metal may be accumulated in vivo, either through controlled exposure in an animal research model or though environmental exposure in a human patient. The heavy metal may be a toxic heavy metal, such as Arsenic (As), Lead (Pb), Antimony (Sb), Bismuth (Bi), Cadmium (Cd), Osmium (Os), Thallium (TI), or Mercury (Hg).

The subject methods may be used to diagnose and/or characterize heavy metal poisoning in a human patient, determine a treatment regimen for a human patient, or characterize accumulation and/or treatment of heavy metals in an animal research model.

For example, detection of heavy metal poisoning in a human patient by the subject methods may be used to determine a treatment to clear the heavy metal, such as by suctioning of the stomach, haemodialysis, treatment with a chelating agent, or treatment with a diuretic. The subject methods may be used to monitor the clearance of accumulated heavy metal in a human patient.

In certain aspects, the methods may include characterizing heavy metal accumulation in an animal research model, such as a rodent exposed to heavy metal under controlled (e.g., measurable) conditions. The effect of treatments on clearance and/or distribution of the heavy metal may be detected by the subject methods. As such, methods include identifying an effect of exposure and/or treatment conditions on the amount and/or subcellular localization of the accumulated heavy metal.

To assess the toxic effect of mercury in central nervous spinal cord of rats exposed to inorganic, organic, and vaporous forms of mercury can be used for imaging by elemental mass spectrometry.

### Samples

Certain aspects of the disclosure provide a method of analysing a biological sample, such as imaging a biological sample. Such samples can comprise a plurality of cells, a plurality of these cells can be subjected to mass cytometry, such as imaging mass cytometry (IMC) in order to provide an image of these cells in the sample. In general, certain aspects of the present disclosure can be used to analyse tissue samples which are now studied by FACS or immunohistochemistry (IHC) techniques, but with the use of labelling atoms which are suitable for detection by mass spectrometry (MS) or optical emission spectrometry (OES).

Any suitable tissue sample can be used in the methods described herein. For example, the tissue can include tissue from one or more of epithelium, muscle, nerve, skin, intestine, pancreas, kidney, brain, liver, blood, bone marrow, buccal swipes, cervical swipes, or any other tissue. Other bodily fluids can be a sample too, such as ascites, lung fluid, spinal fluid, amniotic fluid, blood plasma, blood serum, extracellular fluid, exudate, faeces, urine. Cell lysates can also be analysed as can cell culture supernatants, bacterial culture and/or lysate, viral culture and or culture supernatant. The biological sample may be an immortalized cell line or primary cells obtained from a living subject. For diagnostic, prognostic or experimental (e.g., drug development) purposes the tissue can be from a tumor. In some embodiments, a sample may be from a known tissue, but it might be unknown whether the sample contains tumor cells. Imaging can reveal the presence of targets which indicate the presence of a tumor, thus facilitating diagnosis. Tissue from a tumor may comprise immune cells that are also characterized by the subject methods, and may provide insight into the tumor biology. The tissue sample may comprise formalin-fixed, paraffin-embedded (FFPE) tissue. The tissues can be obtained from any living multicellular organism, such as a mammal, an animal research model (e.g., of a particular disease, such as an immunodeficient rodent with a human tumor xenograft), or a human patient.

The tissue sample may be a section e.g. having a thickness within the range of 2-10 µm, such as between 4-6 µm. Techniques for preparing such sections are well known from the field of IHC e.g. using microtomes, including dehydration steps, fixation, embedding, permeabilization, sectioning etc. Thus, a tissue may be chemically fixed and then sections can be prepared in the desired plane. Cryosectioning or laser capture microdissection can also be used for preparing tissue samples. Samples may be permeabilised e.g. to permit of reagents for labelling of intracellular targets (see above).

The size of a tissue sample to be analysed will be similar to current IHC methods, although the maximum size will be dictated by the laser ablation apparatus, and in particular by the size of sample which can fit into its sample chamber. A size of up to 5 mm x 5 mm is typical, but smaller samples (e.g. 1 mm x 1 mm) are also useful (these dimensions refer to the size of the section, not its thickness).

In addition to being useful for imaging tissue samples, the disclosure can instead be used for imaging of cellular samples such as monolayers of adherent cells or of cells which are immobilised on a solid surface (as in conventional immunocytochemistry). These embodiments are particularly useful for the analysis of adherent cells that cannot be easily solubilized for cell-suspension mass cytometry. Thus, as well as being useful for enhancing current immunohistochemical analysis, the disclosure can be used to enhance immunocytochemistry.

Antibodies and/or histochemical stains, as described above, may allow monitoring of tissue state, such as cell proliferation (e.g., using the target Ki-67 or marker IdU), DNA damage response (e.g., using a marker such as yH2AX), hypoxia (e.g., using the tracer EF5, either as a metal-containing derivative or coupled to a metal-tagged EF5 specific antibody). As described below, the tissue state may be correlated with the presence and/or distribution of metal containing drugs or accumulated heavy metals.

When detecting metal-containing drugs and/or accumulated heavy metals as described below, the biological sample may be obtained from an animal subject. Specifically, the animal subject may be mammalian (e.g., rodent or human), such as an animal research model or a human patient.

Animal research models include any animal genetically engineered and/or put under conditions (e.g., xenograft of a human tumor, exposure to a carcinogen, or exposure to a toxic heavy metal) to induce a diseased state, such as cancer or heavy metal poisoning. In other embodiments, the biological sample is obtained from a human patient, such as a person having or being tested for a cancer or toxic exposure to heavy metal. In either case, the animal subject may be exposed to a chemotherapeutic drug or heavy metal prior to the biological sample being obtained from the animal subject.

Multiplexed detection of metal tags, as described herein, may be used in pulse chase type experiments. Specifically, exposing a living animal or biological sample to metal containing drugs or toxic heavy metals comprising different metal isotope of the same element at different timepoints can be used to monitor the progression of metal retention and/or clearance. In certain aspects, a treatment or change in exposure may coincide with one or more timepoints.

### Labelling of the tissue sample

The disclosure produces samples which have been labelled with labelling atoms, for example a plurality of different labelling atoms, wherein the labelling atoms are detected by an apparatus capable of sampling specific, preferably subcellular, areas of a sample (the labelling atoms therefore represent an elemental tag). The reference to a plurality of different atoms means that more than one atomic species is used to label the sample. These atomic species can be distinguished using a mass detector (e.g. they have different m/Q ratios), such that the presence of two different labelling atoms within a plume gives rise to two different MS signals. The atomic species can also be distinguished using an optical spectrometer *(e.g.* different atoms have different emission spectra), such that the presence of two different labelling atoms within a plume gives rise to two different emission spectral signals.

The methods herein are suitable for the simultaneous detection of many more than two different labelling atoms, permitting multiplex label detection e.g. at least 3, 4, 5, 10, 20, 30, 32, 40, 50 or even 100 different labelling atoms. Labelling atoms can also be used in a combinatorial manner to even further increase the number of distinguishable labels. Giesen et al. 2014 demonstrates the use of 32 different labelling atoms in an imaging method, but laser ablation inductively coupled plasma mass spectrometry (LA-ICP-MS) is intrinsically suitable for parallel detection of higher numbers of different atoms e.g. even over 100 different atomic species, as are the other techniques discussed herein. By labelling different targets with different labelling atoms it is possible to determine the cellular location of multiple targets in a single image.

Labelling the tissue sample generally requires that the labelling atoms are attached to one member of a specific binding pair (SBP). This labelled SBP is contacted with a tissue sample such that it can interact with the other member of the SBP (the target SBP member) if it is present, thereby localising the labelling atom to a specific location in the sample. The method of the disclosure then detects the presence of the labelling atom at this specific location and translates this information into an image in which the target SBP member is present at that location. Rare earth metals and other labelling atoms can be conjugated to SBP members by known techniques e.g. Brückner et al. (2013; Anal. Chem. 86:585-91) describes the attachment of lanthanide atoms to oligonucleotide probes for ICP-MS detection, Gao & Yu (2007; Biosensor Bioelectronics 22:933-40) describes the use of ruthenium to label oligonucleotides, and Fluidigm Canada sells the MaxPar^{™} metal labelling kits which can be used to conjugate over 30 different labelling atoms to proteins (e.g., antibodies including fragments thereof).

As mentioned above, a mass tag comprising one or more labelling atoms is attached to a SBP member, and this mass-tagged SBP member is contacted with the tissue sample where it can find the target SBP member (if present), thereby forming a labelled target SBP (aka a labelled analyte). The target member can comprise any chemical structure that is suitable for attaching to a labelling atom and then for imaging according to the disclosure.

In general terms, methods of the disclosure can be based on any SBP which is already known for use in determining the location of target molecules in tissue samples *(e.g.* as used in IHC or fluorescence in situ hybridisation, FISH), but the SBP member which is contacted with the sample will carry a labelling atom which is detectable by a detector system as described above. Thus the disclosure can readily be implemented by using available IHC and FISH reagents, merely by modifying the labels which have previously been used e.g. to modify a FISH probe to carry a label which can be detected.

The common structure of the mass-tagged SBPs resulting from the commonality of the reaction chemistries used to conjugate the SBPs and mass tags can also have advantages in terms of ensuring that the mass tags are ionised comparably to generate elemental ions when different mass-tagged SBPs are deployed together in a multiplexed reaction. Use of a common conjugation chemistry benefits the highly multiplexed analysis uniquely offered by imaging mass cytometry, as different labelling atoms can be more easily attached to different types of SBPs, allowing for a more customizable and flexible assay design. Accordingly, certain aspects of the present disclosure enable the production of labelled samples in which two or more of the mass-tagged SBP reagents have the same linkage between the mass tag and SBP components of the reagent. Accordingly, certain aspects of the present disclosure provide labelled samples in which two or more of the mass-tagged SBP reagents have the same linkage between the mass tag and SBP components of the reagent. Sometimes, at least 3, at least 4, at least 5, at least 6, at least 7, at least 8, at least 9, at least 10, at least 20, at least 30, at least 40, at least 50, or at least 100 of the mass-tagged SBPs used to stain the stained sample have the same linkage between the mass tag and SBP components of the reagents. In some instances, the SBPs include proteins, comprising the same linkage between the mass tag and SBP components of the reagents. In some instances, the SBPs include nucleic acids, comprising the same linkage between the mass tag and SBP components of the reagents. In some instances the SBPs include nucleic acids and proteins, comprising the same linkage between the mass tag and SBP components of the reagents. In some instances, the SBPs include antibodies, comprising the same linkage between the mass tag and SBP components of the reagents. In some instances the SBPs include nucleic acids and antibodies, comprising the same linkage between the mass tag and SBP components of the reagents. In some instances, the SBPs include antibodies and lectins, comprising the same linkage between the mass tag and SBP components of the reagents. In some instances the SBPs include nucleic acids, lectins and antibodies, comprising the same linkage between the mass tag and SBP components of the reagents. In some instances, the SBPs include peptides, comprising the same linkage between the mass tag and SBP components of the reagents. In some instances the SBPs include nucleic acids and peptides, comprising the same linkage between the mass tag and SBP components of the reagents. In some instances, the SBPs include peptides and antibodies, comprising the same linkage between the mass tag and SBP components of the reagents. In some instances the SBPs include peptides, nucleic acids and antibodies, comprising the same linkage between the mass tag and SBP components of the reagents. In some instances, the SBPs include peptides, antibodies and lectins, comprising the same linkage between the mass tag and SBP components of the reagents. In some instances the SBPs include peptides nucleic acids, lectins and antibodies, comprising the same linkage between the mass tag and SBP components of the reagents. The same linkage may comprise a 1,2,3-triazole, a pyridazine or an isoxazole. In some embodiments of the above, the sample has also been stained with a histochemical stain.

As noted above, the sample may already contain atoms that can be detected in the mass cytometry process (including imaging mass cytometry process), i.e. that are not labelling atoms, for instance wherein the sample is form a subject that has been administered a metal containing drug, or has been environmentally exposed to the metal.

Certain aspects of the present disclosure also provide a method of producing a labelled sample of the disclosure, comprising: providing a sample; and
incubating the sample with more than one mass-tagged SBP, wherein at least two of the mass-tagged SBPs have been formed by the conjugation of the SBP and the mass tag by the same click chemistry reaction, under conditions suitable to allow the SBP to complex with its target SBP member.

In some embodiments, at least two of the mass-tagged SBPs have an SBP component and a mass tag component that are linked by a linker comprising a 1,2,3-triazole. In some embodiments, at least two of the mass-tagged SBPs have SBPs and mass tags that are linked by a linker comprising a pyridazine. In some embodiments, at least two of the mass-tagged SBPs have SBPs and mass tags that are linked by a linker comprising an isoxazole. In some embodiments, at least two of the mass-tagged SBPs have SBPs and mass tags that are linked by a linker comprising a 1,2,3-triazole and at least two of the mass-tagged SBPs have SBPs and mass tags that are linked by a linker comprising a pyridazine. In some embodiments of the method, the SBPs include proteins, comprising the same linkage between the mass tag and SBP components of the reagents. In some embodiments of the method, the SBPs include nucleic acids, comprising the same linkage between the mass tag and SBP components of the reagents. In some embodiments of the method the SBPs include nucleic acids and proteins, comprising the same linkage between the mass tag and SBP components of the reagents. In some embodiments of the method, the SBPs include antibodies, comprising the same linkage between the mass tag and SBP components of the reagents. In some embodiments of the method the SBPs include nucleic acids and antibodies, comprising the same linkage between the mass tag and SBP components of the reagents. In some embodiments of the method, the SBPs include antibodies and lectins, comprising the same linkage between the mass tag and SBP components of the reagents. In some embodiments of the method the SBPs include nucleic acids, lectins and antibodies, comprising the same linkage between the mass tag and SBP components of the reagents. In some embodiments of the method, the SBPs include peptides, comprising the same linkage between the mass tag and SBP components of the reagents. In some embodiments of the method the SBPs include nucleic acids and peptides, comprising the same linkage between the mass tag and SBP components of the reagents. In some embodiments of the method, the SBPs include peptides and antibodies, comprising the same linkage between the mass tag and SBP components of the reagents. In some embodiments of the method the SBPs include peptides, nucleic acids and antibodies, comprising the same linkage between the mass tag and SBP components of the reagents. In some embodiments of the method, the SBPs include peptides, antibodies and lectins, comprising the same linkage between the mass tag and SBP components of the reagents. In some embodiments of the method the SBPs include peptides nucleic acids, lectins and antibodies, comprising the same linkage between the mass tag and SBP components of the reagents. Sometimes, the sample which is labelled in the method may already contain atoms that can be detected in the mass cytometry process (including imaging mass cytometry process), i.e. that are not labelling atoms, for instance wherein the sample is form a subject that has been administered a metal containing drug, or has been environmentally exposed to the metal.

### Kits

Kits of the subject disclosure include reagents that may be used for performing one or more of the method steps described herein. Aspects of the disclosure include kit for use in any of the methods described herein.

Accordingly, certain aspects of the present disclosure provide a kit comprising two or more mass-tagged SBPs of the disclosure, which have the same linkage between the mass tag and SBP components of the reagent. Sometimes the kit comprises, at least 3, at least 4, at least 5, at least 6, at least 7, at least 8, at least 9, at least 10, at least 20, at least 30, at least 40, at least 50, or at least 100 of the mass-tagged SBPs having the same linkage between the mass tag and SBP components of the reagents. In some instances, the SBPs include proteins, comprising the same linkage between the mass tag and SBP components of the reagents. In some instances, the SBPs include nucleic acids, comprising the same linkage between the mass tag and SBP components of the reagents. In some instances the SBPs include nucleic acids and proteins, comprising the same linkage between the mass tag and SBP components of the reagents. In some instances, the SBPs include antibodies, comprising the same linkage between the mass tag and SBP components of the reagents. In some instances the SBPs include nucleic acids and antibodies, comprising the same linkage between the mass tag and SBP components of the reagents. In some instances, the SBPs include antibodies and lectins, comprising the same linkage between the mass tag and SBP components of the reagents. In some instances the SBPs include nucleic acids, lectins and antibodies, comprising the same linkage between the mass tag and SBP components of the reagents. In some instances, the SBPs include peptides, comprising the same linkage between the mass tag and SBP components of the reagents. In some instances the SBPs include nucleic acids and peptides, comprising the same linkage between the mass tag and SBP components of the reagents. In some instances, the SBPs include peptides and antibodies, comprising the same linkage between the mass tag and SBP components of the reagents. In some instances the SBPs include peptides, nucleic acids and antibodies, comprising the same linkage between the mass tag and SBP components of the reagents. In some instances, the SBPs include peptides, antibodies and lectins, comprising the same linkage between the mass tag and SBP components of the reagents. In some instances the SBPs include peptides nucleic acids, lectins and antibodies, comprising the same linkage between the mass tag and SBP components of the reagents. The same linkage may comprise a 1,2,3-triazole, a pyridazine or an isoxazole. In some instances the kit may also comprise a histochemical stain. In some embodiments, the kit may also comprise a mass-tagged non-specific DNA intercalator. In some embodiments, the kit may also contain a metal-containing drug.

In certain aspects, a kit comprises SBPs selected from at least two, at least three, or all four of peptides, nucleic acids, lectins and antibodies. Each SBP may be conjugated by the same click chemistry reaction to a mass tag comprising a different element or isotope.

For instance, a kit may include a drug that includes a first metal, a histochemical stain that specifically binds to an extracellular structure or an intracellular structure other than DNA (wherein the histochemical stain includes a second metal), and an antibody that specifically binds a protein expressed by cells of the biological sample (wherein the antibody is tagged with a third metal; representing a mass-tagged SBP as described herein, such as a mass-tagged SBP of the disclosure). For example, the kit may include a histochemical stain and mass- (e.g. metal-) tagged antibody; a metal-containing drug and a mass- (e.g. metal-) tagged antibody; a metal-containing drug and a histochemical stain; a histochemical stain with a toxic heavy metal (for use in an animal research model); a toxic heavy metal and mass- (e.g. metal-) tagged antibody; or a toxic heavy metal, histochemical stain, and mass- (e.g. metal-) tagged antibody. The metals may be distinguishable from each other elemental mass cytometry.

Kits of the subject disclosure may also include disposables for collecting sample from a living subject, sample preparation reagents as described herein, buffers for suspending dried reagents and/or washing the sample, and/or slides on which to image the biological sample.

### Target Elements and detecting the Distribution of Mass (e.g Metal) Tags

Methods may include detecting the distribution of mass (e.g. metal) tags as described herein. In certain aspects, detecting may include constructing an image (as described further herein) that renders the spatial distribution of the mass (e.g. metal) tags.

Certain methods, kits and/or biological samples may include a plurality of mass (e.g. metal) tags, such as 3 or more, 5 or more, 10 or more, 20 or more, 30 or more, 40 or more, or 50 or more metal tags.

In summary of the above in imaging mass spectrometry, the distribution of one or more target elements (i.e., elements or elemental isotopes) may be of interest. In certain aspects, target elements are labelling atoms as described herein. On other instances, the target element may be an atom that is naturally present in the sample, e.g. the target element may be a metal that is naturally coordinated in the active site of certain enzymes. A labelling atom may be directly added to the sample alone or covalently bound to or within a biologically active molecule. In certain embodiments, labelling atoms (e.g., metal tags) may be conjugated to a member of a specific binding pair (SBP), such as an antibody (that binds to its cognate antigen), aptamer or oligonucleotide for hybridizing to a DNA or RNA target, as describe herein. Labelling atoms may be attached to an SBP by any method known in the art, including the method of the present disclosure. In certain aspects, the labelling atoms are a metal element, such as a lanthanide or transition element or another metal tag as described herein. The metal element may have a mass greater than 60 amu, greater than 80 amu, greater than 100 amu, or greater than 120 amu. Mass spectrometers described herein may deplete elemental ions below the masses of the metal elements, so that abundant lighter elements do not create space-charge effects and/or overwhelm the mass detector.

### Multiplexed analysis

One feature of the disclosure is its ability to detect multiple (e.g. 10 or more, and even up to 100 or more) different target SBP members in a sample e.g. to detect multiple different proteins and/or multiple different nucleic acid sequences. To permit differential detection of these target SBP members their respective SBP members should carry different labelling atoms such that their signals can be distinguished. For instance, where ten different proteins are being detected, ten different antibodies (each specific for a different target protein) can be used, each of which carries a unique label, such that signals from the different antibodies can be distinguished. In some embodiments, it is desirable to use multiple different antibodies against a single target *e.g.* which recognise different epitopes on the same protein. Thus, a method may use more antibodies than targets due to redundancy of this type. In general, however, the disclosure will use a plurality of different labelling atoms to detect a plurality of different targets.

If more than one labelled antibody is used with the disclosure, it is preferable that the antibodies should have similar affinities for their respective antigens, as this helps to ensure that the relationship between the quantity of labelling atoms detected and the abundance of the target antigen in the tissue sample will be more consistent across different SBPs (particularly at high scanning frequencies). Similarly, it is preferable if the labelling of the various antibodies has the same efficiency, so that the antibodies each carry a comparable quantity of the labelling atom.

In some instances, the SBP may carry a fluorescent label as well as an elemental tag. Fluorescence of the sample may then be used to determine regions of the sample, e.g. a tissue section, comprising material of interest which can then be sampled for detection of labelling atoms. E.g. a fluorescent label may be conjugated to an antibody which binds to an antigen abundant on cancer cells, and any fluorescent cell may then be targeted to determine expression of other cellular proteins that are about by SBPs conjugated to labelling atoms.

Where a SBP carries a fluorescent tag in addition to a mass tag, the fluorescent and mass tags may be conjugated to the SBP by different chemistries. For instance, the mass tag may be conjugated using a click chemistry reaction of the disclosure; and the fluorescent tag may be conjugated by the prior art maleimide chemistry to conjugate the fluorescent tag to a sulfhydryl on the SBP. Alternatively, both the fluorescent and mass tags may be conjugated to the SBP by click chemistry.If a target SBP member is located intracellularly, it will typically be necessary to permeabilize cell membranes before or during contacting of the sample with the labels. For example, when the target is a DNA sequence but the labelled SBP member cannot penetrate the membranes of live cells, the cells of the tissue sample can be fixed and permeabilised. The labelled SBP member can then enter the cell and form a SBP with the target SBP member. In this respect, known protocols for use with IHC and FISH can be utilised.

A method may be used detect at least one intracellular target and at least one cell surface target. In some embodiments, however, the disclosure can be used to detect a plurality of cell surface targets while ignoring intracellular targets. Overall, the choice of targets will be determined by the information which is desired from the method, as the disclosure will provide an image of the locations of the chosen targets in the sample.

As described further herein, specific binding partners (i.e., affinity reagents) comprising labelling atoms may be used to stain (contact) a biological sample. Suitable specific binging partners include antibodies (including antibody fragments). Labelling atoms may be distinguishable by mass spectrometry (i.e., may have different masses). Labelling atoms may be referred to herein as mass (e.g. metal) tags when they include one or more metal atoms. Mass (e.g. metal) tags may include a polymer with a carbon backbone and a plurality of pendant groups that each bind a metal atom (e.g., metal-chelating groups loaded with a metal atom). Alternatively, or in addition, metal tags may include a metal nanoparticle. Antibodies may be tagged with a mass (e.g. metal) tag by a covalent or non-covalent interaction.

Antibody stains may be used to image proteins at cellular or subcellular resolution. Aspects of the disclosure include contacting the sample with one or more antibodies that specifically bind a protein expressed by cells of the biological sample, wherein the antibody is tagged with a first mass (e.g. metal) tag. For example, the sample may be contacted with 5 or more, 10 or more, 15 or more, 20 or more, 30 or more, 40 or more, 50 or more antibodies, each with a distinguishable mass (e.g. metal) tag. The sample may further be contacted with one or more histochemical stains before, during (e.g., for ease of workflow), or after (e.g., to avoid altering antigen targets of antibodies) staining the sample with antibodies. The sample may further comprise one or more metal containing drugs and/or accumulated heavy metals as described herein.

Mass- (e.g. metal-) tagged antibodies for use in the subject disclosures may specifically bind a metabolic probe that does not comprise a metal (e.g., EF5). Other mass- (e.g. metal-) tagged antibodies may specifically bind a target (e.g., of epithelial tissue, stromal tissue, nucleus, etc.) of traditional stains used in fluorescence and light microscopy. Such antibodies include anti-cadherin, anti-collagen, anti-keratin, anti-EFS, anti-Histone H3 antibodies, and a number of other antibodies known in the art.

Alternatively or in addition, detecting the distribution of mass (e.g. metal) tags may include measuring the extent of colocalization of two or more mass (e.g. metal) tags (e.g., assigning a value to the degree to which mass (e.g. metal) tags occupy the same or similar location). Such analysis can be useful for identifying subcellular structures at which mass (e.g. metal) tags are accumulated, which may inform understanding of the biology of exposure to the mass (e.g. metal) tags (or chemicals containing the mass (e.g. metal) tags). In certain aspects, the detection of the spatial distribution of mass (e.g. metal) tags may be at subcellular resolution. In certain aspects, some or all of the mass (e.g. metal) tags may not be endogenous to the biological sample.

### Single cell analysis

Methods of the disclosure include laser ablation of multiple cells in a sample, and thus plumes from multiple cells are analysed and their contents are mapped to specific locations in the sample to provide an image. In most cases a user of the method will need to localise the signals to specific cells within the sample, rather than to the sample as a whole. To achieve this, the boundaries of cells (e.g. the plasma membrane, or in some cases the cell wall) in the sample can be demarcated.

Demarcation of cellular boundaries can be achieved in various ways. For instance, a sample can be studied using conventional techniques which can demarcate cellular boundaries, such as microscopy as discussed above. When performing these methods, therefore, an analysis system comprising a camera as discussed above is particularly useful. An image of this sample can then be prepared using a method of the disclosure, and this image can be superimposed on the earlier results, thereby permitting the detected signals to be localised to specific cells. Indeed, as discussed above, in some cases the laser ablation may be directed only to a subset of cells in the sample as determined to be of interest by the use of microscopy based techniques.

To avoid the need to use multiple techniques, however, it is possible to demarcate cellular boundaries as part of the imaging method of the disclosure. Such boundary demarcation strategies are familiar from IHC and immunocytochemistry, and these approaches can be adapted by using labels which can be detected. For instance, the method can involve labelling of target molecule(s) which are known to be located at cellular boundaries, and signal from these labels can then be used for boundary demarcation. Suitable target molecules include abundant or universal markers of cell boundaries, such as members of adhesion complexes *(e.g.* β-catenin or E-cadherin). Some embodiments can label more than one membrane protein in order to enhance demarcation.

In addition to demarcating cell boundaries by including suitable labels, it is also possible to demarcate specific organelles in this way. For instance, antigens such as histones (e.g. H3) can be used to identify the nucleus, and it is also possible to label mitochondrial-specific antigens, cytoskeleton-specific antigens, Golgi-specific antigens, ribosome-specific antigens, *etc.,* thereby permitting cellular ultrastructure to be analysed by methods of the disclosure.

Signals which demarcate the boundary of a cell (or an organelle) can be assessed by eye, or can be analysed by computer using image processing. Such techniques are known in the art for other imaging techniques *e.g.* Arce et al. (2013; Scientific Reports 3, article 2266) describes a segmentation scheme that uses spatial filtering to determine cell boundaries from fluorescence images, reference Ali et al. (2011; Mach Vis Appl 23:607-21) discloses an algorithm which determines boundaries from brightfield microscopy images, reference Pound et al. (2012; The Plant Cell 24:1353-61) discloses the CellSeT method to extract cell geometry from confocal microscope images, and reference Hodneland et al. (2013; Source Code for Biology and Medicine 8:16) discloses the CellSegm MATLAB toolbox for fluorescence microscope images. A method which is useful with the disclosure uses watershed transformation and Gaussian blurring. These image processing techniques can be used on their own, or they can be used and then checked by eye.

Once cellular boundaries have been demarcated it is possible to allocate signal from specific target molecules to individual cells. It can also be possible to quantify the amount of a target analyte(s) in an individual cell e.g. by calibrating the methods against quantitative standards.

### Sample Carrier

In certain embodiments, the sample may be immobilized on a solid support (i.e. a sample carrier), to position it for imaging mass spectrometry. The sample carrier may be optically transparent, for example made of glass or plastic. Where the sample carrier is optically transparent, it enables ablation of the sample material through the support For example, the solid support may include a tissue slide. Alternatively, or in addition, the solid support may include a sample stage (such as a movable stage, also called a translation stage) as described further herein. Sometimes, the sample carrier will comprise features that act as reference points for use with the apparatus and methods described herein, for instance to allow the calculation of the relative position of features/regions of interest that are to be ablated or desorbed and analysed.

### Reference Particles

As described herein, reference particles of known elemental or isotopic composition may be added to the sample (or the sample support) for use as a reference during detection of target elemental ions in the sample. In certain embodiments, reference particles comprise metal elements or isotopes, such as transition metals or lanthanides. For example, reference particles may comprise elements or isotopes of mass greater than 60 amu, greater than 80 amu, greater than 100 amu, or greater than 120 amu.

Target elements, such as labelling atoms, can be normalized within a sample run based on elemental ions detected from individual reference particles. For example, the subject methods may include switching between detecting elemental ions from individual reference particles and detecting only target elemental ions.

### APPARATUS AND TECHNIQUES FOR USE

In general terms, the analyser apparatus disclosed herein comprises two broadly characterised systems for performing imaging elemental mass spectrometry.

The first is a sampling and ionisation system. This system contains a sample chamber, which is the component in which the sample is placed when it is subjected to analysis. The sample chamber comprises a stage, which holds the sample (typically the sample is on a sample carrier, such as a microscope slide, e.g. a tissue section, a monolayer of cells or individual cells, such as where a cell suspension has been dropped onto the microscope slide, and the slide is placed on the stage). The sampling and ionisation system acts to remove material from the sample in the sample chamber (the removed material being called sample material herein) which is converted into ions, either as part of the process that causes the removal of the material from the sample or via a separate ionisation system, downstream of the sampling system.

The ionised material is then analysed by the second system which is the detector system. The detector system can take different forms depending upon the particular characteristic of the ionised sample material being determined, for example a mass detector or an optical emission detector in mass spectrometry-based and optical spectrometer-based analyser apparatus, respectively.

Thus, in operation, the sample is taken into the apparatus, is sampled to generate ionised material (sampling may generate vaporous/particular material, which is subsequently ionised by the ionisation system), and the ions of the sample material are passed into the detector system. Although the detector system can detect many ions, most of these will be ions of the atoms that naturally make up the sample. In some applications, for example analysis of minerals, such as in geological or archaeological applications, this may be sufficient.

In some cases, for example when analysing biological samples, the native element composition of the sample may not be suitably informative. This is because, typically, all proteins and nucleic acids are comprised of the same main constituent atoms, and so while it is possible to tell regions which contain protein/nucleic acid from those that do not contain such proteinaceous or nucleic acid material, it is not possible to differentiate a particular protein from all other proteins. However, by labelling the sample with atoms not present in the material being analysed under normal conditions, or at least not present in significant amounts (for example certain transition metal atoms, such as rare earth metals; see section on labelling below for further detail), specific characteristics of the sample can be determined. In common with IHC and FISH, the detectable labels can be attached to specific targets on or in the sample (such as fixed cells or a tissue sample on a slide), *inter alia* through the use of SBPs such as antibodies or nucleic acids targeting molecules on or in the sample. In order to detect the ionised label, the detector system is used, as it would be to detect ions from atoms naturally present in the sample. By linking the detected signals to the known positions of the sampling of the sample which gave rise to those signals it is possible to generate an image of the atoms present at each position, both the native elemental composition and any labelling atoms. In aspects where native elemental composition of the sample is depleted prior to detection, the image may only be of labelling atoms. The technique allows the analysis of many labels in parallel (also termed multiplexing), which is a great advantage in the analysis of biological samples.

Thus various types of analyser apparatus can be used in practising the disclosure, a number of which are discussed in detail below.

### Analyser apparatus based on mass-detection

### 1. Sampling and ionisation systems

### a. Laser ablation sampling and ionising system

A laser ablation based analyser typically comprises three components. The first is a laser ablation sampling system for the generation of plumes of vaporous and particulate material from the sample for analysis. Before the atoms in the plumes of ablated sample material (including any detectable labelling atoms as discussed below) can be detected the detector system - a mass spectrometer component (MS component; the third component), the sample can be ionised (and atomised). Accordingly, the apparatus comprises a second component which is an ionisation system that ionises the atoms to form elemental ions to enable their detection by the MS component based on mass/charge ratio (some ionisation of the sample material may occur at the point of ablation, but space charge effects result in the almost immediate neutralisation of the charges). The laser ablation sampling system is connected to the ionisation system by a transfer conduit.

### Laser ablation sampling system

In brief summary, the components of a laser ablation sampling system include a laser source that emits a beam of laser radiation that is directed upon a sample. The sample is positioned on a stage within a chamber in the laser ablation sampling system (the sample chamber). The stage is usually a translation stage, so that the sample can be moved relative to the beam of laser radiation whereby different locations on the sample can be sampled for analysis. As discussed below in more detail, gas is flowed through the sample chamber, and the flow of gas carries away the plumes of aerosolised material generated when the laser source ablates the sample, for analysis and construction of an image of the sample based on its elemental composition (including labelling atoms such as labelling atoms from elemental tags). As explained further below, in an alternative mode of action, the laser system of the laser ablation sampling system can also be used to desorb material from the sample.

For biological samples (cells, tissues sections etc.) in particular, the sample is often heterogeneous (although heterogeneous samples are known in other fields of application of the disclosure, i.e. samples of a non-biological nature). A heterogeneous sample is a sample containing regions composed of different materials, and so some regions of the sample can ablate at lower threshold fluence at a given wavelength than the others. The factors that affect ablation thresholds are the absorbance coefficient of the material and mechanical strength of material. For biological tissues, the absorbance coefficient will have a dominant effect as it can vary with the laser radiation wavelength by several orders of magnitude. For instance, in a biological sample, when utilising nanosecond laser pulses a region that contains proteinaceous material will absorb more readily in the 200-230nm wavelength range, while a region containing predominantly DNA will absorb more readily in the 260-280nm wavelength range.

It is possible to conduct laser ablation at a fluence near the ablation threshold of the sample material. Ablating in this manner often improves aerosol formation which in turn can help improve the quality of the data following analysis. Often to obtain the smallest crater, to maximise the resolution of the resulting image, a Gaussian beam is employed. A cross section across a Gaussian beam records an energy density profile that has a Gaussian distribution. In that case, the fluence of the beam changes with the distance from the centre. As a result, the diameter of the ablation spot size is a function of two parameters: (i) the Gaussian beam waist (1/e²), and (ii) the ratio between the fluence applied and the threshold fluence.

Thus, in order to ensure consistent removal of a reproducible quantity of material with each ablative laser pulse, and thus maximise the quality of the imaging data, it is useful to maintain a consistent ablation diameter which in turn means adjusting the ratio of the energy supplied by the laser pulse to the target to the ablation threshold energy of the material being ablated. This requirement represents a problem when ablating a heterogeneous sample where the threshold ablation energy varies across the sample, such as a biological tissue where the ratio of DNA and protein material varies, or in a geological sample, where it varies with the particular composition of the mineral in the region of the sample. To address this, more than one wavelength of laser radiation can be focused onto the same ablation location on a sample, to more effectively ablate the sample based on the composition of the sample at that location.

### Laser system of the laser ablation sampling system

The laser system can be set up to produce single or multiple (i.e. two or more) wavelengths of laser radiation. Typically, the wavelengths of laser radiation discussed refer to the wavelength which has the highest intensity (the "peak" wavelength). If the system produces different wavelengths, they can be used for different purposes, for example, for targeting different materials in a sample (by targeting here is meant that the wavelength chosen is one which is absorbed well by a material).

Where multiple wavelengths are used, at least two of the two or more wavelengths of the laser radiation can be discrete wavelengths. Thus when a first laser source emits a first wavelength of radiation that is discrete from a second wavelength of radiation, it means that no, or a very low level of radiation of the second wavelength is produced by the first laser source in a pulse of the first wavelength, for example, less than 10% of the intensity at the first wavelength, such as less than 5%, less than 4%, less than 3%, less than 2%, or less than 1%. Typically, when different wavelengths of laser radiation are produced by harmonics generation, or other non-linear frequency conversion processes, then when a specific wavelength is referred to herein, it will be understood by the skilled person that there will be some degree of variation about the specified wavelength in the spectrum produced by the laser. For example, a reference to X nm encompasses a laser producing a spectrum in the range X±10nm, such as X±5nm, for example X±3nm.

### Lasers

Generally, the choice of wavelength and power of the laser used for ablation of the sample can follow normal usage in cellular analysis. The laser can have sufficient fluence to cause ablation to a desired depth, without substantially ablating the sample carrier. A laser fluence of between 0.1-5 J/cm² is typically suitable e.g. from 3-4 J/cm² or about 3.5 J/cm², and the laser will ideally be able to generate a pulse with this fluence at a rate of 200Hz or greater. In some instances, a single laser pulse from such a laser should be sufficient to ablate cellular material for analysis, such that the laser pulse frequency matches the frequency with which ablation plumes are generated. In general, to be a laser useful for imaging biological samples, the laser should produce a pulse with duration below 100 ns (preferably below 1 ns) can be focussed to, for example, the specific spot sizes discussed below.

In some LA-based apparatuses and methods herein, the laser used is a solid state laser. Solid state lasers have the advantage that they are compact, the laser light being emitted through the use of a crystalline material that has been doped with an element, typically a rare earth or transition metal. Of particular use is a laser based on a crystal of yttrium aluminium garnet (YAG) Y₃Al₅O₁₂. An alternative crystal is yttrium lithium fluoride (YLF) LiYF₄. The crystal of a solid state laser is often doped with neodymium, ytterbium, erbium, thulium, holmium or chromium. Sometimes, the laser is a neodymium doped laser. Sometimes, the laser is a neodymium YAG laser, which emits λ = 213nm.

Typically, the laser beam used for ablation in the laser systems discussed herein has a spot size, i.e. at the sampling location, of 100µm or less, such as 50µm or less, 25µm or less, 20µm or less, 15µm or less, or 10µm or less, such as about 3 µm or less, about 2 µm or less, about 1 µm or less, about 500 nm or less, about 250 nm or less. The distance referred to as spot size corresponds to the longest internal dimension of the beam, *e.g.* for a circular beam it is the beam diameter, for a square beam it corresponds to the length of the diagonal between opposed corners, for a quadrilateral it is the length of the longest diagonal *etc.* (as noted above, the diameter of a circular beam with a Gaussian distribution is defined as the distance between the points at which the fluence has decreased to 1/e² times the peak fluence). As an alternative to the Gaussian beam, beam shaping and beam masking can be employed to provide the desired ablation spot. For example, in some applications, a square ablation spot with a top hat energy distribution can be useful (i.e. a beam with near uniform fluence as opposed to a Gaussian energy distribution). This arrangement reduces the dependence of the ablation spot size on the ratio between the fluence at the peak of the Gaussian energy distribution and the threshold fluence. Ablation at close to the threshold fluence provides more reliable ablation crater generation and controls debris generation. Accordingly, the laser system may comprise beam masking and/or beam shaping components, such as a diffractive optical element, arranged in a Gaussian beam to re-shame the beam and produce a laser focal spot of uniform or near-uniform fluence, such as a fluence that varies across the beam by less than ±25%, such as less than ±20%, ±15%, ±10% or less than ±5%. Sometimes, the laser beam has a square cross-sectional shape. Sometimes, the beam has a top hat energy distribution.

When used for analysis of biological samples, in order to analyse individual cells the spot size of laser beam used will depend on the size and spacing of the cells. For example, where the cells are tightly packed against one another (such as in a tissue section) one or more laser sources in the laser system can have a spot size which is no larger than these cells. This size will depend on the particular cells in a sample, but in general the laser spot will have a diameter of less than 4 µm *e.g.* within the range 0.1-4 µm, 0.25-3µm, or 0.4-2 µm. Thus a laser spot can have a diameter of about 3 µm or less, about 2 µm or less, about 1 µm or less, about 0.5 µm or less than 0.5 µm, such as about 400nm or less, about 300nm or less, between 250 nm and 2 µm, or between 300 nm and 1 µm. In order to analyse given cells at a subcellular resolution the system uses a laser spot size which is no larger than these cells, and more specifically uses a laser spot size which can ablate material with a subcellular resolution. Sometimes, single cell analysis can be performed using a spot size larger than the size of the cell, for example where cells are spread out on the slide, with space between the cells. Here, a larger spot size can be used and single cell characterisation achieved, because the additional ablated area around the cell of interest does not comprise additional cells. The particular spot size used can therefore be selected appropriately dependent upon the size of the cells being analysed. In biological samples, the cells will rarely all be of the same size, and so if subcellular resolution imaging is desired, the ablation spot size should be smaller than the smallest cell, if constant spot size is maintained throughout the ablation procedure. Small spot sizes can be achieved using focusing of laser beams . A laser spot diameter of 1 µm corresponds to a laser focus point (i.e. the diameter of the laser beam at the focal point of the beam) of 1 µm, but the laser focus point can vary by +20% or more due to spatial distribution of energy on the target (for instance, Gaussian beam shape) and variation in total laser energy with respect to the ablation threshold energy. Suitable objectives for focusing a laser beam include a reflecting objective, such as an objective of a Schwarzschild Cassegrain design (reverse Cassegrain). Refracting objectives can also be used, as can combination reflecting-refracting objectives. A single aspheric lens can also be used to achieve the required focusing. A solid-immersion lens or diffractive optic can also be used to focus the laser beam. Another means for controlling the spot size of the laser, which can be used alone or in combination with the above objectives is to pass the beam through an aperture, prior to focusing. Different beam diameters can be achieved by passing the beam through apertures of different diameter from an array of diameters. In some instances, there is a single aperture of variable size, for example when the aperture is a diaphragm aperture. Sometimes, the diaphragm aperture is an iris diaphragm. Variation of the spot size can also be achieved through dithering of the optics. The one or more lenses and one or more apertures are positioned between the laser and the sample stage.

For rapid analysis of a sample a high frequency of ablation is needed, for example more than 20 Hz (i.e. more than 20 ablations per second, giving more than 20 plumes per second). Commonly, the frequency of ablation by the laser system is at least 40Hz, such as at least 50Hz, or at least 100Hz. For instance, the frequency of ablation by the laser system is within the range 40-2000 Hz, within the range 40-1500 Hz, within the range 40-500 Hz, within the range 40-200 Hz, within the range 40-150 Hz, or within the range 75-150 Hz. An ablation frequency of more than 40 Hz allows imaging of typical samples to be achieved in a reasonable time. The frequency with which laser pulses can be directed at a spot on the sample (assuming full ablation of the material at that spot) and still be individually resolved determines how quickly the pixels of the image can be obtained. Accordingly, if the duration of laser pulse required to ablate the material at a point means that only less than 5 pulses can be directed at a sample per second, the time taken to study a 1mm x 1mm area with ablation at a spot size of 1µm would be over two days. With a rate of 40Hz, this would be around 6-7 hours, with further reductions in the analysis time for further increases in the frequency of pulses. At these frequencies the instrumentation must be able to analyse the ablated material rapidly enough to avoid substantial signal overlap between consecutive ablations, if it is desired to resolve each ablated plume individually. It is preferred that the overlap between signals originating from consecutive plumes is <10% in intensity, more preferably <5%, and ideally <2%. The time required for analysis of a plume will depend on the washout time of the sample chamber (see sample chamber section below), the transit time of the plume aerosol to and through the laser ionisation system, and the time taken to analyse the ionised material. Each laser pulse can be correlated to a pixel on the image of the sample that is subsequently built up, as discussed in more detail below.

The laser may be a femtosecond laser, a picosecond laser or a nanosecond laser.

For completeness, the standard lasers for LA at sub-cellular resolution, as known in the art, are excimer or exciplex lasers. Suitable results can be obtained using an argon fluoride laser (λ = 193 nm). Pulse durations of 10-15 ns with these lasers can achieve adequate ablation.

Overall, the laser pulse frequency and strength are selected in combination with the response characteristics of the MS detector to permit distinct detection of individual laser ablation plumes. In combination with using a small laser spot and a sample chamber having a short washout time, rapid and high resolution imaging is now feasible.

If the laser system emits laser radiation of two or more wavelengths, this may be achieved by the use of two or more laser sources, wherein each laser source is adapted to emit laser radiation at a wavelength that differs from the wavelength of laser radiation emitted be the other laser source(s) in the laser system.

Thus, the laser system may comprise a first laser source that emits laser radiation at a wavelength of 213nm, and a second laser source that emits laser radiation at 266nm (so that the first laser source ablates principally proteinaceous material, and the second ablates principally DNA material). If ablation at a third wavelength of laser radiation is desired, a third laser source is used in the laser system, and so on.

Sometimes, the laser system for emitting multiple wavelengths of laser radiation comprises a single laser source adapted to emit multiple wavelengths of laser radiation (i.e. one laser emits multiple wavelengths of laser radiation; the laser system may include further laser sources). Some laser sources emit laser radiation at a desired wavelength using wavelength conversion methods such as harmonics or sum-frequency generation, by super-continuum generation, by an optical parametric amplifier or oscillator (OPA/OPO) technique, or by a combination of several techniques, as standard in the art. For instance, an Nd-YAG laser generates laser radiation at 1064nm wavelength, which is called its fundamental frequency. This wavelength can be converted into shorter wavelengths (when needed) by the method of harmonics generation. The 4^{th} harmonic of that laser radiation would be at 266nm (1064nm ÷ 4) and the 5^{th} harmonic would be at 213nm. Thus, the 4^{th} harmonic can target the optical band of high absorption for DNA material while the 5^{th} harmonic would target the band of high absorption for proteins. In many laser arrangements generation of the 5^{th} harmonic is based on the generation of the 4^{th} harmonic. Thus the 4^{th} harmonic will be already present in the laser generating the 5^{th} harmonics output, although often the lower harmonics (with longer wavelength) are filtered out in the laser. Removal of the appropriate filters thus enables the emission of multiple wavelengths of laser radiation. Examples of such lasers are commercially available from Coherent, Inc, RP Photonics, Lee Laser *etc.*

Another useful pair of harmonic frequencies is the 4^{th} and the 3^{rd} harmonics of a laser with a fundamental wavelength at around 800nm. The 4^{th} and the 3^{rd} harmonics here would have wavelengths of 200nm and 266nm respectively. Examples of such lasers are commercially available (Coherent, Inc., Spectra Physics).

In some situations, where the first wavelength of laser radiation and the second wavelength of laser radiation are produced by the same laser source, the wavelengths are not produced via harmonics, but from a laser with a broad emission spectrum. The emission spectrum of the laser can be at least 10nm, such as at least 30nm, at least 50nm or at least 100nm. Multiple wavelengths of light are produced by a white light laser or a supercontinuum laser.

### Timing of the laser pulses of different wavelength

In laser systems employing multiple wavelengths, if both wavelengths are emitted by the same laser source, synchronisation between the pulses of each wavelength happens automatically. In systems employing multiple laser sources in the laser ablation sampling system the laser pulses can be synchronised, if this is desired.

Where a single laser source emits multiple wavelengths of laser radiation and/or where two or more laser sources are used to emit multiple wavelengths laser radiation, a time delay between the pulses of different wavelength may be desired. When there are two or more laser sources, a delay can be achieved by firing the laser sources at different timepoints. Accordingly, where the laser system comprises two or more laser sources, the laser system can further comprise a laser pulse timing controller, adapted to generate asynchronous pulses from at least two of the two or more laser sources. Sometimes, the asynchronous pulses are achieved by the use of a delay generator, which introduces a delay into the electrical trigger pulses which cause the pulses of laser radiation to be emitted by the laser sources.

When the laser pulses of different wavelength are emitted by the same laser source (i.e. a single pulse from the laser source comprises multiple wavelengths of light), then asynchronicity of the pulses of different wavelengths can be achieved by a number of different techniques *(e.g.* optical delay lines). When asynchronous pulses of different wavelengths of laser radiation are used, the plume resulting from each individual pulse can be analysed separately. Alternatively, the two pulses, although asynchronous, may be sufficiently close in time to produce a single plume, thus meaning the material from the two pulses is analysed as a single event in the mass spectrometer component of the apparatus.

One technique to introduce a delay into the pulses of different wavelengths emitted by the same laser source is to direct the pulses of different wavelengths on different optical paths. The wavelength which is directed along the longer optical path will take longer to reach the sample, and so will be delayed with respect to the pulse of the wavelength which has the shorter optical path. The optical path of the wavelength can be lengthened through the use of a reflector arrangement. If the reflector arrangement is adjustable, so that the distance between the reflectors can be varied, this in turn varies the path length along which the laser pulse must travel before it hits the sample, and so permits control of the delay between the pulses of the wavelength directed along the shorter optical path versus the longer optical path. The separation of one wavelength from the one or more other wavelengths can be achieved, for example, by the use of a selectively reflective reflector (e.g. a dichroic filter) or by the use of other beam splitters such as prisms *etc.*

Another technique is to place an optical delay line, such as a variable optical delay line, between the laser source and the stage holding the sample to be ablated. Optical delay lines are commercially available from Oz Optics, Thorlabs, Newport, RF Optic *etc.* By introducing a delay into one of the wavelengths of laser radiation relative to the other, asynchronous delivery of the different wavelengths is achieved from an originally synchronous pulse (i.e. a controlled time delay is introduced into the pulse of one wavelength with respect to the pulse of the other wavelength). Adapting the delay lines appropriately can be used to control the length of delay.

### Laser ablation focal point

To maximise the efficiency of a laser to ablate material from a sample, the sample should be at a suitable position with regard to the laser's focal point, for example at the focal point, as the focal point is where the laser beam will have the smallest diameter and so most concentrated energy. This can be achieved in a number of ways. A first way is that the sample can be moved in the axis of the laser light directed upon it (i.e. up and down the path of the laser light / towards and away from the laser source) to the desired point at which the light is of sufficient intensity to effect the desired ablation. Alternatively, or additionally, lenses can be used to move the focal point of the laser light and so its effective ability to ablate material at the location of the sample, for example by demagnification. The one or more lenses are positioned between the laser and the sample stage. A third way, which can be used alone or in combination with either or both of the two preceding ways, is to alter the position of the laser.

To assist the user of the system in placing the sample at the most suitable location for ablation of material from it, a camera can be directed at the stage holding the sample (discussed in more detail below). Accordingly, the disclosure provides a laser ablation sampling system comprising a camera directed on the sample stage. The image detected by the camera can be focussed to the same point at which the laser is focussed. This can be accomplished by using the same objective lens for both laser ablation and optical imaging. By bringing the focal point of two into accordance, the user can be sure that laser ablation will be most effective when the optical image is in focus. Precise movement of the stage to bring the sample into focus can be effected by use of piezo activators, as available from Physik Instrumente, Cedrat-technologies, Thorlabs and other suppliers.

In a further mode of operation, the laser ablation is directed to the sample through the sample carrier. In this instance, the sample support should be chosen so that it is transparent (at least partially) to the frequency of laser radiation being employed to ablate the sample. Ablation through the sample can have advantages in particular situations, because this mode of ablation can impart additional kinetic energy to the plume of material ablated from the sample, driving the ablated material further away from the surface of the sample, so facilitating the ablated material's being transported away from the sample for analysis in the detector. Likewise, desorption based methods which remove slugs of sample material can also be mediated by laser radiation which passes through the carrier. The additional kinetic energy provided to the slug of material being desorbed can assist in catapulting the slug away from the sample carrier, and so facilitating the slug's being entrained in the carrier gas being flowed through the sample chamber.

In order to achieve 3D-imaging of the sample, the sample, or a defined area thereof, can be ablated to a first depth, which is not completely through the sample. Following this, the same area can be ablated again to a second depth, and so on to third, fourth, etc. depths. This way a 3D image of the sample can be built up. In some instances, it may be preferred to ablate all of the area for ablation to a first depth before proceeding to ablate at the second depth. Alternatively, repeated ablation at the same spot may be performed to ablate through different depths before proceeding onto the next location in the area for ablation. In both instances, deconvolution of the resulting signals at the MS to locations and depths of the sample can be performed by the imaging software.

### Laser system optics for multiple modes of operation

As a matter of routine arrangement, optical components can be used to direct laser radiation, optionally of different wavelengths, to different relative locations. Optical components can also be arranged in order to direct laser radiation, optionally of different wavelengths, onto the sample from different directions. For example one or more wavelengths can be directed onto the sample from above, and one or more wavelengths of laser radiation (optionally different wavelengths) can be directed from below (i.e. through the substrate, such as a microscope slide, which carries the sample, also termed the sample carrier), or indeed the same wavelength can be directed from above and/or below. This enables multiple modes of operation for the same apparatus. Accordingly, the laser system can comprise an arrangement of optical components, arranged to direct laser radiation, optionally of different wavelengths, onto the sample from different directions. Thus optical components may be arranged such that the arrangement directs laser radiation, optionally of different wavelengths, onto the sample from opposite directions. "Opposite" directions in this context is not limited to laser radiation directed perpendicularly onto the sample from above and below (which would be 180° opposite), but includes arrangements which direct laser radiation onto the sample at angles other than perpendicular to the sample. There is no requirement for the laser radiation directed onto the sample from different directions to be parallel. Sometimes, when the sample is on a sample carrier, the reflector arrangement can be arranged to direct laser radiation of a first wavelength directly onto the sample and to direct laser radiation of a second wavelength to the sample through the sample carrier.

Directing laser radiation through the sample carrier to the sample can be used to ablate the sample. In some systems, however, directing the laser radiation through the carrier can be used for "LIFTing" modes of operation, as discussed below in more detail in relation to desorption based sampling systems (although as will be appreciated by one of skill in the art, ablation and LIFTing can be performed by the same apparatus, and so what is termed herein a laser ablation sampling system can also act as a desorption based sampling system). The NA (numerical aperture) of the lens used to focus the laser radiation onto the sample from the first direction may be different from the NA of the lens used to focus the laser radiation (optionally at a different wavelength) onto the sample from the second direction. The lifting operation (e.g. where laser radiation is directed through the sample carrier) often employs a spot size of greater diameter than when ablation is being performed.

### Sample chamber of the laser ablation sampling system

The sample is placed in the sample chamber when it is subjected to laser ablation. The sample chamber comprises a stage, which holds the sample (typically the sample is on a sample carrier). When ablated, the material in the sample forms plumes, and the flow of gas passed through the sample chamber from a gas inlet to a gas outlet carries away the plumes of aerosolised material, including any labelling atoms that were at the ablated location. The gas carries the material to the ionisation system, which ionises the material to enable detection by the detector. The atoms, including the labelling atoms, in the sample can be distinguished by the detector and so their detection reveals the presence or absence of multiple targets in a plume and so a determination of what targets were present at the ablated locus on the sample. Accordingly, the sample chamber plays a dual role in hosting the solid sample that is analysed, but also in being the starting point of the transfer of aerosolised material to the ionisation and detection systems. This means that the gas flow through the chamber can affect how spread out the ablated plume of material becomes as it passes through the system. A measure of how spread out the ablated plume becomes is the washout time of the sample chamber. This figure is a measure of how long it takes material ablated from the sample to be carried out of the sample chamber by the gas flowing through it.

The spatial resolution of the signals generated from laser ablation (i.e. when ablation is used for imaging rather than exclusively for clearing, as discussed below) in this way depends on factors including: (i) the spot size of the laser, as signal is integrated over the total area which is ablated; and the speed with which plumes are generated versus the movement of the sample relative to the laser, and (ii) the speed at which a plume can be analysed, relative to the speed at which plumes are being generated, to avoid overlap of signal from consecutive plumes as mentioned above. Accordingly, being able to analyse a plume in the shortest time possible minimises the likelihood of plume overlap (and so in turn enables plumes to be generated more frequently), if individual analysis of plumes is desired.

Accordingly, a sample chamber with a short washout time (e.g. 100 ms or less) is advantageous for use with the apparatus and methods disclosed herein. A sample chamber with a long washout time will either limit the speed at which an image can be generated or will lead to overlap between signals originating from consecutive sample spots (e.g. Kindness et al. (2003; Clin Chem 49:1916-23), which had signal duration of over 10 seconds). Therefore aerosol washout time is a key limiting factor for achieving high resolution without increasing total scan time. Sample chambers with washout times of ≤100 ms are known in the art. For example, Gurevich & Hergenröder (2007; J. Anal. At. Spectrom., 22:1043-1050) discloses a sample chamber with a washout time below 100 ms. A sample chamber was disclosed in Wang et al. (2013; Anal. Chem. 85:10107-16) (see also WO 2014/146724) which has a washout time of 30 ms or less, thereby permitting a high ablation frequency *(e.g.* above 20 Hz) and thus rapid analysis. Another such sample chamber is disclosed in WO 2014/127034. The sample chamber in WO 2014/127034 comprises a sample capture cell configured to be arranged operably proximate to the target, the sample capture cell including: a capture cavity having an opening formed in a surface of the capture cell, wherein the capture cavity is configured to receive, through the opening, target material ejected or generated from the laser ablation site and a guide wall exposed within the capture cavity and configured to direct a flow of the carrier gas within the capture cavity from an inlet to an outlet such that at least a portion of the target material received within the capture cavity is transferrable into the outlet as a sample. The volume of the capture cavity in the sample chamber of WO 2014/127034 is less than 1cm³ and can be below 0.005cm³. Sometimes the sample chamber has a washout time of 25ms or less, such as 20ms, 10ms or less, 5 ms or less, 2 ms or less, 1 ms, less or 500 µs or less , 200 µs or less, 100 µs or less, 50 µs or less, or 25 µs or less. For example, the sample chamber may have a washout time of 10 µs or more. Typically, the sample chamber has a washout time of 5 ms or less.

For completeness, sometimes the plumes from the sample can be generated more frequently than the washout time of the sample chamber, and the resulting images will smear accordingly (e.g. if the highest possible resolution is not deemed necessary for the particular analysis being undertaken).

The sample chamber typically comprises a translation stage which holds the sample (and sample carrier) and moves the sample relative to the beams of laser radiation. When a mode of operation is used which requires the direction of laser radiation through the sample carrier to the sample, e.g. as in the lifting methods discussed herein, the stage holding the sample carrier should also be transparent to the laser radiation used.

Thus, the sample may be positioned on the side of the sample carrier (e.g., glass slide) facing the laser radiation as it is directed onto the sample, such that ablation plumes are released on, and captured from, the same side as that from which the laser radiation is directed onto the sample. Alternatively, the sample may be positioned on the side of the sample carrier opposite to the laser radiation as it is directed onto the sample (i.e. the laser radiation passes through the sample carrier before reaching the sample), and ablation plumes are released on, and captured from, the opposite side to the laser radiation.

One feature of a sample chamber, which is of particular use where specific portions in various discrete areas of sample are ablated, is a wide range of movement in which the sample can be moved in the x and y (i.e. horizontal) axes in relation to the laser (where the laser beam is directed onto the sample in the z axis), with the x and y axes being perpendicular to one another. More reliable and accurate relative positions are achieved by moving the stage within the sample chamber and keeping the laser's position fixed in the laser ablation sampling system of the apparatus. The greater the range of movement, the more distant the discrete ablated areas can be from one another. The sample is moved in relation to the laser by moving the stage on which the sample is placed. Accordingly, the sample stage can have a range of movement within the sample chamber of at least 10mm in the x and y axes, such as 20mm in the x and y axes, 30mm in the x and y axes, 40mm in the x and y axes, 50mm in the x and y axes, such as 75mm in the x and y axes. Sometimes, the range of movement is such that it permits the entire surface of a standard 25mm by 75mm microscope slide to be analysed within the chamber. Of course, to enable subcellular ablation to be achieved, in addition to a wide range of movement, the movement should be precise. Accordingly, the stage can be configured to move the sample in the x and y axes in increments of less than 10µm, such as less than 5µm, less than 4µm, less than 3µm, less than 2µm, 1µm, or less than 1µm, such as less than 500nm, less than 200 nm, less than 100nm. For example, the stage may be configured to move the sample in increments of at least 50 nm. Precise stage movements can be in increments of about 1µm, such as 1µm±0.1µm. Commercially available microscope stages can be used, for example as available from Thorlabs, Prior Scientific, and Applied Scientific Instrumentation. Alternatively, the motorised stage can be built from components, based on positioners providing the desired range of movement and suitably fine precision movement, such as the SLC-24 positioners from Smaract.

Naturally, when a sample stage in a sample chamber has a wide range of movement, the sample must be sized appropriately to accommodate the movements of the stage. Sizing of the sample chamber is therefore dependent on size of the sample to be involved, which in turn determines the size of the mobile sample stage. Exemplary sizes of sample chamber have an internal chamber of 10 x 10cm, 15 x 15cm or 20 x 20cm. The depth of the chamber may be 3cm, 4cm or 5cm. The skilled person will be able to select appropriate dimensions following the teaching herein. The internal dimensions of the sample chamber for analysing biological samples using a laser ablation sampler must be bigger than the range of movement of the sample stage, for example at least 5mm, such as at least 10mm. This is because if the walls of the chamber are too close to the edge of the stage, the flow of the carrier gas passing through the chamber which takes the ablated plumes of material away from the sample and into the ionisation system can become turbulent. Turbulent flow disturbs the ablated plumes, and so instead of remaining as a tight cloud of ablated material, the plume of material begins to spread out after it has been ablated and carried away to the ionisation system of the apparatus. A broader peak of the ablated material has negative effects on the data produced by the ionisation and detection systems because it leads to interference due to peak overlap, and so ultimately, less spatially resolved data, unless the rate of ablation is slowed down to such a rate that it is no longer experimentally of interest.

As noted above, the sample chamber comprises a gas inlet and a gas outlet that takes material to the ionisation system. However, it may contain further ports acting as inlets or outlets to direct the flow of gas in the chamber and/or provide a mix of gases to the chamber, as determined to be appropriate by the skilled artisan for the particular ablative process being undertaken.

### Camera

In addition to identifying the most effective positioning of the sample for laser ablation, the inclusion of a camera (such as a charged coupled device image sensor based (CCD) camera or an active pixel sensor based camera), or any other light detecting means in a laser ablation sampling system enables various further analyses and techniques. A CCD is a means for detecting light and converting it into digital information that can be used to generate an image. In a CCD image sensor, there are a series of capacitors that detect light, and each capacitor represents a pixel on the determined image. These capacitors allow the conversion of incoming photons into electrical charges. The CCD is then used to read out these charges, and the recorded charges can be converted into an image. An active-pixel sensor (APS) is an image sensor consisting of an integrated circuit containing an array of pixel sensors, each pixel containing a photodetector and an active amplifier, e.g. a CMOS sensor.

A camera can be incorporated into any laser ablation sampling system discussed herein, or a secondary ion mass spectrometry (SIMS) or matrix-assisted laser desorption ionization (MALDI) system as described herein. The camera can be used to scan the sample to identify cells of particular interest or regions of particular interest (for example cells of a particular morphology), or for fluorescent probes specific for an antigen, or an intracellular or structure. In certain embodiments, the fluorescent probes are histochemical stains or antibodies that also comprise a detectable mass tag. Once such cells have been identified, then laser pulses can be directed at these particular cells to ablate material for analysis, for example in an automated (where the system both identifies and ablates the features/regions(s), such as cells(s), of interest) or semi-automated process (where the user of the system, for example a clinical pathologist, identifies the features/regions(s) of interest, which the system then ablates in an automated fashion). This enables a significant increase in the speed at which analyses can be conducted, because instead of needing to ablate the entire sample to analyse particular cells, the cells of interest can be specifically ablated. This leads to efficiencies in methods of analysing biological samples in terms of the time taken to perform the ablation, but in particular in the time taken to interpret the data from the ablation, in terms of constructing images from it. Constructing images from the data is one of the more time-consuming parts of the imaging procedure, and therefore by minimising the data collected to the data from relevant parts of the sample, the overall speed of analysis is increased.

The camera may record the image from a confocal microscope. Confocal microscopy is a form of optical microscopy that offers a number of advantages, including the ability to reduce interference from background information (light) away from the focal plane. This happens by elimination of out-of-focus light or glare. Confocal microscopy can be used to assess unstained samples for the morphology of the cells, or whether a cell is a discrete cell or part of a clump of cells. Often, the sample is specifically labelled with fluorescent markers (such as by labelled antibodies or by labelled nucleic acids). These fluorescent makers can be used to stain specific cell populations (e.g. expressing certain genes and/or proteins) or specific morphological features on cells (such as the nucleus, or mitochondria) and when illuminated with an appropriate wavelength of light, these regions of the sample are specifically identifiable. Some systems described herein therefore can comprise a laser for exciting fluorophores in the labels used to label the sample. Alternatively, an LED light source can be used for exciting the fluorophores. Non-confocal (e.g. wide field) fluorescent microscopy can also be used to identify certain regions of the biological sample, but with lower resolution than confocal microscopy.

An alternative imaging technique is two-photon excitation microscopy (also referred to as non-linear or multiphoton microscopy). The technique commonly employs near-IR light to excite fluorophores. Two photons of IR light are absorbed for each excitation event. Scattering in the tissue is minimized by IR. Further, due to the multiphoton absorption, the background signal is strongly suppressed. The most commonly used fluorophores have excitation spectra in the 400-500 nm range, whereas the laser used to excite the two-photon fluorescence lies in near-IR range. If the fluorophore absorbs two infrared photons simultaneously, it will absorb enough energy to be raised into the excited state. The fluorophore will then emit a single photon with a wavelength that depends on the type of fluorophore used that can then be detected.

When a laser is used to excite fluorophores for fluorescence microscopy, sometimes this laser is the same laser that generates the laser light used to ablate material from the biological sample, but used at a power that is not sufficient to cause ablation of material from the sample. Sometimes the fluorophores are excited by the wavelength of light that the laser then ablates the sample with. In others, a different wavelength may be used, for example by generating different harmonics of the laser to obtain light of different wavelengths, or exploiting different harmonics generated in a harmonic generation system, discussed above, apart from the harmonics which are used to ablate the sample. For example, if the fourth and/or fifth harmonic of a Nd:YAG laser are used, the fundamental harmonic, or the second to third harmonics, could be used for fluorescence microscopy.

As an example technique combining fluorescence and laser ablation, it is possible to label the nuclei of cells in the biological sample with an antibody or nucleic acid conjugated to a fluorescent moiety. Accordingly, by exciting the fluorescent label and then observing and recording the positions of the fluorescence using a camera, it is possible to direct the ablating laser specifically to the nuclei, or to areas not including nuclear material. The division of the sample into nuclei and cytoplasmic regions will find particular application in field of cytochemistry. By using an image sensor (such as a CCD detector or an active pixel sensor, *e.g.* a CMOS sensor), it is possible to entirely automate the process of identifying features/regions of interest and then ablating them, by using a control module (such as a computer or a programmed chip) which correlates the location of the fluorescence with the x,y coordinates of the sample and then directs the ablation laser to that location. As part of this process the first image taken by the image sensor may have a low objective lens magnification (low numerical aperture), which permits a large area of the sample to be surveyed. Following this, a switch to an objective with a higher magnification can be used to home in on the particular features of interest that have been determined to fluoresce by higher magnification optical imaging. These features recorded to fluoresce may then be ablated by a laser. Using a lower numerical aperture lens first has the further advantage that the depth of field is increased, thus meaning features buried within the sample may be detected with greater sensitivity than screening with a higher numerical aperture lens from the outset.

In methods and systems in which fluorescent imaging is used, the emission path of fluorescent light from the sample to the camera may include one or more lenses and/or one or more optical filters. By including an optical filter adapted to pass a selected spectral bandwidth from one or more of the fluorescent labels, the system is adapted to handle chromatic aberrations associated with emissions from the fluorescent labels. Chromatic aberrations are the result of the failure of lenses to focus light of different wavelengths to the same focal point. Accordingly, by including an optical filter, the background in the optical system is reduced, and the resulting optical image is of higher resolution. A further way to minimise the amount of emitted light of undesired wavelengths that reaches the camera is to exploit chromatic aberration of lenses deliberately by using a series of lenses designed for the transmission and focus of light at the wavelength transmitted by the optical filter, akin to the system explained in WO 2005/121864.

A higher resolution optical image is advantageous in this coupling of optical techniques and laser ablation sampling, because the accuracy of the optical image then determines the precision with which the ablating laser can be directed to ablate the sample.

Accordingly, in some embodiments disclosed herein, the apparatus of certain aspects of the present disclosure comprises a camera. This camera can be used on-line to identify features/areas of the sample, e.g. specific cells, which can then be ablated (or desorbed by LIFTing - see below)

In a further mode of operation combining both fluorescence analysis and laser ablation sampling, instead of analysing the entire slide for fluorescence before targeting laser ablation to those locations, it is possible to fire a pulse from the laser at a spot on the sample (at low energy so as only to excite the fluorescent moieties in the sample rather than ablate the sample) and if a fluorescent emission of expected wavelength is detected, then the sample at the spot can be ablated by firing the laser at that spot at full energy, and the resulting plume analysed by a detector as described below. This has the advantage that the rastering mode of analysis is maintained, but the speed is increased, because it is possible to pulse and test for fluorescence and obtain results immediately from the fluorescence (rather than the time taken to analyse and interpret ion data from the detector to determine if the region was of interest), again enabling only the loci of importance to be targeted for analysis. Accordingly, applying this strategy in imaging a biological sample comprising a plurality of cells, the following steps can be performed: (i) labelling a plurality of different target molecules in the sample with one or more different labelling atoms and one or more fluorescent labels, to provide a labelled sample; (ii) illuminating a known location of the sample with light to excite the one or more fluorescent labels; (iii) observing and recording whether there is fluorescence at the location; (iv) if there is fluorescence, directing laser ablation at the location, to form a plume; (v) subjecting the plume to inductively coupled plasma mass spectrometry, and (vi) repeating steps (ii)-(v) for one or more further known locations on the sample, whereby detection of labelling atoms in the plumes permits construction of an image of the sample of the areas which have been ablated.

In some instances, the sample, or the sample carrier, may be modified so as to contain optically detectable (e.g., by optical or fluorescent microscopy) moieties at specific locations. The fluorescent locations can then be used to positionally orient the sample in the apparatus. The use of such marker locations finds utility, for example, where the sample may have been examined visually "offline," such as in a piece of apparatus other than the disclosed apparatus of the present disclosure. Such an optical image can be marked with feature(s)/region(s) of interest, corresponding to particular cells by, say, a physician, before the optical image with the feature(s)/region(s) of interest highlighted and the sample are transferred to an apparatus according to the present disclosure. Here, by reference to the marker locations in the annotated optical image, the apparatus of the present disclosure can identify the corresponding fluorescent positions by use of the camera and calculate an ablative and/or desorptive (LIFTing) plan for the positions of the laser pulses accordingly. Accordingly, some aspects of the present disclosure comprise an orientation controller module capable of performing the above steps.

In some instances, selection of the features/regions of interest may performed using the apparatus of the present disclosure, based on an image of the sample taken by the camera of the apparatus of the present disclosure.

### Transfer conduit

The transfer conduit forms a link between the laser ablation sampling system and the ionisation system, and allows the transportation of plumes of sample material, generated by the laser ablation of the sample, from the laser ablation sampling system to the ionisation system. Part (or all) of the transfer conduit may be formed, for example, by drilling through a suitable material to produce a lumen (e.g., a lumen with a circular, rectangular or other cross-section) for transit of the plume. The transfer conduit sometimes has an inner diameter in the range 0.2 mm to 3 mm. Sometimes, the internal diameter of the transfer conduit can be varied along its length. For example, the transfer conduit may be tapered at an end. A transfer conduit sometimes has a length in the range of 1 centimeter to 100 centimeters. Sometimes the length is no more than 10 centimeters *(e.g.,* 1-10 centimeters), no more than 5 centimeters *(e.g.,* 1-5 centimeters), or no more than 3 cm (e.g., 0.1-3 centimeters). Sometimes the transfer conduit lumen is straight along the entire distance, or nearly the entire distance, from the ablation system to the ionisation system. Other times the transfer conduit lumen is not straight for the entire distance and changes orientation. For example, the transfer conduit may make a gradual 90 degree turn. This configuration allows for the plume generated by ablation of a sample in the laser ablation sampling system to move in a vertical plane initially while the axis at the transfer conduit inlet will be pointing straight up, and move horizontally as it approaches the ionisation system *(e.g.* an ICP torch which is commonly oriented horizontally to take advantage of convectional cooling). The transfer conduit can be straight for a distance of least 0.1 centimeters, at least 0.5 centimeters or at least 1 centimeter from the inlet aperture though which the plume enters or is formed. In general terms, typically, the transfer conduit is adapted to minimize the time it takes to transfer material from the laser ablation sampling system to the ionisation system.

### Transfer conduit inlet, including sample cone

The transfer conduit comprises an inlet in the laser ablation sampling system (in particular within the sample chamber of the laser ablation system; it therefore also represents the principal gas outlet of the sample chamber). The inlet of the transfer conduit receives sample material ablated from a sample in the laser ablation system, and transfers it to the ionisation system. In some instances, the laser ablation sampling system inlet is the source of all gas flow along the transfer conduit to the ionisation system. In some instances, the laser ablation sampling system inlet that receives material from the laser ablation sampling system is an aperture in the wall of a conduit along which a second "transfer" gas is flowed (as disclosed, for example in WO2014146724 and WO2014147260) from a separate transfer flow inlet. In this instance, the transfer gas forms a significant proportion, and in many instances the majority of the gas flow to the ionisation system. The sample chamber of the laser ablation sampling system contains a gas inlet. Flowing gas into the chamber through this inlet creates a flow of gas out of the chamber though the inlet of the transfer conduit. This flow of gas captures plumes of ablated material, and entrains it as it into the transfer conduit (typically the laser ablation sampling system inlet of the transfer conduit is in the shape of a cone, termed herein the sample cone) and out of the sample chamber into the conduit passing above the chamber. This conduit also has gas flowing into it from the separate transfer flow inlet (left hand side of the figure, indicated by the transfer flow arrow). The component comprising the transfer flow inlet, laser ablation sampling system inlet and which begins the transfer conduit which carries the ablated sample material towards the ionisation system can also termed a flow cell (as it is in WO2014146724 and WO2014147260).

The transfer flow fulfils at least three roles: it flushes the plume entering the transfer conduit in the direction of the ionisation system, and prevents the plume material from contacting the side walls of the transfer conduit; it forms a "protection region" above the sample surface and ensures that the ablation is carried out under a controlled atmosphere; and it increases the flow speed in the transfer conduit. Usually, the viscosity of the capture gas is lower than the viscosity of the primary transfer gas. This helps to confine the plume of sample material in the capture gas in the center of the transfer conduit and to minimize the diffusion of the plume of sample material downstream of the laser ablation sampling system (because in the center of the flow, the transport rate is more constant and nearly flat). The gas(es) may be, for example, and without limitation, argon, xenon, helium, nitrogen, or mixtures of these. A common transfer gas is argon. Argon is particularly well-suited for stopping the diffusion of the plume before it reaches the walls of the transfer conduit (and it also assists improved instrumental sensitivity in apparatus where the ionisation system is an argon gas-based ICP). The capture gas is preferably helium. However, the capture gas may be replaced by or contain other gases, e.g., hydrogen, nitrogen, or water vapor. At 25° C, argon has a viscosity of 22.6 µPas, whereas helium has a viscosity of 19.8 µPas. Sometimes, the capture gas is helium and the transfer gas is argon.

As described in WO2014169394, the use of a sample cone minimizes the distance between the target and the laser ablation system inlet of the transfer conduit. Because of the reduced distance between the sample and the point of the cone through which the capture gas can flow cone, this leads to improved capture of sample material with less turbulence, and so reduced spreading of the plumes of ablated sample material. The inlet of the transfer conduit is therefore the aperture at the tip of the sample cone. The cone projects into the sample chamber.

An optional modification of the sample cone is to make it asymmetrical. When the cone is symmetrical, then right at the center the gas flow from all directions neutralizes, so the overall flow of gas is zero along the surface of the sample at the axis of the sample cone. By making the cone asymmetrical, a non-zero velocity along the sample surface is created, which assists in the washout of plume materials from the sample chamber of the laser ablation system.

In practice, any modification of the sample cone that causes a non-zero vector gas flow along the surface of the sample at the axis of the cone may be employed. For instance, the asymmetric cone may comprise a notch or a series of notches, adapted to generate non-zero vector gas flow along the surface of the sample at the axis of the cone. The asymmetric cone may comprise an orifice in the side of the cone, adapted to generate non-zero vector gas flow along the surface of the sample at the axis of the cone. This orifice will imbalance gas flows around the cone, thereby again generating a non-zero vector gas flow along the surface of the sample at the axis of the cone at the target. The side of the cone may comprise more than one orifice and may include both one or more notches and one or more orifices. The edges of the notch(es) and/or orifice(s) are typically smoothed, rounded or chamfered in order to prevent or minimize turbulence.

Different orientations of the asymmetry of the cone will be appropriate for different situations, dependent on the choice of capture and transfer gas and flow rates thereof, and it is within the abilities of the skilled person to appropriately identify the combinations of gas and flow rate for each orientation.

All of the above adaptations may be present in a single asymmetric sample cone as use in the present disclosure. For example, the cone may be asymmetrically truncated and formed from two different elliptical cone halves, the cone may be asymmetrically truncated and comprise one of more orifices and so on.

The sample cone is therefore adapted to capture a plume of material ablated from a sample in the laser ablation system. In use, the sample cone is positioned operably proximate to the sample, e.g. by manoeuvring the sample within the laser ablation sampling system on a movable sample carrier tray, as described already above. As noted above, plumes of ablated sample material enter the transfer conduit through an aperture at the narrow end of the sample cone. The diameter of the aperture can be a) adjustable; b) sized to prevent perturbation to the ablated plume as it passes into the transfer conduit; and/or c) about the equal to the cross-sectional diameter of the ablated plume.

### Tapered conduits

In tubes with a smaller internal diameter, the same flow rate of gas moves at a higher speed. Accordingly, by using a tube with a smaller internal diameter, a plume of ablated sample material carried in the gas flow can be transported across a defined distance more rapidly at a given flow rate (e.g. from the laser ablation sampling system to the ionisation system in the transfer conduit). One of the key factors in how quickly an individual plume can be analysed is how much the plume has diffused during the time from its generation by ablation through to the time its component ions are detected at the mass spectrometer component of the apparatus (the transience time at the detector). Accordingly, by using a narrow transfer conduit, the time between ablation and detection is reduced, thereby meaning diffusion is decreased because there is less time in which it can occur, with the ultimate result that the transience time of each ablation plume at the detector is reduced. Lower transience times mean that more plumes can be generated and analyzed per unit time, thus producing images of higher quality and/or faster.

The taper may comprise a gradual change in the internal diameter of the transfer conduit along said portion of the length of the transfer conduit (i.e. the internal diameter of the tube were a cross section taken through it decreases along the portion from the end of the portion towards the inlet (at the laser ablation sampling system end) to the outlet (at the ionisation system end). Usually, the region of the conduit near where ablation occurs has a relatively wide internal diameter. The larger volume of the conduit before the taper facilitates the confinement of the materials generated by ablation. When the ablated particles fly off from the ablated spot they travel at high velocities. The friction in the gas slows these particles down but the plume can still spread on a sub-millimeter to a millimeter scale. Allowing for sufficient distances to the walls helps with the containment of the plume near the center of the flow.

Because the wide internal diameter section is only short (of the order of 1-2mm), it does not contribute significantly to the overall transience time providing the plume spends more time in the longer portion of the transfer conduit with a narrower internal diameter. Thus, a larger internal diameter portion is used to capture the ablation product and a smaller internal diameter conduit is used to transport these particles rapidly to the ionisation system.

The diameter of the narrow internal diameter section is limited by the diameter corresponding to the onset of turbulence. A Reynolds number can be calculated for a round tube and a known flow. In general a Reynolds number above 4000 will indicate a turbulent flow, and thus should be avoided. A Reynolds number above 2000 will indicate a transitional flow (between non-turbulent and turbulent flow), and thus may also be desired to be avoided. For a given mass flow of gas the Reynolds number is inversely proportional to the diameter of the conduit. The internal diameter of the narrow internal diameter section of the transfer conduit commonly is narrower than 2mm, for example narrower than 1.5mm, narrower than 1.25mm, narrower than 1mm, but greater than the diameter at which a flow of helium at 4 liters per minute in the conduit has a Reynolds number greater than 4000.

Rough or even angular edges in the transitions between the constant diameter portions of the transfer conduit and the taper may cause turbulence in the gas flow, and typically are avoided.

### Sacrificial flow

At higher flows, the risk of turbulence occurring in the conduit increases. This is particularly the case where the transfer conduit has a small internal diameter (e.g. 1mm). However, it is possible to achieve high speed transfer (up to and in excess of 300m/s) in transfer conduits with a small internal diameter if a light gas, such as helium or hydrogen, is used instead of argon which is traditionally used as the transfer flow of gas.

High speed transfer presents problems insofar as it may cause the plumes of ablated sample material to be passed through the ionisation system without an acceptable level of ionisation occurring. The level of ionisation can drop because the increased flow of cool gas reduces the temperature of the plasma at the end of the torch. If a plume of sample material is not ionised to a suitable level, information is lost from the ablated sample material - because its components (including any labelling atoms/elemental tags) cannot be detected by the mass spectrometer. For example, the sample may pass so quickly through the plasma at the end of the torch in an ICP ionisation system that the plasma ions do not have sufficient time to act on the sample material to ionise it. This problem, caused by high flow, high speed transfer in narrow internal diameter transfer conduits can be solved by the introduction of a flow sacrificing system at the outlet of the transfer conduit. The flow sacrificing system is adapted to receive the flow of gas from the transfer conduit, and pass only a portion of that flow (the central portion of the flow comprising any plumes of ablated sample material) onwards into the injector that leads to the ionisation system. To facilitate dispersion of gas from the transfer conduit in the flow sacrificing system, the transfer conduit outlet can be flared out.

The flow sacrificing system is positioned close to the ionisation system, so that the length of the tube (e.g. injector) that leads from the flow sacrificing system to the ionisation system is short *(e.g.* ~1cm long; compared to the length of the transfer conduit which is usually of a length of the order of tens of cm, such as ~50cm). Thus the lower gas velocity within the tube leading from the flow sacrificing system to the ionisation system does not significantly affect the total transfer time, as the relatively slower portion of the overall transport system is much shorter.

In most arrangements, it is not desirable, or in some cases possible, to significantly increase the diameter of the tube (e.g. the injector) which passes from the flow sacrificing system to the ionisation system as a way of reducing the speed of the gas at a volumetric flow rate. For example, where the ionisation system is an ICP, the conduit from the flow sacrificing system forms the injector tube in the center of the ICP torch. When a wider internal diameter injector is used, there is a reduction in signal quality, because the plumes of ablated sample material cannot be injected so precisely into the center of the plasma (which is the hottest and so the most efficiently ionising part of the plasma). The strong preference is for injectors of 1mm internal diameter, or even narrower *(e.g.* an internal diameter of 800µm or less, such as 600µm or less, 500µm or less or 400µm or less). Other ionisation techniques rely on the material to be ionised within a relatively small volume in three dimensional space (because the necessary energy density for ionisation can only be achieved in a small volume), and so a conduit with a wider internal diameter means that much of the sample material passing through the conduit is outside of the zone in which energy density is sufficient to ionise the sample material. Thus narrow diameter tubes from the flow sacrificing system into the ionisation system are also employed in apparatus with non-ICP ionisation systems. As noted above, if a plume of sample material is not ionised to a suitable level, information is lost from the ablated sample material - because its components (including any labelling atoms/elemental tags) cannot be detected by the mass spectrometer.

Pumping can be used to help ensure a desired split ratio between the sacrificial flow and the flow passing into the inlet of the ionisation system. Accordingly, sometimes, the flow sacrificing system comprises a pump attached to the sacrificial flow outlet. A controlled restrictor can be added to the pump to control the sacrificial flow. Sometimes, the flow sacrificing system also comprises a mass flow controller, adapted to control the restrictor.

Where expensive gases are used, the gas pumped out of the sacrificial flow outlet can be cleaned up and recycled back into the same system using known methods of gas purification. Helium is particularly suited as a transport gas as noted above, but it is expensive; thus, it is advantageous to reduce the loss of helium in the system (i.e. when it is passed into the ionisation system and ionised). Accordingly, sometimes a gas purification system is connected to the sacrificial flow outlet of the flow sacrificing system.

### lonisation system

In order to generate elemental ions, it is necessary to use a hard ionisation technique that is capable of vaporising, atomising and ionising the atomised sample.

### Inductively coupled plasma torch

Commonly, an inductively coupled plasma is used to ionise the material to be analysed before it is passed to the mass detector for analysis. It is a plasma source in which the energy is supplied by electric currents produced by electromagnetic induction. The inductively coupled plasma is sustained in a torch that consists of three concentric tubes, the innermost tube being known as the injector.

The induction coil that provides the electromagnetic energy that maintains the plasma is located around the output end of the torch. The alternating electromagnetic field reverses polarity many millions of times per second. Argon gas is supplied between the two outermost concentric tubes. Free electrons are introduced through an electrical discharge and are then accelerated in the alternating electromagnetic field whereupon they collide with the argon atoms and ionise them. At steady state, the plasma consists of mostly of argon atoms with a small fraction of free electrons and argon ions.

The ICP can be retained in the torch because the flow of gas between the two outermost tubes keeps the plasma away from the walls of the torch. A second flow of argon introduced between the injector (the central tube) and the intermediate tube keeps the plasma clear of the injector. A third flow of gas is introduced into the injector in the centre of the torch. Samples to be analysed are introduced through the injector into the plasma.

The ICP can comprise an injector with an internal diameter of less than 2mm and more than 250µm for introducing material from the sample into the plasma. The diameter of the injector refers to the internal diameter of the injector at the end proximal to the plasma. Extending away from the plasma, the injector may be of a different diameter, for example a wider diameter, wherein the difference in diameter is achieved through a stepped increase in diameter or because the injector is tapered along its length. For instance, the internal diameter of the injector can be between 1.75mm and 250µm, such as between 1.5mm and 300µm in diameter, between 1.25mm and 300µm in diameter, between 1mm and 300µm in diameter, between 900µm and 300µm in diameter, between 900µm and 400µm in diameter, for example around 850µm in diameter. The use of an injector with an internal diameter less than 2mm provides significant advantages over injectors with a larger diameter. One advantage of this feature is that the transience of the signal detected in the mass detector when a plume of sample material is introduced into the plasma is reduced with a narrower injector (the plume of sample material being the cloud of particular and vaporous material removed from the sample by the laser ablation sampling system). Accordingly, the time taken to analyse a plume of sample material from its introduction into the ICP for ionisation until the detection of the resulting ions in the mass detector is reduced. This decrease in time taken to analyse a plume of sample material enables more plumes of sample material to be detected in any given time period. Also, an injector with a smaller internal diameter results in the more accurate introduction of sample material into the centre of the induction coupled plasma, where more efficient ionisation occurs (in contrast to a larger diameter injector which could introduce sample material more towards the fringe of the plasma, where ionisation is not as efficient).

ICP torches (Agilent, Varian, Nu Instruments, Spectro, Leeman Labs, PerkinElmer, Thermo Fisher *etc.)* and injectors (for example from Elemental Scientific and Meinhard) are available.

### Other ionisation techniques

### Electron lonisation

Electron ionisation involves bombarding a gas-phase sample with a beam of electrons. An electron ionisation chamber includes a source of electrons and an electron trap. A typical source of the beam of electrons is a rhenium or tungsten wire, usually operated at 70 electron volts energy. Electron beam sources for electron ionisation are available from Markes International. The beam of electrons is directed towards the electron trap, and a magnetic field applied parallel to the direction of the electrons travel causes the electrons to travel in a helical path. The gas-phase sample is directed through the electron ionisation chamber and interacts with the beam of electrons to form ions. Electron ionisation is considered a hard method of ionisation since the process typically causes the sample molecules to fragment. Examples of commercially available electron ionisation systems include the Advanced Markus Electron lonisation Chamber.

### Optional further components of the laser ablation based sampling and ionisation system

### Ion deflector

Mass spectrometers detect ions when they hit a surface of their detector. The collision of an ion with the detector causes the release of electrons from the detector surface. These electrons are multiplied as they pass through the detector (the first released electron knocks out further electrons in the detector, these electrons then hit secondary plates which further amplify the number of electrons). The number of electrons hitting the anode of the detector generates a current. The number of electrons hitting the anode can be controlled by altering the voltage applied to the secondary plates. The current is an analog signal that can then be converted into a count of the ions hitting the detector by an analog-digital converter. When the detector is operating in its linear range, the current can be directly correlated to the number of ions. The quantity of ions that can be detected at once has a limit (which can be expressed as the number of ions detectable per second). Above this point, the number electrons released by ions hitting the detector is no longer correlated to the number of ions. This therefore places an upper limit on the quantitative capabilities of the detector.

When ions hit the detector, its surface becomes damaged by contamination. Over time, this irreversible contamination damage results in fewer electrons being released by the detector surface when an ion hits the detector, with the ultimate result that the detector needs replacing. This is termed "detector aging", and is a well-known phenomenon in MS.

Detector life can therefore be lengthened by avoiding the introduction of overloading quantities of ions into the MS. As noted above, when the total number of ions hitting the MS detector exceeds the upper limit of detection, the signal is not as informative as when the number of ions is below the upper limit because it is no longer quantitative. It is therefore desirable to avoid exceeding the upper limit of detection as it results in accelerated detector aging without generating useful data.

Analysis of large packets of ions by mass spectrometry involves a particular set of challenges not found in normal mass spectrometry. In particular, typical MS techniques involve introducing a low and constant level of material into the detector, which should not approach the upper detection limit or cause accelerated aging of the detector. On the other hand, laser ablation- and desorption-based techniques analyse a relatively large amount of material in a very short time window in the MS: e.g. the ions from a cell-sized patch of a tissue sample which is much larger than the small packets of ions typically analysed in MS. In effect, it is a deliberate almost overloading of the detector with analysed packed of ions resulting from ablation or lifting. In between the analysis events the signal is at baseline (a signal that is close to zero because no ions from labelling atoms are deliberately being entering into the MS from the sampling and ionisation system; some ions will inevitably be detected because the MS is not a complete vacuum).

Thus in apparatus described herein, there is an elevated risk of accelerated detector aging, because the ions from packets of ionised sample material labelled with a large number of detectable atoms can exceed the upper limit of detection and damage the detector without providing useful data.

To address these issues, the apparatus can comprise an ion deflector positioned between the sampling and ionisation system and the detector system (a mass spectrometer), operable to control the entry of ions into the mass spectrometer. In one arrangement, when the ion deflector is on, the ions received from the sampling and ionisation system are deflected (i.e. the path of the ions is changed and so they do not reach the detector), but when the deflector is off the ions are not deflected and reach the detector. How the ion deflector is deployed will depend on the arrangement of the sampling and ionisation system and MS of the apparatus. E.g. if the portal through which the ions enter the MS is not directly in line with the path of ions exiting the sampling and ionisation system, then by default the appropriately arranged ion deflector will be on, in order to direct ions from the sampling and ionisation system into the MS. When an event resulting from the ionisation a packet of ionised sample material considered likely to overload the MS is detected (see below), the ion deflector is switched off, so that the rest of the ionised material from the event is not deflected into the MS and can instead simply hit an internal surface of the system, thereby preserving the life of the MS detector. The ion deflector is returned to its original state after the ions from the damaging event have been prevented from entering the MS, thereby allowing the ions from subsequent packets of ionised sample material to enter the MS and be detected.

Alternatively, in arrangements where (under normal operating conditions) there is no change in the direction of the ions emerging from the sampling and ionisation system before they enter the MS the ion deflector will be off, and the ions from the sampling and ionisation system will pass through it to be analysed in the MS. To prevent damage when a potential overload of the detector is detected, in this configuration the ion deflector is turned on, and so diverts ions so that they do not enter the detector in order to prevent damage to the detector.

The ions entering the MS from ionisation of sample material (such as a plume of material generated by laser ablation or desorption) do not enter the MS all at the same time, but instead enter as a peak with a frequency that follows a probability distribution curve about a maximum frequency: from baseline, at first a small number of ions enters the MS and are detected, and then the frequency of ions increases to a maximum before the number decreases again and trails off to baseline. An event likely to damage the detector can be identified because instead of a slow increase in the frequency of ions at the leading edge of the peak, there is a very quick increase in counts of ions hitting the detector.

The flow of ions hitting the detector of a TOF MS, a particular type of detector as discussed below, is not continual during the analysis of the ions in a packet of ionised sample material.

The TOF comprises a pulser which releases the ions periodically into the flight chamber of the TOF MS in pulsed groups. By releasing the ions all at the known same time, the time of flight mass determination is enabled. The time between the releases of pulses of ions for time of flight mass determination is known as an extraction or push of the TOF MS. The push is in the order of microseconds. The signal from one or more packets of ions from the sampling and ionisation system therefore covers a number of pushes.

Accordingly, when the ion count reading jumps from the baseline to a very high count within one push (i.e. the first portion of the ions from a particular packet of ionised sample material) then it can be predicted that the main body of ions resulting from ionisation of the packet of sample material will be even greater, and so exceed the upper detection limit. it is at this point that an ion deflector can be operated to ensure that the damaging bulk of the ions are directed away from the detector (by being activated or deactivated, depending on the arrangement of the system, as discussed above).

Suitable ion deflectors based on quadrupoles are available in the art (e.g. from Colutron Research Corporation and Dreebit GmbH).

### b. Desorption based sampling and ionising system

A desorption based analyser typically comprises three components. The first is a desorption system for the generation of slugs of sample material from the sample for analysis. Before the atoms in the slugs of desorbed sample material (including any detectable labelling atoms as discussed below) can be detected, the sample must be ionised (and atomised). Accordingly, the apparatus comprises a second component which is an ionisation system that ionises the atoms to form elemental ions to enable their detection by the MS detector component (third component) based on mass/charge ratio. The desorption based sampling system and the ionisation system are connected by a transfer conduit. In many instances the desorption based sampling system is also a laser ablation based sampling system.

### Desorption sampling system

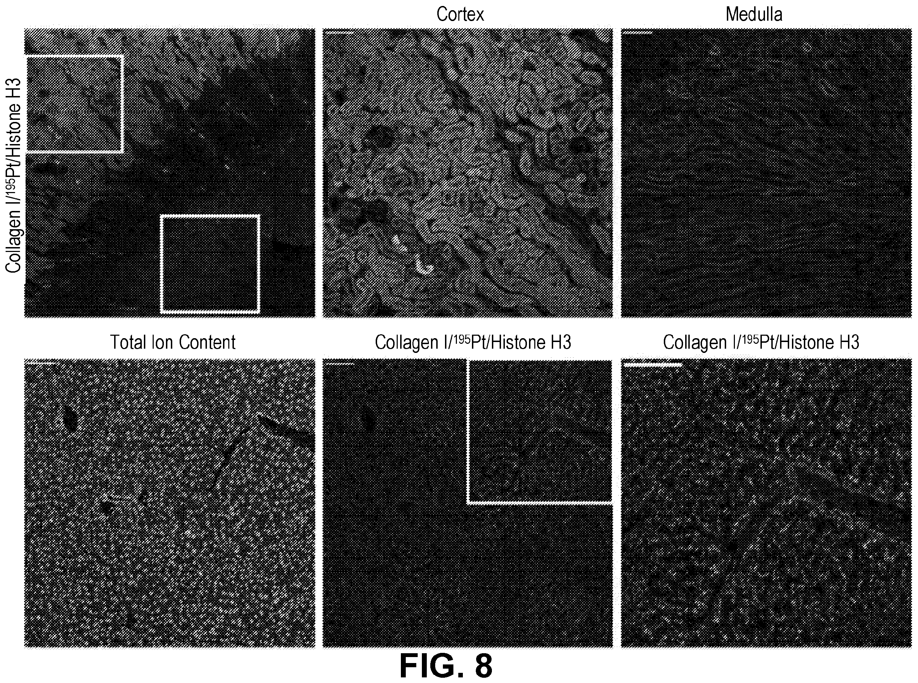

In some instances, rather than laser ablation being used to generate a particulate and/or vaporised plume of sample material, a bulk mass of sample material is desorbed from the sample carrier on which it is located without substantial disintegration of the sample and its conversion into small particles and/or vaporisation (see e.g. Figure 8 of WO2016109825, and the accompanying description). Herein, the term slug is used to refer to this desorbed material (one particular form of a packet of sample material discussed herein). The slug can have dimensions from 10 nm to 10 µm, from 100 nm to 10 µm, and in certain instances from 1 µm to 100 µm. This process can be termed sample catapulting. Commonly, the slug represents a single cell (in which case the process can be termed cell catapulting).

The slug of sample material released from the sample can be a portion of the sample which has been cut into individual slugs for desorption prior to the desorption step, optionally in a process prior to the sample being inserted into the apparatus. A sample divided into discrete slugs prior to analysis is called a structured sample. Each of these individual slugs therefore represents a discrete portion of the sample that can be desorbed, ionised and analysed in the apparatus. By analysis of slugs from the discrete sites, an image can be built up with each slug representing a pixel of the image, in the same way that each location of a sample ablated by the laser ablation sampling system described above.

A structured sample may be prepared by various methods. For instance, a sample carrier comprising topographic features configured to cut a biological sample may be used. Here, a biological sample is applied onto the surface of the carrier, which causes the topographic features to cut and section the sample, in turn causing the sections of biological material to be retained by the plurality of discrete sites between the features to provide a structured biological sample. Alternatively, the sample carrier may not comprise such topographical features (in effect, a flat surface like a microscope slide, optionally functionalised as discussed below), in which case the sample may be applied to the sample carrier and the sample may be sectioned to define slugs of sample that can be desorbed for ionisation and analysis. The sectioning of the sample can be accomplished by mechanical tools such as blades or stamps, if the sample is a tissue section. Alternatively, the material around the sections of the sample to be desorbed can be removed by laser ablation in the same or a separate sample preparation setup. In certain techniques, the material can be removed by a setup employing a focused electron or ion beam. The focused electron or ion beam can lead to particularly narrow cuts (potentially on the 10 nm scale) between subsections leading to a pixel size on the order of 1 µm or in certain instances, 100 nm.

The slugs of sample material can be released from the carrier and each discrete portion of sample material sequentially introduced into the detector for analysis as a discrete event (generating a pixel of an image by the techniques discussed below). The benefits of sequential introduction of discrete material as opposed to random introduction of biological samples as in conventional mass cytometry or mass spectrometry include a higher sample processing rate. This is because the slug is transported from the sample chamber to the ionisation system as preferably a single piece of matter, and so cannot spread out as a plume of ablated material would in a flow of gas (in particular a gas flow in which there is some turbulence).

### Desorption for sampling

Sample material can be desorbed from the sample by thermal energy, mechanical energy, kinetic energy, and a combination of any of the foregoing. This kind of sampling is useful in particular in analysing biological samples.

In certain instances, sample material may be released from the sample by thermal mechanisms. For example, the surface of sample carrier becomes sufficiently hot to desorb a slug of sample material. The sample carrier may be coated to facilitate the bulk desorption process, for example with polyethylene naphthalate (PEN) polymer or PMMA polymer film. Heat can be provided by a radiative source such as a laser (such as the laser of a laser ablation sampling system discussed above). The energy applied to the surface should be sufficient to desorb the biological material, preferably without altering the sample material if it is froma biological sample. Any suitable radiation wavelength can be used, which can depend in part on the absorptive properties of the sample carrier. A surface or layer of the sample carrier may be coated with or include an absorber that absorbs laser radiation for conversion to heat. The radiation may be delivered to a surface of the carrier other than the surface on which the sample is located, or it may be delivered to the surface carrying the sample, such as through the thickness of the carrier. The heated surface may be a surface layer or may be an inner layer of a multilayer structure of the sample carrier. One example of the use of laser radiation energy is in a technique called lifting (laser induced forward transfer; see e.g. Doraiswamy et al., 2006, Applied Surface Science, 52: 4743-4747; Fernández-Pradas, 2004, Thin Solid Films 453-454: 27-30; Kyrkis et al., in Recent Advances in Laser Processing of Materials, Eds. Perriere et al., 2006, Elsivier), in which the sample carrier may comprise a desorption film layer. The desorption film can absorb the radiation to cause release of the desorption film and/or the biological sample *(e.g.* in some instances the sample film desorbs from the sample carrier together with the biological sample, in other instances, the film remains attached to the sample carrier, and the biological sample desorbs from the desorption film).

Desorption by heating can take place on a nanosecond, picosecond or femtosecond time scale, depending on the laser used for desorption.

The sample can be desorbed by the action of a layer of an electrical conductor that heats up upon the application of a current. In such the sites from which sample material is desorbed are electrically connected to electrodes and the sites are individually addressable.

A sample may be attached to the sample carrier by a cleavable photoreactive moiety. Upon irradiating the cleavable photoreactive moiety with radiation (e.g. from a laser in the laser system of the laser ablation sampling system), the photoreactive moiety can cleave to release sample material. The sample carrier may comprise (i) a cleavable photoreactive moiety that couples the sample to the sample carrier and (ii) a desorption film as discussed above. In this situation, a first laser radiation pulse may be used to cause cleavage of the photoreactive moiety and a second laser radiation pulse may be used to target the desorption film to cause separation of the sample from the sample carrier by lifting (or a thermal energy pulse introduced by other means may be used to heat the desorption film and so cause separation of sample material from the sample carrier). The first and second pulses may be of different wavelengths. Thus in some methods (*e.g.* comprising both ablation and desorption), separation of the sample from the sample carrier may involve multiple laser pulses of different wavelengths. In some instances, cleavage of the photoreactive moiety and lifting may be accomplished by the same laser pulse.

The sample carrier may include a coating or layer of a chemically reactive species that imparts kinetic energy to the sample to release the sample from the surface. For example, a chemically reactive species may release a gas such as, for example, H₂, CO₂, N₂ or hydrochlorofluorocarbons. Examples of such compounds include blowing and foaming agents, which release gas upon heating. Generation of gas can be used to impart kinetic energy to desorbing sample material that can improve the reproducibility and direction of release of the material.

A sample carrier may comprise photoinitiated chemical reactants that undergo an exothermic reaction to generate heat for desorbing sample material. The coating of the carrier, or indeed particular chemical linkages in that carrier, discussed in the above paragraphs (that is irradiated by the laser to release the slug of sample material from the carrier) is an example of a material that can be targeted by a wavelength of laser radiation.

The sites on the sample carrier from which slugs of sample material are to be desorbed may be mounted and/or coupled to MEMS devices configured to facilitate release of a biological material from the discrete sites on a carrier.

A slug of the sample can be released or desorbed from a discrete site using nano-heaters, bubble jets, piezoelectrics, ultrasonics, electrostatics, or a combination of any of the foregoing.

Each, or a combination, of these techniques permits ordered detachment of a slug of sample material from the sample carrier. However, often, the location on the sample that is of interest does not represent a discrete entity, such as a lone cell, at a discrete site which can be easily desorbed in isolation. Instead, the cell of interest may be surrounded by other cells or material, of which analysis is not required or desired. Trying to perform desorption (*e.g*. lifting) of the feature/region of interest may therefore desorb both the cell of interest and surrounding material together. Atoms, such as labelling atoms which are used in elemental tags (see discussion below), from the surrounding area of the sample (*e.g*. from other cells which have been labelled) that are carried in a desorbed slug of material in addition to the specific feature/region (*e*.*g*. cell) of interest could therefore contaminate the reading for the location of interest.

The techniques of ablation and desorption (such as by lifting) can be combined in a single method. For example, to perform precise desorption of a feature/region (*e.g*. cell) of interest on a biological sample, *e.g*. a tissue section sample or cell suspension dispersion, on the sample carrier, laser ablation can be used to ablate the area around the cell of interest to clear it of other material. After clearing the surrounding area by ablation, the feature/region of interest can then be desorbed from the sample carrier, and then ionised and analyzed by mass spectrometry in line with standard mass cytometry or mass spectrometry procedures. In line with the above discussion, thermal, photolytic, chemical, or physical techniques can be used to desorb material from a feature/region of interest, optionally after ablation has been used to clear the area surrounding the location that will be desorbed. Often, lifting will be employed, to separate the slug of material from the sample carrier (*e.g.* a sample carrier which has been coated with a desorption film to assist the lifting procedure, as discussed above with regard to desorption of discrete slugs of sample material).

The feature/region of interest can be identified by another technique before the laser ablation and desorption (*e.g*. by lifting) is performed. The inclusion of a camera (such as a charged coupled device image sensor based (CCD) camera or a CMOS camera or an active pixel sensor based camera), or any other light detecting means as described in the preceding sections is one way of enabling these techniques, for both online and offline analyses. The camera can be used to scan the sample to identify cells of particular interest or features/regions of particular interest (for example cells of a particular morphology). Once such locations have been identified, the locations can be lifted after laser pulses have been directed at the area around the feature/region of interest to clear other material by ablation before the location (e.g. cell) is lifted. This process may be automated (where the system both identifies, ablates and lifts the feature(s)/region(s) of interest) or semi-automated process (where the user of the system, for example a clinical pathologist, identifies the feature(s)/region(s) of interest, following which the system then performs ablation and lifting in an automated fashion). This enables a significant increase in the speed at which analyses can be conducted, because instead of needing to ablate the entire sample to analyze particular cells, the cells of interest can be specifically ablated.

The camera can record the image from a microscope (e.g. a confocal microscope). The identification may be by light microscopy, for example by examining cell morphology or cell size, or on whether the cell is a discrete single cell (in contrast to a member of a clump of cells). Sometimes, the sample can be specifically labelled to identify the feature(s) (e.g. cell(s)) of interest. Often, fluorescent markers are used to specifically stain the cells of interest (such as by using labelled antibodies or labelled nucleic acids), as discussed above in relation to methods of ablating visually-identified features/regions of interest; that section is not repeated here in full in the interests of brevity, but one of skill in the art will immediately appreciate that the features of those methods can be applied to desorption based methods and that this is within the technical teaching of this document. A high resolution optical image is advantageous in this coupling of optical techniques and lifting, because the accuracy of the optical image then determines the precision with which the ablating laser source can be directed to ablate the area surrounding the cell of interest which can subsequently be ablated.

Sometimes, no data are recorded from the ablation performed to clear the area around the location to be desorbed (*e.g*. the cell of interest). Sometimes, data is recorded from the ablation of the surrounding area. Useful information that can be obtained from the surrounding area includes what target molecules, such as proteins and RNA transcripts, are present in the surrounding cells and intercellular milieu. This may be of particular interest when imaging solid tissue samples, where direct cell-cell interactions are common, and what proteins etc. are expressed in the surrounding cells may be informative on the state of the cell of interest.

### Camera

The camera used in the desorption based sampling system can be as described above for the laser ablation based sampling system, and the discussion for the camera of the laser ablation based sampling system should be read in here.

### Sample chamber

The sample chamber used in the desorption based sampling system can be as described above for the laser ablation based sampling system. In instances where sampling of large slugs of sample material is being undertaken, the skilled practitioner will appreciate that gas flow volumes may need to be increased to ensure that the slug of material is entrained in the flow of gas and carried into the transfer conduit for transport to the ionisation system.

### Transfer conduit

The sample chamber used in the desorption based sampling system can be as described above for the laser ablation based sampling system. In instances where sampling of large slugs of sample material is being undertaken, the skilled practitioner will appreciate that the diameter of the lumen of the conduit will need to be appropriately sized to accommodate any slugs without the slug contacting the side of the lumen (because any contact may lead to fragmentation of the slug, and to the overlapping of signals - where atoms from the slug resulting the nth desorption event are spread into the detection window for the n+1th or subsequent slugs).

### Ionisation system of the desorption based system

In many instances, the lifting techniques discussed above involve the removal of relatively large slugs of sample material (10 nm to 10 µm, from 100 nm to 10 µm, and in certain instances from 1 µm to 100 µm) which have not been converted into particulate and vaporous material. Accordingly, an ionisation technique which is capable of vaporising and atomising this relatively large quantity of material is required.

### Inductively coupled plasma torch

One such suitable ionisation system is an inductively coupled plasma, as already discussed above in the section beginning on page 95 in relation to laser ablation based sampling and ionisation systems.

### Optional further components of the desorption based sampling and ionisation system

### Ion deflector

The ion deflector used in the desorption based sampling system can be as described above for the laser ablation based sampling system. Given the potential for desorption based sampling to remove intact large slugs of sample material, ion deflectors can be particularly useful in this kind of system for protecting the detector.

### c. Secondary ion generation systems

A secondary ion based analyser typically comprises two components. The first is a system for the generation of ions from the sample for analysis. In this apparatus, this is achieved by directing a first focused primary ion beam onto the sample to generate ejected secondary ions; herein it is called a secondary ion generation system. These ejected ions (including any detectable ions from labelling atoms as discussed below) can be detected by a detector system (the second component) for instance a mass spectrometer (detectors are discussed in more detail below). This technique is known as secondary ion mass spectrometry (SIMS).

The secondary ion generation system comprises: a primary ion source for producing primary ions; a primary ion column for passing the primary ions to the sample in a sample chamber; a sample chamber; and an ion microscope for collecting the secondary ions.

In operation, the primary ions produced by the primary ion source bombard the surface of a sample in the sample chamber, transferring energy to the atoms of the sample. This bombardment generates a series of collisions between atoms within the sample. Some atoms near the surface of the sample recoil with enough energy to escape from the surface of the sample (sputtering). Some emitted particles are in an ionised state - these are the secondary ions, which can be subsequently detected.

### Primary Ion Source

The primary ions can be any suitable ion for generating sputtering from the sample to be analysed. Examples of primary ion sources are: the Duoplasmatron which generates oxygen (¹⁶O-, ¹⁶O₂⁺, ¹⁶O₂⁻), argon (⁴⁰Ar⁺), xenon (Xe⁺), SF₅⁺, or C₆₀⁺ primary ions; a surface ionisation source which generates ¹³³Cs⁺ primary ions; and liquid metal ion guns (LMIG) which generate Ga⁺ primary ions. Other primary ions include cluster ions such as Auₙ⁺ (n=1-5), Biₙ^{q+} (n=1-7, q=1 and 12), c₆₀^{q+} probes (q=1-3) and large Ar clusters (Muramoto, Brison, & Castner, 2012).

The choice of ion source depends on the type of SIMS being deployed (i.e. static or dynamic) and the sample to be analysed. Static SIMS involves using a low primary ion beam current (1nA/cm²), usually a pulsed ion beam. Because of the low current, each ion strikes a new section of the sample surface, removing only a monolayer of particles (2nm). Hence, static SIMS is suitable for imaging and surface analysis (Gamble & Anderton, 2016). Dynamic SIMS involves using a high primary ion beam current (10mA/cm²), usually a continuous primary ion beam, which results in the fast removal of surface particles. As a result, is possible to use dynamic SIMS for depth profiling. Furthermore, since more material is removed from the sample surface, dynamic SIMS gives a better detection limit than static SIMS. Dynamic SIMS typically produces high image resolution (less than 100nm) (Vickerman & Briggs, 2013).

Oxygen primary ions enhance ionisation of electropositive elements (Malherbe, Penen, Isaure, & Frank, 2016) and are used in the commercially available Cameca IMS 1280-HR, whereas caesium primary ions are used to investigate electronegative elements (Kiss, 2012) and are used in the commercially available Cameca NanoSIMS 50.

Liquid metal ion guns can produce a tightly focused beam and so are commonly used in static SIMS. An example of a SIMS instrument using a liquid metal ion gun is the SurfaceSeer-I by KORE Technology.

For rapid analysis of a sample a high frequency of ablation is needed, for example more than 20 Hz (i.e. more than 20 ablations per second, giving more than 20 plumes per second). Commonly, the frequency of primary ion pulse generation by the primary ion source is at least 40Hz, such as at least 50Hz, or at least 100Hz. For instance, the frequency of ion pulses is within the range 40-2000 Hz, within the range 40-1500 Hz, within the range 40-500 Hz, within the range 40-200 Hz, within the range 40-150 Hz, or within the range 75-150 Hz. Afrequency of more than 40 Hz allows imaging of typical samples to be achieved in a reasonable time. The frequency with which ion pulses can be directed at a spot on the sample and still be individually resolved determines how quickly the pixels of the image can be obtained

### Primary Ion Column

The primary ion column directs the primary ions to the sample. The primary ion column comprises: a mass filter in order to filter out impurities in the primary ion beam; lenses and apertures as appropriate in order to control the intensity and shape of the primary ion beam; and deflection plates in order to shape the primary ion beam and optionally raster the primary ion beam across the surface of the sample (Villacob, 2016). Ion lenses and other components for constructing the primary ion column are commercially available, *e.g.* from Agilent.

Typically, the ion beam used for secondary ion generation herein has a spot size (*i.e.* size of the primary ion beam when it hits the sample) of 100µm or less, such as 50um or less, 25µm or less, 20µm or less, 15µm or less, or 10µm or less. The distance referred to as spot size corresponds to the longest internal dimension of the ion beam, *e.g.* for a circular beam it is a beam of diameter 2µm, for a square beam corresponds to the length of the diagonal between opposed corners, for a quadrilateral it is the length of the longest diagonal *etc.* Beam shaping and beam masking can be employed to provide the spot shape and size.

When used for analysis of biological samples, in order to analyse individual cells the spot size of ion beam used will depend on the size and spacing of the cells. For example, where the cells are tightly packed against one another (such as in a tissue section) the ion beam can have a spot size which is no larger than these cells if single cell analysis is to be conducted. This size will depend on the particular cells in a sample, but in general the ion beam spot will have a diameter of less than 4 µm *e.g.* within the range 0.1-4 µm, 0.25-3µm, or 0.4-2 µm. Thus a primary ion beam spot can have a diameter of about 3 µm or less, about 2 µm or less, about 1 µm or less, about 0.5 µm or less than 0.5 µm, such as about 400nm or less, about 300nm or less, between 250nm and 2 um, or between 300 nm and 1 um. In order to analyse cells at a subcellular resolution the system uses a primary ion beam spot size which is no larger than these cells, and more specifically uses a primary ion beam spot size which can ablate material with a subcellular resolution. Sometimes, single cell analysis can be performed using a spot size larger than the size of the cell, for example where cells are spread out on the slide, with space between the cells. The particular spot size used can therefore be selected appropriately dependent upon the size of the cells being analysed. In biological samples, the cells will rarely all be of the same size, and so if subcellular resolution imaging is desired, the ion beam spot size should be smaller than the smallest cell, if constant spot size is maintained throughout the secondary ion generation procedure.

### Sample Chamber

The sample chamber of the secondary ion generation system shares many features in common with the sample chamber of the laser ablation-based and lifting-based sampling systems discussed above. It comprises a stage to support the sample. The stage may be a translation stage, movable in the x-y or x-y-z axes. The sample chamber will also comprise an outlet, through which material removed from the sample by the primary ion beam can be directed. The outlet is connected to the detector, enabling analysis of the secondary ions.

The principal difference between the sample chamber of the secondary ion generation system and the sample chambers of the laser ablation-based and lifting-based sampling systems is that the chamber is held under vacuum in order to prevent collisions between secondary ions and other particles within the chamber, which could result in loss of charge from the secondary ions - on a similar basis contrary to the laser ablation and desorption based sample chambers. Loss of secondary ions would result in reduced sensitivity for the apparatus.

### Ion microscope

The secondary ion beams are captured from the sample via an electrostatic lens positioned near to the sample, known in the art as an immersion lens (or an extraction lens). The immersion lens removes the secondary ions immediately from the locality of the sample. This is typically achieved by the sample and the lens having a large difference in voltage potential. Depending on the polarity of the sample vis-à-vis the immersion lens, positive or negative secondary ions are captured by the immersion lens. The polarity of the secondary ions as captured by the immersion lens is independent of the polarity of the ions of the primary ion beam.

The secondary ions are then transferred to the detector by via one or more further electrostatic lenses (known as transfer lenses in the art). The transfer lens(es) focus(es) the beam of secondary ions into the detector. Typically, in systems with multiple transfer lenses, only one transfer lens is engaged in a given analysis. Each lens may provide a different magnification of the sample surface. Commonly, further ion manipulation components are present between the immersion lens and the detector, for example one or more apertures, mass filters or sets of deflector plates. Together, the immersion lens, transfer lens, and any further components, form the ion microscope. Components for the production of an ion microscope are available from commercial suppliers e.g. Agilent.

### Camera

The secondary ion generation system may also comprise a camera. Camera systems are discussed above in relation to laser ablation sampling systems, and the features of the above camera can also be present in the secondary ion generation system, except where incompatible (*e.g.* it can be connected to a light microscope, such as a confocal microscope, but it is not possible to focus a primary ion beam through the same optics as the light which is directed to the camera, because one beam is ions and the other photons).

### Post lonisation

Secondary ionisation of neutral ejected mass particles can be achieved by a variety of techniques, for example laser ionisation (*e.g*. by a femtosecond laser) and ionisation by an electron beam.

The ions generated in secondary neutral mass spectrometry (SNMS) can then captured by the immersion lens and transferred to the detector as described above for the secondary ions generated directly from primary ion bombardment.

### 2. Mass detector system

Exemplary types of mass detector system include quadrupole, time of flight (TOF), magnetic sector, high resolution, single or multicollector based mass spectrometers.

The time taken to analyse the ionised material will depend on the type of mass analyser which is used for detection of ions. For example, instruments which use Faraday cups are generally too slow for analysing rapid signals. Overall, the desired imaging speed, resolution and degree of multiplexing will dictate the type(s) of mass analyser which should be used (or, conversely, the choice of mass analyser will determine the speed, resolution and multiplexing which can be achieved).

Mass spectrometry instruments that detect ions at only one mass-to-charge ratio (m/Q, commonly referred to as m/z in MS) at a time, for example using a point ion detector, will give poor results in imaging detecting. Firstly, the time taken to switch between mass-to-charge ratios limits the speed at which multiple signals can be determined, and secondly, if ions are at low abundance then signals can be missed when the instrument is focused on other mass-to-charge ratios. Thus it is preferred to use a technique which offers substantially simultaneous detection of ions having different m/Q values.

### Detector types

### Quadrupole detector

Quadrupole mass analysers comprise four parallel rods with a detector at one end. An alternating RF potential and fixed DC offset potential is applied between one pair of rods and the other so that one pair of rods (each of the rods opposite each other) has an opposite alternative potential to the other pair of rods. The ionised sample is passed through the middle of the rods, in a direction parallel to the rods and towards the detector. The applied potentials affect the trajectory of the ions such that only ions of a certain mass-charge ratio will have a stable trajectory and so reach the detector. Ions of other mass-charge ratios will collide with the rods.

### Magnetic Sector detector

In magnetic sector mass spectrometry, the ionised sample is passed through a curved flight tube towards an ion detector. A magnetic field applied across the flight tube causes the ions to deflect from their path. The amount of deflection of each ion is based on the mass to charge ratio of each ion and so only some of the ions will collide with the detector - the other ions will be deflected away from the detector. In multicollector sector field instruments, an array of detectors is be used to detect ions of different masses. In some instruments, such as the ThermoScientific Neptune Plus, and Nu Plasma II, the magnetic sector is combined with an electrostatic sector to provide a double-focussing magnetic sector instrument that analyses ions by kinetic energy, in addition to mass to charge ratio. In particular those multidetectors having a Mattauch-Herzog geometry can be used (*e.g*. the SPECTRO MS, which can simultaneously record all elements from lithium to uranium in a single measurement using a semiconductor direct charge detector). These instruments can measure multiple m/Q signals substantially simultaneously. Their sensitivity can be increased by including electron multipliers in the detectors. Array sector instruments are always applicable, however, because, although they are useful for detecting increasing signals, they are less useful when signal levels are decreasing, and so they are not well suited in situations where labels are present at particularly highly variable concentrations.

### Time of Flight (TOF) detector

A time of flight mass spectrometer comprises a sample inlet, an acceleration chamber with a strong electric field applied across it, and an ion detector. A plume of ionised sample molecules is introduced through the sample inlet and into the acceleration chamber. Initially, each of the ionised sample molecules has the same kinetic energy but as the ionised sample molecules are accelerated through the acceleration chamber, they are separated by their masses, with the lighter ionised sample molecules travelling faster than heaver ions. The detector then detects all the ions as they arrive. The time taking for each particle to reach the detector depends on the mass to charge ratio of the particle.

Thus a TOF detector can quasi-simultaneously register multiple masses in a single sample. In theory TOF techniques are not ideally suited to ICP ion sources because of their space charge characteristics, but TOF instruments can in fact analyse an ICP ion aerosol rapidly enough and sensitively enough to permit feasible single-cell imaging. Whereas TOF mass analyzers are normally unpopular for atomic analysis because of the compromises required to deal with the effects of space charge in the TOF accelerator and flight tube, tissue imaging using the disclosure can be effective by detecting only the labelling atoms, and so other atoms (e.g. those having an atomic mass below 100) can be removed. This results in a less dense ion beam, enriched in the masses in (for example) the 100-250 dalton region, which can be manipulated and focused more efficiently, thereby facilitating TOF detection and taking advantage of the high spectral scan rate of TOF. Thus rapid imaging can be achieved by combining TOF detection with choosing labelling atoms that are uncommon in the sample and ideally having masses above the masses seen in an unlabelled sample e.g. by using the higher mass transition elements. Using a narrower window of label masses thus means that TOF detection to be used for efficient imaging.

Suitable TOF instruments are available from Tofwerk, GBC Scientific Equipment (*e.g.* the Optimass 9500 ICP-TOFMS), and Fluidigm Canada (*e.g.* the CyTOF^{™} and CyTOF^{™}2 instruments). These CyTOF^{™} instruments have greater sensitivity than the Tofwerk and GBC instruments and are known for use in mass cytometry because they can rapidly and sensitively detect ions in the mass range of rare earth metals (particularly in the m/Q range of 100-200; see Bandura et al. (2009; Anal. Chem., 81:6813-22)). Thus these are preferred instruments for use with the disclosure, and they can be used for imaging with the instrument settings already known in the art *e.g.* Bendall et al. (2011; Science 332, 687-696) & Bodenmiller et al. (2012; Nat. Biotechnol. 30:858-867). Their mass analysers can detect a large number of markers quasi-simultaneously at a high mass-spectrum acquisition frequency on the timescale of highfrequency laser ablation or sample desorption. They can measure the abundance of labelling atoms with a detection limit of about 100 per cell, permitting sensitive construction of an image of the tissue sample. Because of these features, mass cytometry can now be used to meet the sensitivity and multiplexing needs for tissue imaging at subcellular resolution. By combining the mass cytometry instrument with a high-resolution laser ablation sampling system and a rapid-transit low-dispersion sample chamber it has been possible to permit construction of an image of the tissue sample with high multiplexing on a practical timescale.

The TOF may be coupled with a mass-assignment corrector. The vast majority of ionisation events generate M⁺ ions, where a single electron has been knocked out of the atom. Because of the mode of operation of the TOF MS there is sometimes some bleeding (or cross-talk) of the ions of one mass (M) into the channels for neighbouring masses (Mt1), in particular where a large number of ions of mass M are entering the detector (i.e. ion counts which are high, but not so high that an ion deflector positioned between the sampling ionisation system and MS would prevent them from entering the MS, if the apparatus were to comprise such an ion deflector). As the arrival time of each M⁺ ion at the detector follows a probability distribution about a mean (which is known for each M), when the number of ions at mass M⁺ is high, then some will arrive at times that would normally be associated with the M-1⁺ or M+1⁺ ions. However, as each ion has a known distribution curve upon entering the TOF MS, based on the peak in the mass M channel it is possible to determine, the overlap of ions of mass M into the M±1 channels (by comparison to the known peak shape). The calculation is particularly applicable for TOF MS, because the peak of ions detected in a TOF MS is asymmetrical. Accordingly it is therefore possible to correct the readings for the M-1, M and M+1 channels to appropriately assign all of the detected ions to the M channel. Such corrections have particular use in correcting imaging data due to the nature of the large packets of ions produced by sampling and ionisation systems such as those disclosed herein involving laser ablation (or desorption as discussed below) as the techniques for removing material from the sample. Programs and methods for improving the quality of data by de-convoluting the data from TOF MS are discussed in WO2011/098834, US patent 8723108 and WO2014/091243.

### Dead-time corrector

As noted above, signals in the MS are detected on the basis of collisions between ions and the detector, and the release of electrons from the surface of the detector hit by the ions. When a high count of ions is detected by the MS resulting in the release of a large number of electrons, the detector of the MS can become temporarily fatigued, with the result that the analog signal output from the detector is temporarily depressed for one or more of the subsequent packets of ions. In other words, a particularly high count of ions in a packet of ionised sample material causes a lot of electrons to be released from the detector surface and secondary multiplier in the process of detecting the ions from that packet of ionised sample material, meaning that fewer electrons are available to be released when the ions in subsequent packets of ionised sample material hit the detector, until the electrons in the detector surface and secondary amplifier are replenished.

Based on a characterisation of the behaviour of the detector, it is possible to compensate for this dead-time phenomenon. A first step is to analyse the ion peak in the analog signal resulting from the detection of the nth packet of ionised sample material by the detector. The magnitude of the peak may be determined by the height of the peak, by the area of the peak, or by a combination of peak height and peak area.

The magnitude of the peak is then compared to see if it exceeds a predetermined threshold. If the magnitude is below this threshold, then no correction is necessary. If the magnitude is above the threshold, then correction of the digital signal from at least one subsequent packet of ionised sample material will be performed (at least the (n+1)th packet of ionised sample material, but possibly further packets of ionised sample material, such as (n+2)th, (n+3)th, (n+4)th *etc.*) to compensate for the temporary depression of the analog signal from these packets of ionised sample material resulting from the fatiguing of the detector caused by the nth packet of ionised sample material. The greater the magnitude of the peak of the nth packet of ionised sample material, the more peaks from subsequent packets of ionised sample material will need to be corrected and the magnitude of correction will need to be greater. Methods for correcting such phenomena are discussed in Stephan et al. (1994; Vac. Sci. Technol. 12:405), Tyler and Peterson (2013;. Surf Interface Anal. 45:475-478), Tyler (2014; Surf Interface Anal. 46:581-590), WO2006/090138 and US patent 6229142, and these methods can be applied by the dead-time corrector to the data, as described herein.

### Analyser apparatus based on optical emission spectra detection

### 1. Sampling and ionisation systems

### a. Laser ablation based sampling and ionising system

The laser ablation sampling system sampling system described above in relation to mass-based analysers can be employed in an OES detector-based system. For detection of atomic emission spectra, most preferably, an ICP is used to ionise the sample material removed from the sample, but any hard ionisation technique that can produce elemental ions can be used.

As appreciated by one of skill in the art, certain optional further components of the laser ablation based sampling and ionising system above, described in relation to avoiding overload of the mass-based detector, may not be applicable to all OES detector-based systems, and would not be incorporated, if inappropriate, by the skilled artisan. Furthermore, the skilled artisan will appreciate that while OES can detect elements, it cannot distinguish between isotopes of the element. Accordingly, where target SBPs/analytes are to be distinctively analysed, OES should be conducted with reagents labelled with different elements, rather isotopes of the same element.

### b. Desorption based sampling and ionising system

The desorption-based sampling system described above in relation to mass-based analysers can be employed in an OES detector-based system. For detection of atomic emission spectra, most preferably, an ICP is used to ionise the sample material removed from the sample, but any hard ionisation technique that can produce elemental ions can be used.

As appreciated by one of skill in the art, certain optional further components of the desorption based sampling and ionising system above, described in relation to avoiding overload of the mass-based detector, may not be applicable to all OES detector-based systems, and would not be incorporated, if inappropriate, by the skilled artisan.

### 2. Photodetectors

Exemplary types of photodetectors include photomultipliers and charged-coupled devices (CCDs). Photodetetors may be used to image the sample and/or identify a region of interest prior to imaging by elemental mass spectrometry.

Photomultipliers comprise a vacuum chamber comprising a photocathode, several dynodes, and an anode. A photon incident on the photocathode causes the photocathode to emit an electron as a consequence of the photoelectric effect. The electron is multiplied by the dynodes due to the process of secondary emission (discussed in more detail with reference to SIMS) to produce a multiplied electron current, and then the multiplied electron current is detected by the anode to provide a measure of detection of electromagnetic radiation incident on the photocathode. Photomultipliers are available from, for example, ThorLabs.

A CCD comprises a silicon chip containing an array of light-sensitive pixels. During exposure to light, each pixel generates an electric charge in proportion to the intensity of light incident on the pixel. After the exposure, a control circuit causes a sequence of transfers of electric charge to produce a sequence of voltages. These voltages can then be analysed to produce an image. Suitable CCDs are available from, for example, Cell Biosciences.

### Constructing an image

The apparatus above can provide signals for multiple atoms in packets of ionised sample material removed from the sample (be that by ablation, ion bombardment or any other technique). Detection of an atom in a packet of sample material reveals its presence at the position of ablation, be that because the atom is naturally present in the sample or because the atom has been localised to that location by a labelling reagent. By generating a series of packets of ionised sample material from known spatial locations on the sample's surface the detector signals reveal the location of the atoms on the sample, and so the signals can be used to construct an image of the sample. By labelling multiple targets with distinguishable labels it is possible to associate the location of labelling atoms with the location of cognate targets, so the method can build complex images, reaching levels of multiplexing which far exceed those achievable using existing techniques. The images generated by the methods can reproduce the staining patterns and the proportion of cells expressing a given marker as determined by IFM, thereby confirming the method's suitability for imaging.

Assembly of signals into an image will use a computer and can be achieved using known techniques and software packages. For instance, the GRAPHIS package from Kylebank Software may be used, or other packages such as TERAPLOT can also be used. Imaging using MS data from techniques such as MALDI-MSI is known in the art *e.g*. Robichaud et al. (2013; J Am Soc Mass Spectrom 24 5:718-21) discloses the 'MSiReader' interface to view and analyze MS imaging files on a Matlab platform, and Klinkert et al. (2014; Int J Mass Spectrom http://dx.doi.org/10.1016/j.ijms.2013.12.012) discloses two software instruments for rapid data exploration and visualization of both 2D and 3D MSI data sets in full spatial and spectral resolution *e.g.* the 'Datacube Explorer' program.

Images obtained using the methods disclosed herein can be further analysed e.g. in the same way that IHC results are analysed. For instance, the images can be used for delineating cell sub-populations within a sample, and can provide information useful for clinical diagnosis. Similarly, SPADE analysis can be used to extract a cellular hierarchy from the high-dimensional cytometry data which methods of the disclosure provide (Qiu et al. (2011; Nat. Biotechnol. 29:886-91)).

### Apparatus comprising the sample of the present disclosure

Also provided herein is an apparatus as described above comprising a sample of the present disclosure (e.g., a sample that has been stained with reagents of certain aspects of the present disclosure). In some instances the apparatus is an imaging mass cytometer. In some instances the apparatus is a mass cytometer.

### Computer control of methods disclosed herein

The methods disclosed herein may also be provided as a computer program product including a non-transitory, machine-readable medium having stored thereon instructions that may be used to program a computer (or other electronic device) to perform the processes described herein. The machine-readable medium may include, but is not limited to, hard drives, floppy diskettes, optical disks, CD-ROMs, DVD-ROMs, ROMs, RAMs, EPROMs, EEPROMs, magnetic or optical cards, solid-state memory devices, or other types of media/computer-readable medium suitable for storing electronic instructions. Accordingly, the disclosure also provides a machine-readable medium comprising instructions for performing a method as disclosed herein.

### Definitions

The term "comprising" encompasses "including" as well as "consisting" e.g. a composition "comprising" X may consist exclusively of X or may include something additional e.g. X + Y. The term "about" in relation to a numerical value x is optional and means, for example, x+10%. The word "substantially" does not exclude "completely" e.g. a composition which is "substantially free" from Y may be completely free from Y. Where necessary, the word "substantially" may be omitted from the definition of the disclosure.

Molecular weights (Mₙ) and polydispersity indexes (PDI = M_{w}/Mₙ) were obtained by aqueous gel permeation chromatography (GPC), performed at room temperature, with 0.2 M NaNO₃ as the eluent. Molecular weights are referenced to polyethylene glycol (PEG) standards.

### EXAMPLES

### Example 1 - Histochemical Stains Imaging

In histology and histopathology, tissue is prepared for viewing under a microscope using either chemical fixation or frozen sections. To obtain an image of the tissue employing the imaging mass cytometry, the sections are stained with one or more metal-labelled affinity molecules such as antibodies or complimentary oligonucleotides. In this disclosure, in combination with immunohistochemistry using metal-labelled antibodies, the metal-containing stains are used to identify particular cellular structures.

The present disclosure describes reagents, methods, and kits for imaging mass cytometry. Certain aspects of the present disclosure relate to compounds such as Ruthenium Red used to identify mucinous stroma, Trichrome stain for identification of collagen fibers, osmium tetroxide as stain for cell membrane compartments.

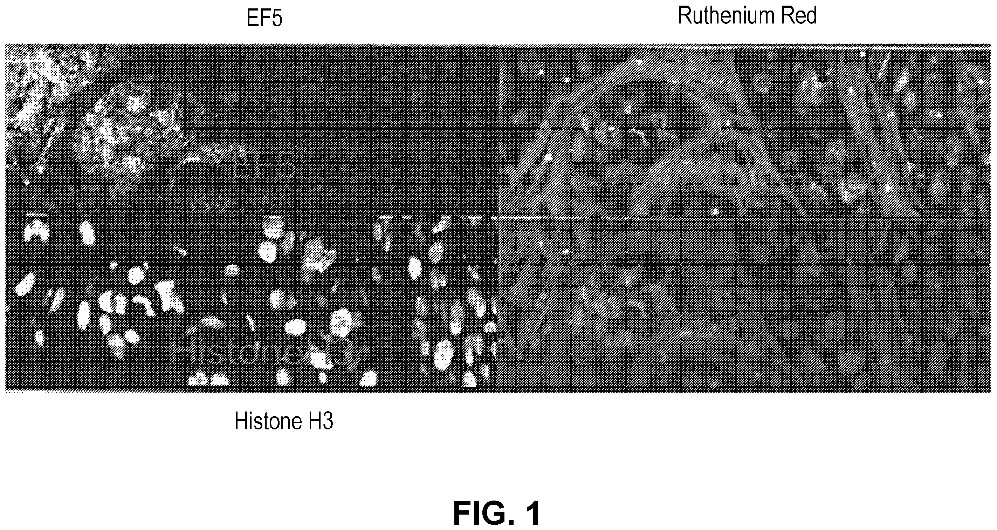

Fig. 1 shows Ruthenium Red staining of mucosal stroma. FFPE section of pancreatic adenocarcinoma xenograft tissue. The mouse was injected with nitroimidazole EFS and hypoxic regions are detected with metal-labelled anti-EFS antibodies. Nuclear staining is achieved using anti-Histone H3 metal-labelled antibody.

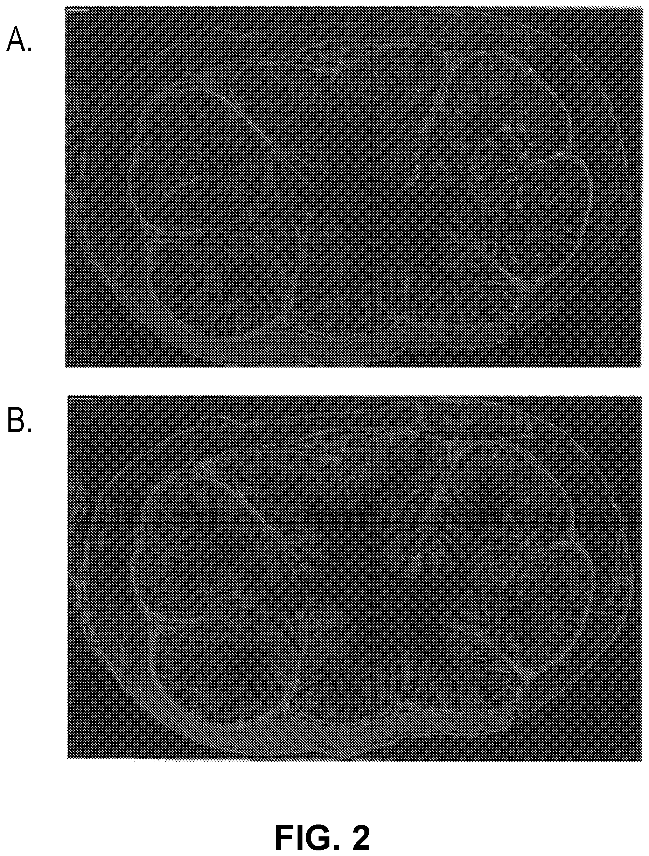

Fig. 2A shows an FFPE section of mouse gut that was stained with Trichrome stain, which contains phosphotungstic and phosphomolybdenic acids, for identification of collagen fibers (detection of 184 W in green is shown) and metal labelled antibodies (anti-E-Cadherin in red and anti-Histone H3 in green).

Fig. 2B shows staining with lododeoxyuridine (5-lodo-2'-deoxyuridine, IdU) is a synthetic thymidine nucleoside analog. IdU is used to allow the detection of growing or proliferating cells in living tissues. During the S-phase of cell division, DNA replication occurs, and IdU can be incorporated into the newly synthesized DNA by substituting for naturally occurring thymidine.

Conventional immunohistochemistry uses antibodies against IdU to visualize growing cells. IMC does not require the use of antibodies to this nucleotide analog. The illustration shows the same mouse gut, with mass cytometer channel set for 1271 (red). The mouse was injected with IdU prior to tissue harvest.

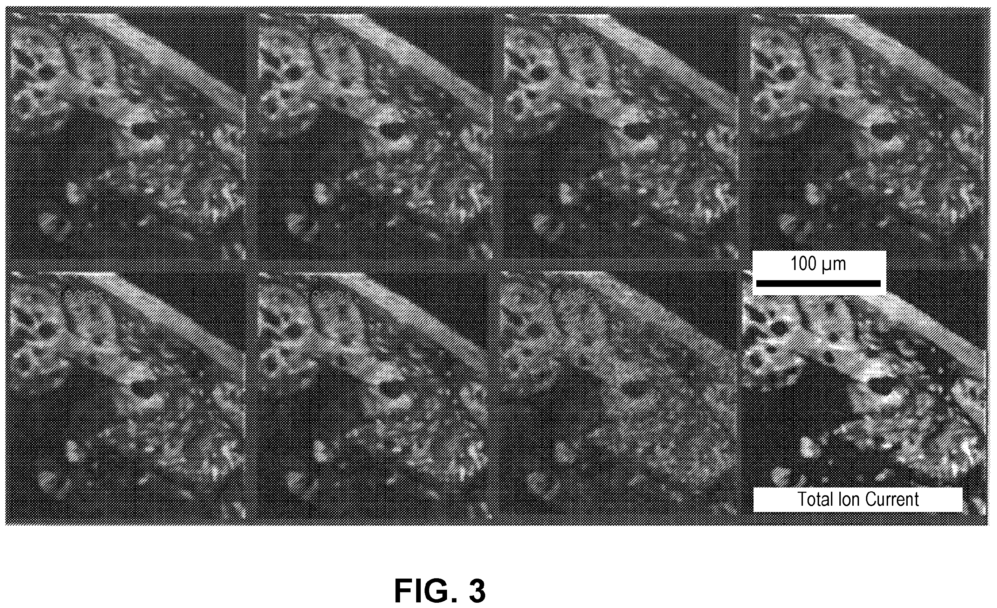

Fig. 3 shows Os04 postfixation is used widely as a contrast agent for electron microscopy micrographs. An example is given here of an ablation of mouse gut tissue embedded in Epoxy resin for Electron Microscopy. The tissue section is 1 um thick.

### Example 2 - Metal-Containing Drug Imaging

Imaging mass cytometry was used for direct visualization of platinum localization in tissue sections from tumor and normal tissues of cisplatin-treated mice bearing pancreas cancer patient-derived xenografts. This recently-developed technology enabled simultaneous detection of multiple markers to define cell lineage, DNA damage response, cell proliferation and functional state, providing a highly detailed view of drug incorporation in tumor and normal tissues at the cellular level. A striking and unanticipated finding was the extensive binding of platinum to collagen fibers in both tumor and normal mouse tissues. Time course experiments indicated the slow release of stroma-bound platinum, although it is currently unclear if released platinum retains biological activity. Imaging mass cytometry offers a unique window into the in vivo effects of platinum compounds, and it is likely that this can be extended into the clinic in order to optimize the use of this important class of agent.

The platinum-containing drugs are used extensively in the treatment of solid tumors. Cisplatin and the related compounds carboplatin and oxaliplatin form stable adducts with DNA, leading to the formation of lethal strand breaks. In contrast to other DNA-damaging agents, rapidly proliferating normal tissues including bone mar- row and intestinal mucosa are relatively resistant to cisplatin, and renal tubule damage is the limiting toxicity in humans.

Pancreatic cancers developing in individuals with germline mutations in the breast cancer-related genes BRCA1 and BRCA2 are unusually responsive to cisplatin. Thus, assignment of treatment towards platinum-containing regimens in BRCA-positive pancreatic cancer patients is an important step towards individualized treatment for these highly lethal cancers. However, clinical benefit is limited due to the development of drug resistance. Resistance to cisplatin is multifactorial, and includes alterations in drug uptake, efflux and detoxification that limit the initial DNA binding; enhanced capacity to excise DNA adducts; and suppression of death effector pathways.

Previous studies have used bulk assays to measure the amount of Pt in tissues obtained from patients following cisplatin treatment, with little information available about the localization of cisplatin within the tumor microenvironment and in normal tissues. Mass cytometry is able to detect elements in the mass range 75-209, and previously described its use to measure the uptake of platinum-based drugs in cell suspensions from human tumor xenografts. Here IMC was used to study the tissue distribution and quantitation of cisplatin in conjunction with multiple biomarkers in pancreatic cancer patient-derived xenografts. These studies revealed extensive binding of cisplatin to collagen fibers in the tumor stroma, as well as in normal tissues including kidney, intestine and skin.

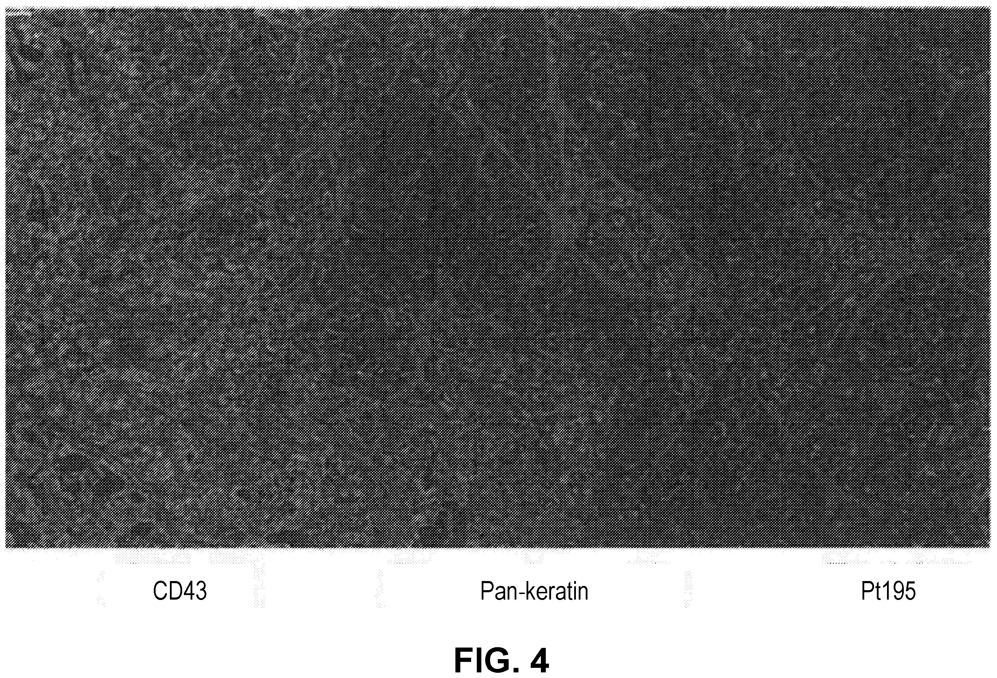

As shown in Fig. 4, immunodeficient mice received orthotopic transplantation of human pancreatic tumor tissue. Once the tumors were established, mice were injected with 40 mg/kg saline solution of cisplatin. Excised tumors were formalin-fixed and paraffin embedded, tissue sections cut at 5 micron thickness and processed for immunostaining and imaging by IMC. The image shows localization of cisplatin (measured by Pt195) in blue, mouse leukocytes in red (CD43 marker) and pancreatic carcinoma cells in green (Pan-keratin marker).

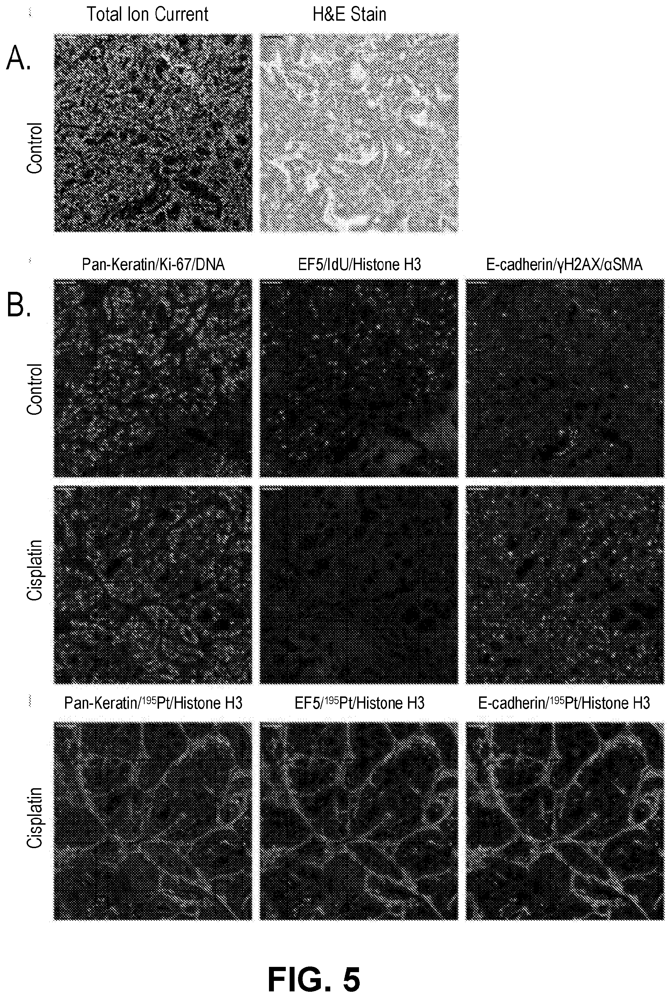

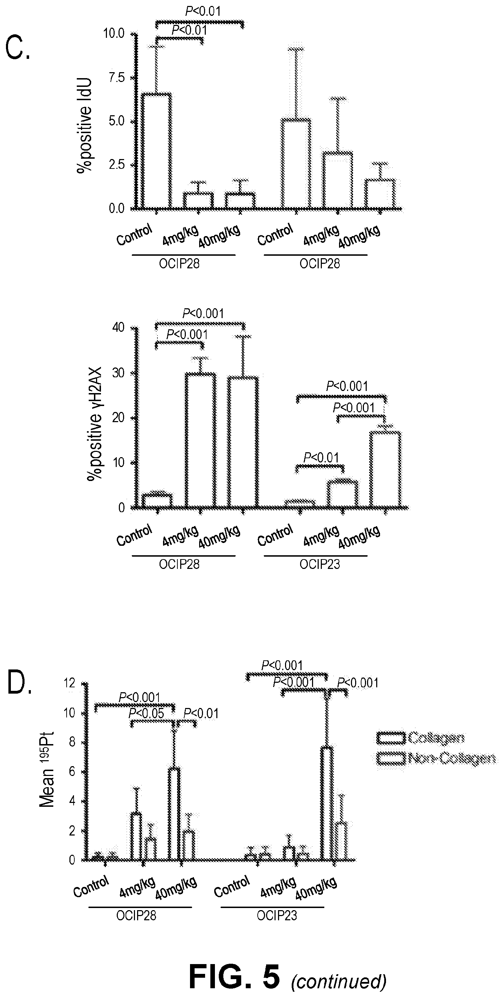

### Experimental Design for Figures 5-16:

The study was designed to test the potential of imaging mass cytometry to provide novel insights into the actions of cisplatin in patient-derived pancreatic cancer xenografts, selected based on the presence or absence of germline BRCA mutations. The research plan exploits IMC's unique ability for combined single cell measurement of Pt with panels of antibodies towards determinants of cell lineage, treatment response and tumor microenvironment, as well as direct measurement of 127I-IdU incorporation to assess DNA synthesis.

Animal experiments were carried out using protocols approved by the University Health Network Animal Care Committee. Fresh pancreatectomy samples that were superfluous to diagnostic needs were obtained from the University Health Network Tumor Tissue Bank (Toronto, Canada) according to a protocol that was approved by the University Health Network Research Ethics Board. Patient-derived xenografts (PDX) were established at the orthotopic site of 4- to 5-week-old severe combined immunodeficiency mice as previously described. Non-tumor bearing mice and two models, designated as OCIP23 and OCIP28 were used for these experiments. These models were selected from our extensive pancreatic PDX resource on the basis of similar growth characteristics and significantly different cisplatin sensitivity, with the BRCA2-mutant model OCIP28 being highly sensitive, while the BRCA-wild type OCIP23 is resistant20. Cisplatin was obtained from the Princess Margaret Hospital pharmacy (Toronto, Canada). Duplicate mice in each group were treated with a single intraperitoneal dose of 0, 4 mg/kg or 40 mg/kg cisplatin for 24 h, or at 4 mg/kg and sacrificed after 4 h, 24 h, 48 h or 7 days for non-tumor bearing mice. The 2-nitroimidazole hypoxia probe EF519 (obtained from Dr. Cameron J. Koch, University of Pennsylvania) at a dose of 30 mg/kg, and the thymidine analogue IdU (ThermoFisher, US) at 60 mg/kg, were given 4 h intravenously and 30 min intraperitoneally prior to sacrifice, respectively. Tumors and normal tissues (kidney, liver, small intestine, colon and skin) were excised, fixed and processed for paraffin embedding.

Additionally, monoisotopic cisplatin prepared from the stable isotopes 194Pt and 196Pt was obtained from Fluidigm Corporation (South San Francisco, CA, catalogue #201194 and 201196), sterile filtered, and injected intraperitoneally into tumor-bearing mice at a dose of 4 mg/kg. The first dose consisted of 194Pt-cisplatin, and a second dose of 196Pt-cisplatin was given 4 days later. Pairs of mice were sacrificed 24 h after the 196Pt-cisplatin dose.

FFPE sections were dewaxed and rehydrated as routine. Heat-induced epitope retrieval was conducted in a water bath at 95 °C in antigen retrieval buffer (R&D Systems Inc., Minneapolis, MN) for 10 min. After immediate cooling, the sections were blocked with 3% BSA in PBS for 45 min. Samples were incubated overnight at 4 °C with a final concentration of 5-10 µg/mL of metal-conjugated antibody cocktails diluted in PBS/0.5% BSA (Table 1). Samples were then washed with 2× PBS/0.1% Triton X-100 and 2× PBS and exposed to 25 µM Ir-Intercalator (Fluidigm) for 30 min at RT for nuclei staining. The samples were rinsed twice in distilled water and air-dried before IMC analysis.

The immunostained and dried samples are inserted into a novel laser ablation chamber (evolved from that described by Giensen, et al.) where the tissue is scanned by a pulsed deep UV laser focused to a 1 micrometer diameter spot size. Tissue from an ablation spot is totally vaporized on each laser shot and the plume is carried with high time-fidelity into the inductively coupled plasma ion source for simultaneous analysis by the mass cytometer (HeliosTM, Fluidigm). The metal isotopes associated with each spot are simultaneously measured and indexed against the location of each spot. The cross contamination between spots is less than 2% yielding sharp contrast images. The tissue is scanned spot-by-spot along a raster scan line, and sequential scan lines ultimately yield an intensity map of the target proteins throughout the tissue or the region of interest.

Data processing was performed using in-house-developed Wolfram Mathematica^{®} (V10.3) algorithms. The transient signal data were exported from the mass cytometer in text format. The file consists of push numbers (i.e., time) in rows and mass channels in columns, and the measured values are given as ion counts. Images of each mass channel were reconstructed by plotting the laser shot signals in the same order that they were recorded, line scan by line scan. Multi-dimensional RGB images were overlaid for desired channel combinations. Comprehensive tissue cell segmentation was performed using Definiens^{®} Developer XD platform for digital pathology (Definiens AG; Munich, Germany). The Definiens Developer platform is used to generate tissue-specific image segmentation utilizing pathologist-trained rulesets. The rulesets contain machine learning algorithms which are applied to extract features that distinguish individual cell type such as stroma and epithelium, among others. The morphological assessment of both tumor and non-tumor tissue was performed by an expert pathologist (IS). Epithelial and stromal cell regions were selected based on morphology, e.g. shape of cell, size of cell, nuclear and cytoplasmic features, location of cells, etc. Subsequently, specific antibodies were used to confirm appropriate labelling for epithelial and stromal cells, i.e. collagen I and αSMA antibodies for stromal, and Pan-keratin and E-Cadherin antibodies for epithelial regions (Supplementary Fig. 18). Table 1 contains parameters used for the segmentation algorithm. After a supervised cell and tissue identification, a high-throughput quantification of cellular staining intensity provides multiple channel data quantification at the same time in one particular tissue area.

The 134Xe+ ion, which is always present due to impurities in the argon plasma source in naturally occurring quantities, was monitored and used to normalize mean values for all reported channels for sensitivity fluctuations. 134Xe+ signal is uniformly distributed across all images, and its variability from image to image reflects a given state of the instrument tuning. The normalization algorithm includes construction of the 134Xe+ signal intensity histograms per image and following fit with a Gaussian function reflecting the counting statistics. The mean 134Xe+ signal values were used as normalization values. One image was arbitrarily chosen as a reference image (i.e. normalization factor = 1), and normalization factors were defined as the ratio of normalization values of a particular image and the reference image. Actual normalization of all channels was performed after the segmentation procedure.

The standard deviation of normalized values is taking into account the standard deviation of the measured values and the standard deviation of 134Xe ion signal. The means reported in the bar graphs are an average from multiple images. The standard deviations presented with these means account for additional errors in ion signal measurement that arise from variation between a. different areas of the same sample, and b. between different samples (i.e. different mice). Both errors arise from distinguishable sources, and the error bars represent the propagation of both errors. One-way ANOVA and Student's t-test were used to determine statistical significance. All statistical analysis was done using GraphPadTM Prism^{®} software (La Jolla, CA).

### Characterization of patient-derived xenografts:

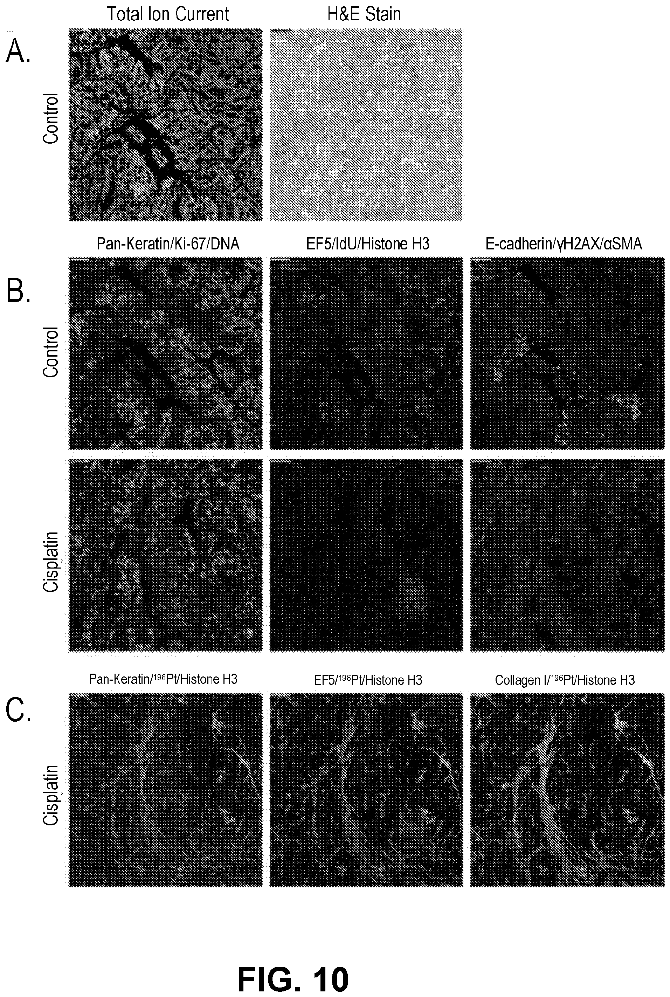

Grayscale images of tissue sections generated from the total ion current (TIC) at the detector per pixel were used as a proxy to an optical image. TIC, as a computer-generated image, showed similar morphological features to hematoxylin/eosin (H&E) stained sections from the same tissue block. Although it is expected that the best analogy should be achieved with the greatest number of detected channels, rational selection of nuclei, membrane and stroma markers is helpful. As illustrated in Fig. 5 and Supplementary Fig. 10, the PDX models OCIP28 and OCIP23 have typical features of pancreatic ductal adenocarcinoma, with the malignant epithelium arranged into glandular structures embedded in a fibrous stroma. For illustrative purposes, combinations of images generated from the individual IMC markers were false colored and overlaid to show their spatial correlations. For example, as seen in Fig. 5b, cells incorporating IdU also express the proliferation marker Ki-67, and they are absent from regions of hypoxia marked by the tracer EF5, as previously observed by fluorescence imaging.

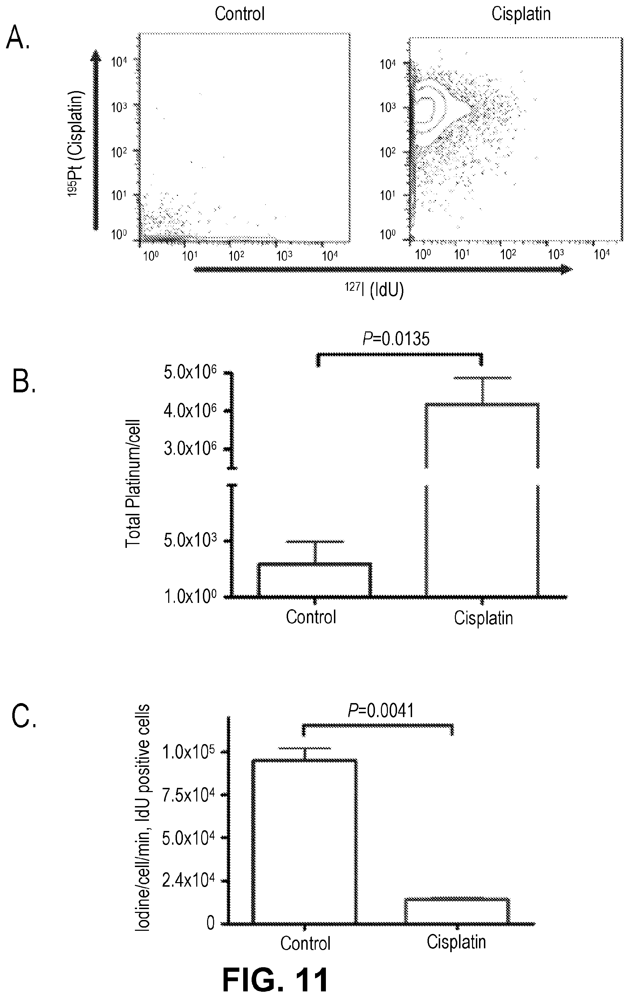

Expression of the DNA damage marker γH2AX is substantially confined to necrotic tissue adjacent to regions of hypoxia. Also seen are abundant cells expressing the mesenchymal marker αSMA, interspersed between the cytokeratin and E-cadherin-expressing epithelial cells. Following treatment with 4 mg/kg cisplatin, which roughly corresponds to the dose intensity used in humans, BRCA-mutant OCIP28 tumors showed loss of IdU incorporation and a striking increase in γH2AX expression, whereas these effects were much less apparent in BRCA-wild type OCIP23 that is resistant to cisplatin20. Even when the dose of cisplatin was increased to 40mg/kg the effects on IdU uptake and γH2AX in OCIP23 were less than those in the BRCA-mutant model at the lower dose, (Fig. 5c and Supplementary Fig. 10). Cell suspensions were prepared following published protocols from fresh tumor pieces after cisplatin treatment, and whole-cell analyses were made by mass cytometry (CyTOF^{®} 2). The values for Pt atoms/cell and IdU incorporation (iodine-127I/cell/minute) are shown in Supplementary Fig. 11, and are very similar to those previously obtained with cell line-derived xenografts.

### Cisplatin distribution in tumor tissue:

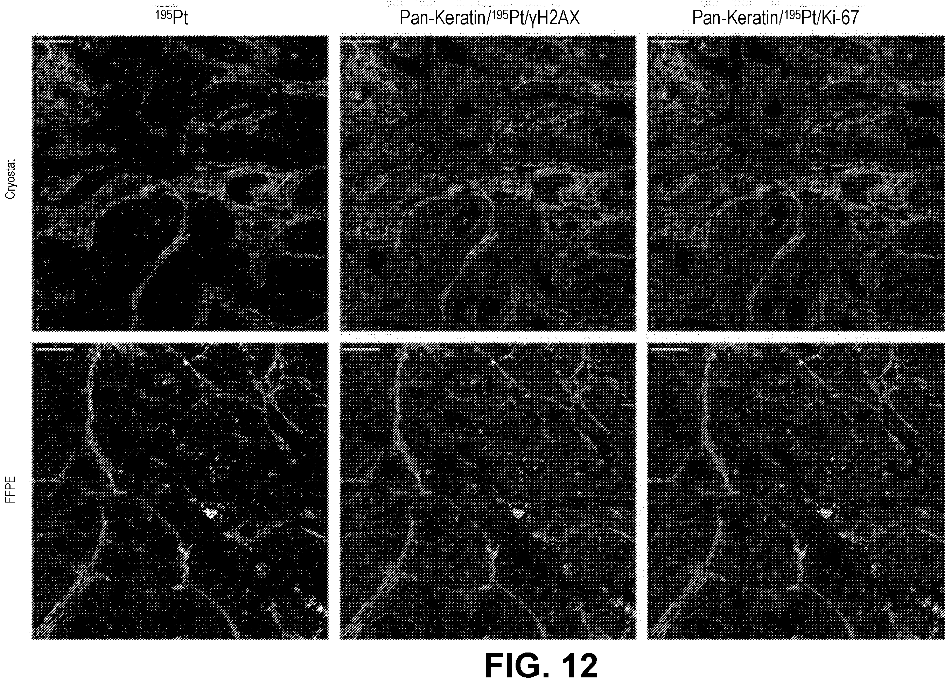

Images of each of the major isotopes of platinum were readily captured by IMC: the most abundant form, 195Pt, is used for illustration. Tissue sections prepared by formalin fixation and paraffin embedding undergo extensive washing and solvent treatment, which might remove loosely bound cisplatin. Therefore, it was first examined paraffin-embedded and cryostat sections cut from snap frozen samples prepared from the same tumor. It was found that the intensities and distribution patterns of platinum labelling were very similar, with no appreciable loss of signal in the paraffin sections, indicating that Pt remaining 24 hours after cisplatin treatment is, most likely, tissue-bound (Supplementary Fig. 12). Therefore, most of the work was done using FFPE sections. At 4 mg/kg cisplatin, Pt was detected above background in OCIP28 tumors, but not in OCIP23 where it was, however, readily detected at the higher dose.

Unexpectedly, the intensity of 195Pt was considerably greater in the stromal component than in the tumor epithelial tissue. Additional staining for Collagen I showed co-localization with 195Pt, and it was intimately associated with the mesenchymal marker αSMA, as expected (Fig. 5d). Strong collagen binding of Pt was observed in both PDX models tested, and the intensity was highly significant when compared to epithelial tumor tissue (Fig. 5e).

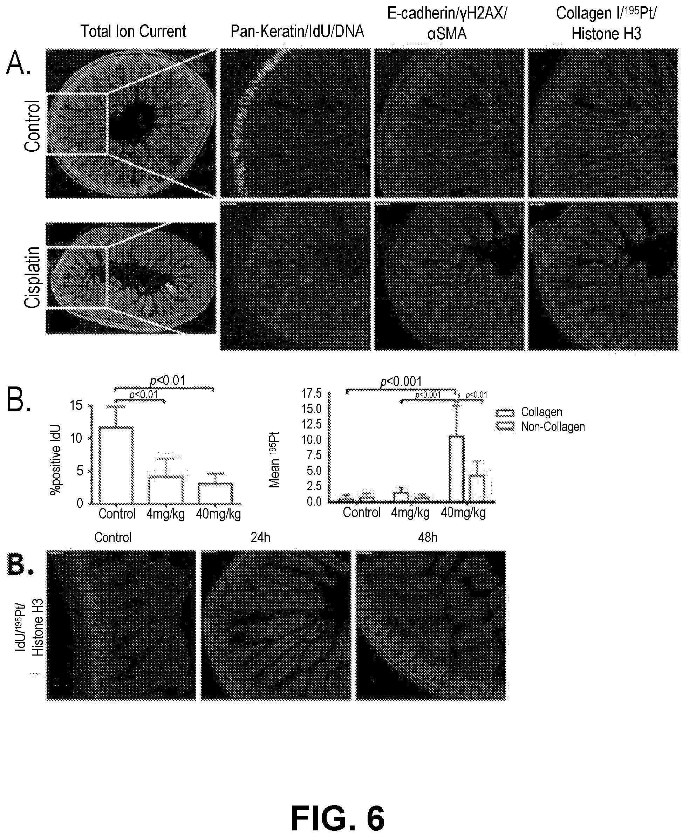

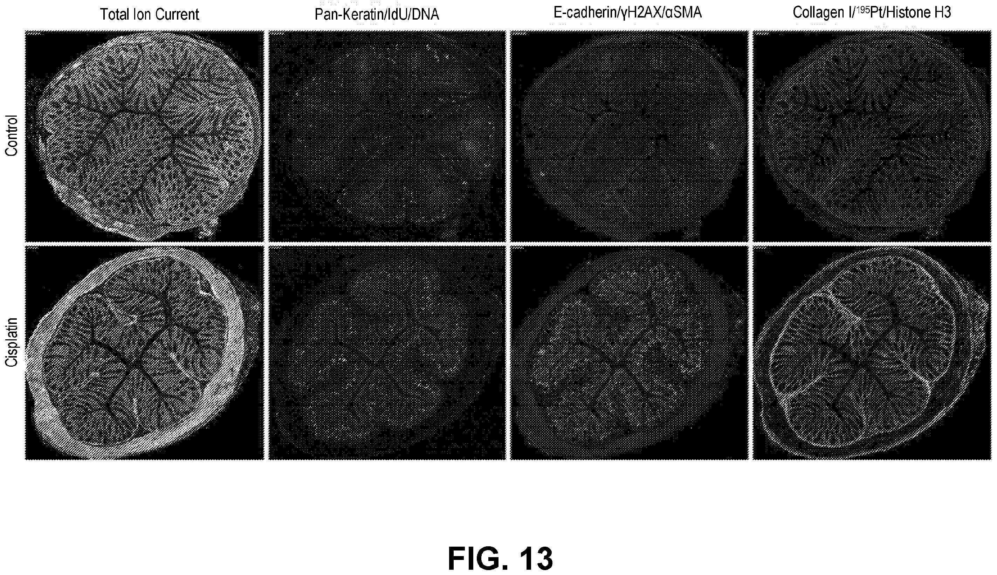

Cisplatin distribution in normal intestine: The small intestine and colon were harvested from mice receiving 4 mg/kg cisplatin, with IdU given 30 min prior to sacrifice. Paraffin sections were cut for IMC staining using the same antibody cocktail as for the tumor tissue (Table 1). As shown in Fig. 6, there was intense IdU uptake by the basal crypt cells of untreated control mice, whereas the labelling of colonic mucosa was lower (Supplementary Fig. 13). DNA synthesis was strongly inhibited following cisplatin treatment, accompanied by increased γH2AX labelling. As in tumor tissue, there was extensive binding of platinum to collagen in the lamina propria and outer wall of the small intestine, as well as in apical villous epithelial cells, and a similar pattern was also observed in the colon (Supplementary Fig. 13). Interestingly, at 48 hours after cisplatin treatment the IdU uptake by the basal crypt cells recovered, despite the persistence of platinum binding to collagen in the underlying basement membrane (Fig. 6c).

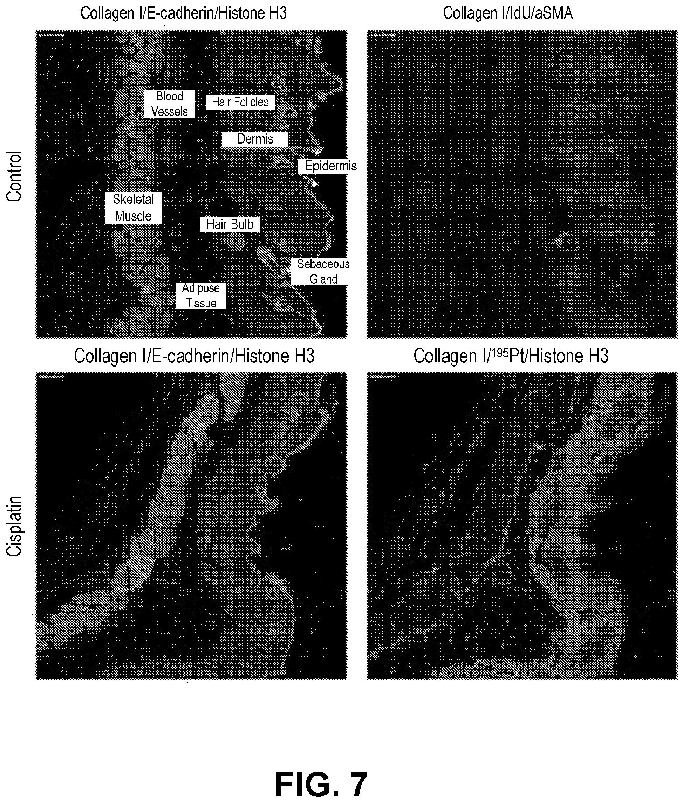

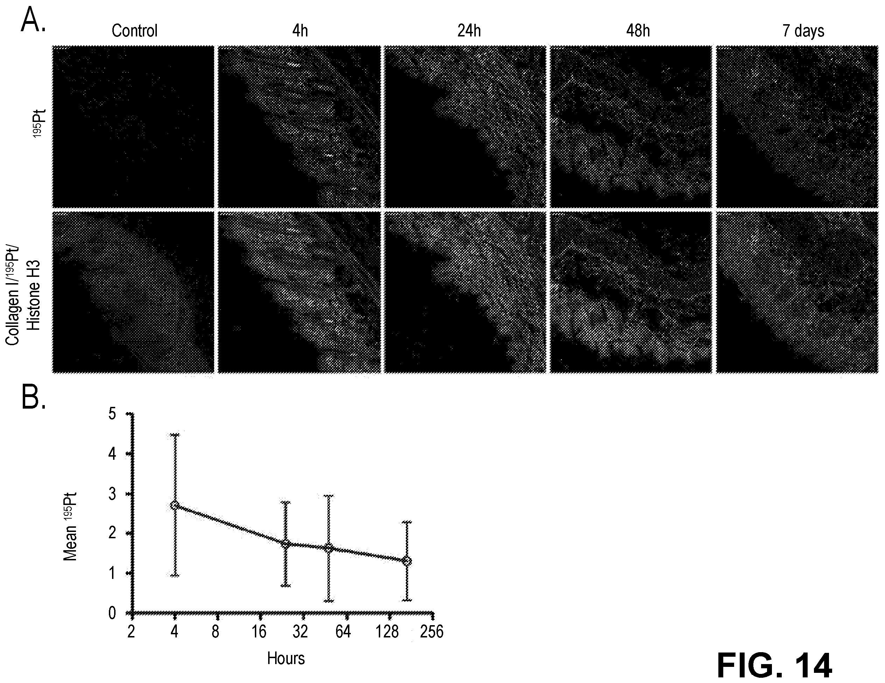

Cisplatin distribution in skin: Sections cut through the thickness of the abdominal wall illustrate the histological features of several distinct tissues as viewed by IMC (Fig. 7). Similar to the intestinal wall and tumor tissue, binding of platinum to the dermal collagen was striking. Skin samples were taken from mice at 4 h, 24 h, 48 h, and seven days after cisplatin treatment, in order to determine the rate of loss of collagen-bound platinum. As illustrated in Supplementary Fig. 14a, dermal platinum decreased over time following a single dose of 4 mg/kg cisplatin (Supplementary Fig. 14b).

### Cisplatin in normal kidney and liver:

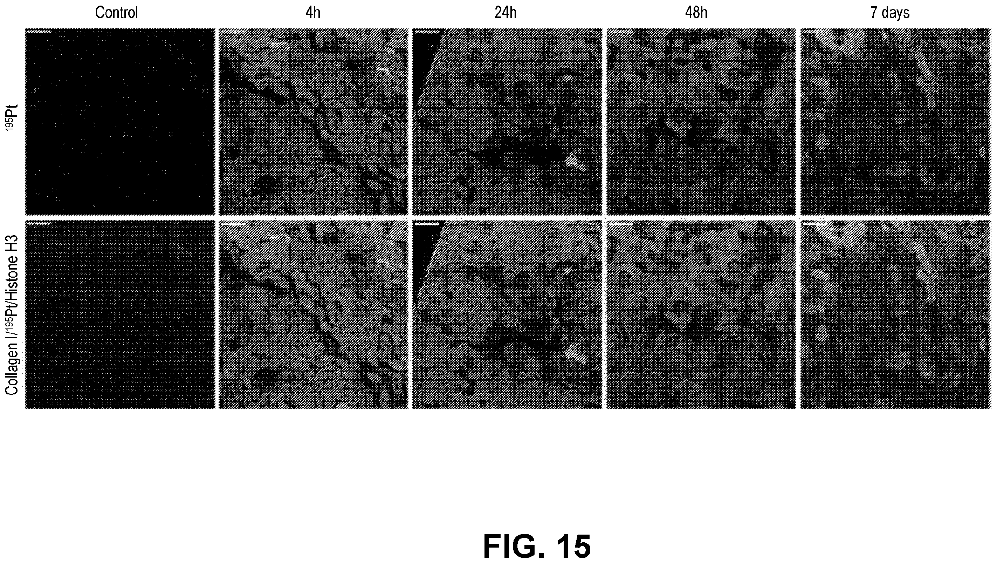

The kidney and liver are the major organs involved in drug metabolism and excretion, although the liver plays a minor role in the case of cisplatin, which is mainly eliminated in the urine21. Renal tubular damage is the most serious toxic effect of cisplatin in humans7. Examination of the kidneys by IMC 24 hours after 4 mg/kg cisplatin administration showed a striking pattern, with large amounts of platinum in the cortical tubules and little accumulation in the glomeruli or renal medulla (Fig. 8a). Furthermore, high levels of platinum persisted for up to seven days post treatment, consistent with nephrotoxicity of this agent (Supplementary Fig. 15). In contrast, very little platinum was detected in the liver post-cisplatin (Fig. 8b). However, a distinct pattern of platinum accumulation was noted demonstrating a zone 3 sinusoidal and perivenular distribution that does not correlate with collagen (Fig. 8b, inset).

### Discussion:

In the present experiments, the unique potential of IMC was exploited to image the cellular content of platinum in BRCA-mutant and wild-type patient-derived pancreatic cancer xenograft-bearing mice treated with cisplatin, and in key normal host tissues. In combination with incorporation of 1271-IdU and the 2-nitroimidazole hypoxia tracer EF5, the expression of the DNA damage response marker γH2AX, and cell lineage-specific biomarkers, a number of novel and clinically important observations were made.

Platinum was readily detected in tissue sections following treatment with 4 mg/kg cisplatin, which gives drug exposure similar to that achieved in patients. Although the free drug is rapidly cleared through the kidneys, it is extensively protein bound which is consistent with our observation that Pt levels in formalin-fixed, paraffin-embedded tissue are comparable to those in frozen sections. In the BRCA2-mutant PDX OCIP28, there was pronounced loss of IdU incorporation and increased yH2AX labelling following treatment with 4 mg/kg cisplatin, in line with our previous observation that this model is highly responsive to cisplatin, and explained by deficient homologous repair (HR) consequent to the loss of BRCA2. In contrast, the HR-proficient model OCIP23 showed little response to 4 mg/kg cisplatin, and relatively modest effects on IdU and yH2AX when the dose was increased to 40 mg/kg. Although these findings are consistent with the clinical observation that pancreatic cancers developing in individuals with germline BRCA1/2 mutations can be highly sensitive to platinum drugs9, we note that Pt levels in the epithelial compartment of OCIP28 were significantly greater than those in OCIP23 following the clinically-relevant dose of 4 mg/kg. Resistance to cisplatin is multifactorial, and includes drug transport and detoxification pathways that limit cellular accumulation of the drug. It is possible that these mechanisms are expressed to a greater extent in OCIP23, and explain in part the differences in cisplatin sensitivity. This could be further studied exploiting the unique ability of IMC to measure Pt levels in combination with multiple antibodies directed towards proteins linked to cisplatin resistance, including the MRP2 efflux pump and enzymes involved in glutathione metabolism, and extended to include additional PDX models. Fresh insights gained by such a study could have significant clinical utility, given the importance of platinum drugs in cancer treatment.

Using IMC, we obtained images of a range of normal tissues at a resolution comparable to that of light micros- copy. Cisplatin is most active against proliferating cells, yet it appears to have little harmful effect on the intestinal mucosa of cancer patients. We were therefore interested to compare cisplatin accumulation and pharmacological effects in the small intestine basal crypt cells to those in the PDX. As shown in Fig. 6, IdU uptake by the crypt cells was greatly reduced 24 h following cisplatin, accompanied by increased γH2AX. This indicates that cisplatin has a significant acute effect on the small intestinal mucosa, but after 48 h IdU incorporation restarted, suggesting that these cells are able to repair drug-induced damage during a brief period of cell cycle arrest, and then resume normal growth.

Renal tubule damage is the most important side effect of cisplatin in humans, and it is only partially prevented by aggressive hydration regimens7. Accordingly, we noted intense Pt uptake by the tubular cells of the renal cortex that persisted over time, compared to the glomeruli and renal medulla, where Pt was virtually absent. This is in contrast with an earlier autopsy study of cancer patients receiving cisplatin, where the levels of Pt in cortex and medulla were similar24. This might be explained by species differences, or the longer duration of treatment in the patients. The ability to image Pt at subcellular resolution using IMC offers a novel approach to the study of platinum-induced nephrotoxicity, and to explore new approaches to mitigate what remains a significant problem in clinical oncology.

Hepatotoxicity of platinum-based drugs has been previously described and well recognized25. Our observation that cisplatin accumulates in the liver, revealing a zone 3 sinusoidal and perivenular distribution, is of interest, since the mechanism of injury is believed to be damage to the sinusoidal endothelial cells in the liver followed by subsinusoidal fibrosis, obstruction and congestion, leading to veno-occlusive disease/sinusoidal obstruction syndrome.

A striking and unanticipated finding was the extent of platinum binding to collagen, which was considerably greater than the epithelial cell Pt in both tumor tissue and normal intestine. Intense Pt binding to dermal collagen; a site that is readily accessible to punch biopsy to allow a similar study in patients receiving cisplatin. A time course study found that dermal collagen-bound Pt is slowly released, but it is unclear if it remains pharmacologically active. However, it is of interest that platinum can be detected in patients' plasma for several years post treatment with cisplatin, and that the levels are correlated with long term neuro- and ototoxicity.

Furthermore, ultrafiltrates of patient plasma were able to form DNA adducts, indicating the retention of biological activity. The source of plasma platinum in these patients remains uncertain, although it is believed to be tissue-bound. Early studies suggested that cisplatin is inactivated following protein binding29, but there are reports of benefit from intralesional treatment with cisplatin/collagen gels, with pharmacokinetic data consistent with slow release of drug in cancer patients. Based on the IMC finding that cisplatin is extensively bound to collagen, it could suggest to have a significant effect within the tumor microenvironment due to the slow release of drug in sufficient amounts to affect tumor growth. It is reported that continuous exposure to low dose metronomic chemotherapy improves the therapeutic index of some cytotoxic agents.

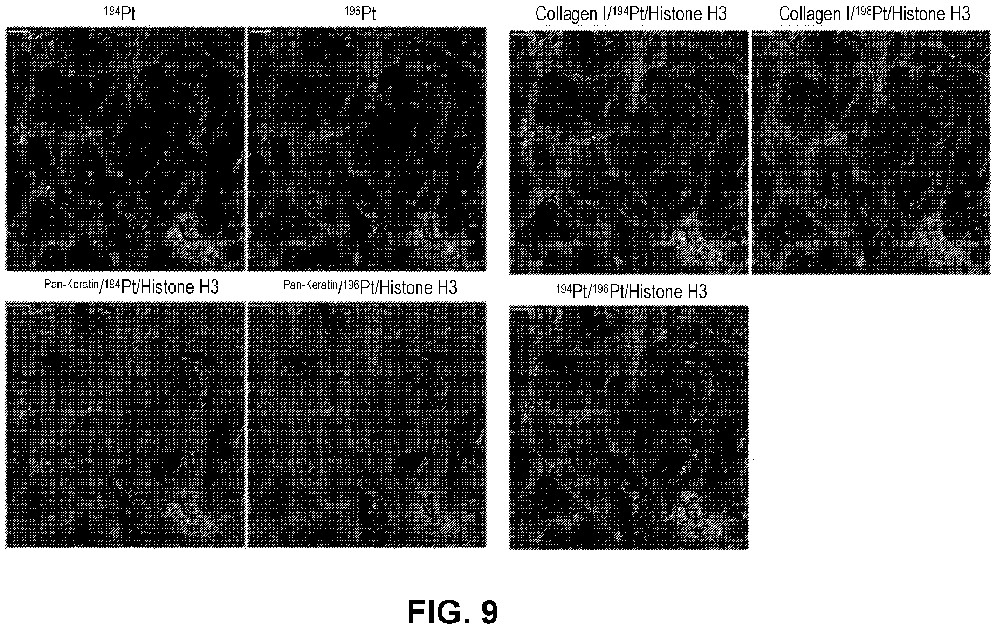

The most significant new finding using IMC is the extensive binding of cisplatin to collagen within the tumor stroma and in normal tissues. The main limitations of the present study are: 1) it does not establish if collagen-bound cisplatin remains active; 2) the other clinically-important platinum drugs, carboplatin and oxaliplatin, were not included; 3) it does not establish if these effects also occur in cancer patients. To address this monoisotopic 194Pt and 196Pt forms of cisplatin were used for in vivo pulse/chase, to examine if collagen-bound cisplatin redistributes over time, and some preliminary data are shown in Fig. 9. In this experiment, tumor-bearing mice were treated with 4 mg/kg 194Pt-cisplatin on Day 1, 4 mg/kg 196Pt-cisplatin on Day 5, and sacrificed on Day 6. As shown in Fig. 9, the two isotopes showed a similar distribution pattern, with cisplatin remaining substantially bound to collagen. However, a loss of 194Pt compared to 196Pt was noted, indicating its slow dissociation from collagen.

Additionally, monoclonal antibodies to Pt-DNA adducts have been described, and the inventors will test if these can be used successfully to allow dual measurement of Pt biodistribution and drug-induced DNA damage using IMC. If so, this could be used to test if monoisotopic cisplatin, released over time from the tumor stroma, produces DNA damage in adjacent cancer cells. Although IMC has not yet been applied to carboplatin- or oxaliplatin- treated tumors, the inventors have previously shown that Pt is readily detected in oxaliplatin-treated tumor cells using the CyTOF, suggesting that IMC will be equally useful studying these agents in animal models, and also in patient samples.

Our plans for clinical translation involve two lines of research. One is a clinical study of collagen binding by cisplatin and oxaliplatin, based on IMC analysis of sequential skin biopsies obtained during treatment, compared to the corresponding plasma protein-bound and ultrafiltrable platinum. If collagen-bound platinum is the main source of plasma Pt post-treatment, then the two values are expected to correlate. In a separate group of patients treated with neoadjuvant platinum-based chemotherapy, the extent to which Pt binding to tumor collagen matches that in the patient's skin will be determined, and if tumor Pt is correlated with treatment response. Our other research direction, and the original motivation for this work, is to investigate the mechanisms of platinum sensitivity and resistance in pancreatic cancers. It exploits the unique capability of IMC for single cell Pt measurements in combination with large panels of antibodies directed to the known determinants of treatment response, including proteins involved in drug transport and detoxification, as well as initial DNA damage and damage responses.

In summary, the findings reported in this paper affirm that imaging mass cytometry represents a powerful new platform for studying complex biological processes in intact tissue. Although the main impact is expected to be in the area of cancer pathology, our results point to as-yet under-explored applications to the study of normal tissues and non-malignant pathology.

Fig. 5 shows cisplatin effects on tumor proliferation, DNA damage and cisplatin distribution in the tumor. (A) Representative total ion current image (left) and H&E stain (right) of control OCIP28. Scale bar = 100 µ m. (B) Representative Pan-Keratin, Ki-67, DNA, EF5, IdU, Histone H3, E-cadherin, γ H2AX, and α SMA images of tumors from control and cisplatin-treated (40 mg/kg for 24 h) mice. Scale bar = 100 µ m. (C) Percentage of positive IdU (top) and γ H2AX (bottom) bar graph of OCIP28 and OCIP23 xenografts. Quantitative results were obtained in all figures using the segmentation algorithm of Definiens Developer. (D) Representative Pan-Keratin, EF5, Collagen I, 195Pt, and Histone H3 images of cisplatin-treated (40 mg/kg for 24 h) in OCIP28. Scale bar = 100 µ m. (E) Platinum distribution in collagen and non-collagen viable tumor tissue area in OCIP28 and OCIP23 xenografts.

Fig. 6 shows cisplatin effects and distribution in the small intestine. (A) Representative total ion current images, Pan-Keratin, IdU, DNA, E-cadherin, γ H2AX, and α SMA, Collagen I, 195Pt, and Histone H3 images of small intestine from control and cisplatin-treated (4 mg/kg for 24 h) OCIP28 PDX mice. Scale bar = 100 µ m. (B) Percentage of positive IdU bar graph (left) and platinum distribution in collagen and non-collagen epithelial tissue area of small intestine. (C) Representative IdU, 195Pt, and Histone H3 images of control, cisplatin-treated (4 mg/kg for 24 h and 48 h) small intestine. Scale bar = 100 µ m.

Fig. 7 shows histological features of skin identified by IMC, and distribution of platinum following treatment with cisplatin. Representative Collagen I, E-cadherin, Histone H3, IdU, and α SMA images of control (top) and cisplatin-treated (4 mg/kg for 24 h, bottom) mouse skin. Scale bar = 100 µ m.

Fig. 8. shows cisplatin distribution in kidney and liver. (a) Representative Collagen I, 195Pt, and Histone H3 images of cisplatin-treated (4 mg/kg for 4 h) mouse kidney. Scale bar = 100 µ m. (b) Representative total ion current image (left) and Collagen I, 195Pt, and Histone H3 image (right) of cisplatin-treated (4 mg/kg for 24 h) mouse liver.

Fig. 9 shows kinetics of cisplatin distribution measured using monoisotopic cisplatin. Tumor-bearing mice were treated with 194Pt-cisplatin on Day 1, 196Pt-cisplatin on Day 5, and sacrificed on Day 6. The images are of a single tissue section showing the relative distribution and abundance of the two isotopes (top; grayscale), Pan-Keratin, Collagen I and Histone H3 vs Pt (lower panels). The bottom panel shows the two isotopes, with the color intensity indicating the preponderance of 196Pt at this time point. Scale bar = 100 µm.

Fig. 10 shows cisplatin effects on tumor proliferation, DNA damage and ¹⁹⁵Pt distribution in OCIP23. (A) Representative total ion current image (left) and H&E stain (right) of control OCIP23. Scale bar = 100 µm. (B) Representative Pan-Keratin, Ki-67, DNA, EF5, IdU, Histone H3, E-cadherin, γH2AX, and αSMA images of OCIP23, control and 40 mg/kg cisplatin-treated mice for 24 h. Scale bar = 100 µm. (C) Representative Pan-Keratin, EF5, Collagen I, ¹⁹⁵Pt, and Histone H3 images of cisplatin-treated (40 mg/kg for 24 h) OCIP23 tumor-bearing mice. Scale bar = 100 µm.

Fig. 11 shows cisplatin effects on cell proliferation and platinum uptake determined by mass cytometry. (A) Representative plots of 127I versus 195Pt in control and cisplatin-treated (40 mg/kg for 24 h) OCIP28 tumor-bearing mice. (B) Total platinum uptake of control and cisplatin-treated (40 mg/kg for 24 h) OCIP28 tumors. (C) Iodine atoms per cell data following a 30 min IdU pulse for identification of S-phase cells following a single dose of cisplatin.

Fig. 12 shows platinum distribution in cryostat and FFPE sections. Representative 195Pt, Pan-Keratin, Ki-67, and yH2AX images of OCIP28 cisplatin-treated (40 mg/kg for 24 h) cryostat (top) and FFPE tumor sections (bottom). Scale bar = 100 µm.

Fig. 13 shows platinum distribution in large intestine. Representative total ion current, Pan-Keratin, IdU, DNA, E-cadherin, γH2AX, αSMA, Collagen I, 195Pt, and Histone H3 images of large intestine from control (top) and cisplatin-treated (40 mg/kg for 24h, bottom) OCIP23 mice. Scale bar = 100 µm.

Fig. 14 shows platinum distribution in non-tumor bearing mouse skin. (A) Representative 195Pt (gray, top) and Collagen I, 195Pt, and Histone H3 (bottom) images of control and cisplatin-treated (4 mg/kg for 4 h, 24 h, 48 h, and 7 days) mouse skin. Scale bar = 100 µm. (B) Time course of platinum distribution in dermal collagen.

Fig. 15 shows platinum distribution in non-tumor bearing mouse kidney. Representative 195Pt (gray, top) and Collagen I, 195Pt, and Histone H3 (bottom) images of control and cisplatin-treated (4 mg/kg for 4 h, 24 h, 48 h, and 7 days) mouse kidney. Scale bar = 100 µm.

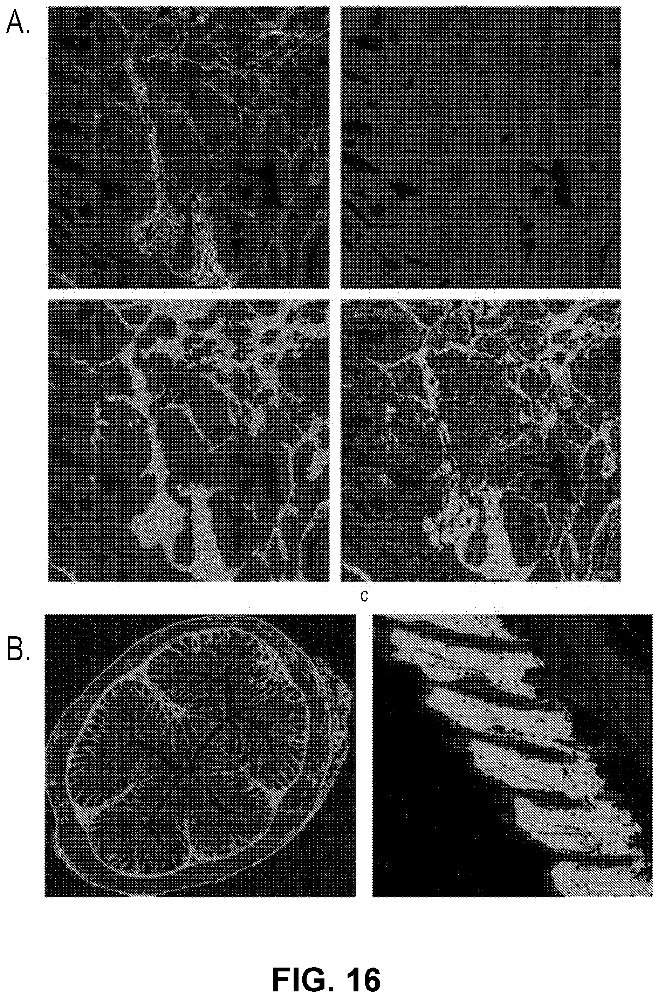

Fig. 16 shows Definiens Developer image analysis. (A) Representative image of cisplatin-treated (40 mg/kg for 24 h) OCIP28 tumor. Top left: E-cadherin (red), γH2AX (green), DNA (blue), and Collagen I (cyan). Top right: the epithelium layer (red) is identified based on E-cadherin and Pan-Keratin. The stroma layer (green) is identified based on αSMA and Collagen I. Lumen is in black. Bottom left: Epithelial cells (red) and stromal cells (cyan) are identified based on DNA, and grown based on the ratios of stain present. Bottom right: The collagen layer (cyan) is identified from the stroma layer (blue). (B) The collagen layer (cyan) is identified from the stroma layer (blue) in mouse large intestine. (C) The collagen layer (cyan) is identified from the stroma layer (blue) in mouse skin.

### Example 3 - Reagents used in Examples 4-12, and characterization techniques

Azido-PEG3-maleimide, TCO-PEG3-maleimide were purchased from Click Chemistry Tools (Scottsdale, AZ). 1,4,7,10-tetraazacylcododecane-1,4,7,10- tetraacetic acid (DOTA) was purchased from Macrocylics Inc. tert-butyl 2-aminoethylcarbamate, trifluoracetic acid were purchased from Sigma Aldrich Canada. Amicon Ultra-15 centrifugal filter units were purchased from Millipore Canada. DBCO-PEG5-NHS and TCO-PEG5-NHS were purchased from Click Chemistry Tools (Scottsdale, AZ). Alexa Fluor^{™} 594 C5 Maleimide was supplied by Sigma Aldrich Canada (Oakville, Ontario). All Abs were purchased from Biolegends (San Diego, CA) unless otherwise noted. Phalloidin Amine (Mwt.=~902 Da) was purchased from AAT Bioquest (Sunny Vale, CA) and Lectin from Triticum vulgaris (WGA, ~Mwt=38kDa) was purchased from Sigma Aldrich Canada (Oakville, Ontario).

Human Leukemia cell lines were obtained from ATCC (American Type Culture Collection, Manassas, VA). All cells were daily maintained in Dulbecco's modified eagle medium (DMEM) supplemented with 2 mM L-glutamine and 10% heat inactivated fetal bovine serum (FBS) at 37 □C with 5% CO2. Cells were sub cultured every 2-3 days to maintain sub confluency. Cryopreserved Peripheral blood mononuclear cells (PBMC, Catalog Number: CTL-UP1) and anti-aggregate wash supplement 20× (Catalog Number: CTL-AA-001) were purchased from Cellular Technology (Shaker Heights, OH). For all mass cytometry experiment cell viability was typically >90%.

| **Reagent** | **Name (clone)** | **Catalog No.** | **Vendor** |
|---|---|---|---|
| **Maxpar^{®} antibodies** | ¹⁷⁰Er- CD3 (UCHT1) | 31170001B | Fluidigm |
| | ¹⁵⁴Sm- CD45 (HI30) | 3154001B | Fluidigm |
| | ¹⁶⁰Gd- CD14 (M5E2) | 3160001B | Fluidigm |
| | ¹⁴⁷Sm- CD20 (2H7) | 3147001B | Fluidigm |
| | ¹⁴⁵Nd- CD4 (RPA-T4) | 3145001B | Fluidigm |
| | ¹⁴⁶ Nd- CD8a(RPA-T8) | 3146001B | Fluidigm |
| | ¹⁴⁸Nd- CD16 (3G8) | 3148004B | Fluidigm |
| | ¹⁶⁶ Er- CD34 (581) | 3166012B | Fluidigm |
| | ¹²Yb- CD57 (HCD57) | 3172009B | Fluidigm |
| | ¹⁵¹Eu- CD107a (H4A3) | 3151002B | Fluidigm |
| **Antibodies for Click Conjugation** | LEAF^{™} CD3 (UCHT1) | 300414 | Biolegend |
| | LEAF^{™} CD14 (M5E2) | 301810 | Biolegend |
| | LEAF^{™} CD16 (3G8) | 302014 | Biolegend |
| | LEAF^{™} CD4 (RPA-T4) | 300516 | Biolegend |
| | LEAF^{™} CD57 (HCD57) | 322325 | Biolegend |
| | CD3(UCHT1) | 300443 | Biolegend |
| **Fluidigm cell identification** | Intercalator-Rh (¹⁰³Rh) | 201103A | Fluidigm |
| | Intercalator-Ir (¹⁹¹Ir191/ ¹⁹³Ir) | 201192A | Fluidigm |

The nominal molecular weights (Mn) and polydispersities (Mw/Mn) of all anionic, water-soluble samples were measured with a Viscotek gel-permeation chromatograph (GPC) equipped with a Viscotek VE3580 refractive index detector, and Polyanalytik AquaGel PAA-203 and PAA-204 columns (kept at room temperature). The flow rate was maintained at 0.7 mL/min using a Viscotek VE1122 Solvent Delivery System and VE7510 GPC Degasser. An eluent of 0.2 M KNO₃, 200 ppm NaN₃, was used. The system was calibrated with poly(ethylene glycol) standards.

1H NMR. 1H NMR (400 MHz) spectra were recorded on a Varian Hg 400 or a Varian Vnmr S 400 spectrometer with a 45° pulse width and at a temperature of 25 °C. All water-soluble polymers were dissolved in D₂O, with chemical shifts referenced to the HDO peak at 4.77 ppm. Polymers were analyzed with 512 transients and a delay time of 10 s.

The number of DBCO molecule per protein was determined by measuring the absorbance at 309 and 280 nm using NanoDrop^{™} 2000/2000c Spectrophotometer (Thermofisher Scientific, Waltham, MA). The obtained absorbance values and the respective molar extinction coefficients used to calculate the molar concentrations and degree of DBCO labeling for each conjugate.

ICP-MS analysis employed an ELAN 9000 instrument to determine the conjugation efficiency of mass tags with the biomolecules. Metal-labeled conjugates were diluted with 2 vol % HNO₃ until ppb concentrations were obtained. Standard lanthanide solutions were prepared by a series of 10-fold dilutions of a stock solution (1000 mg/L, 2% HNO₃, PerkinElmer) containing the desired lanthanide elements.

### Example 4 - Preparation of azide-terminated, diethylene triamine pentaacetic acid (DTPA) substituted polyacrylamide

50 mg of azide-terminated poly(methyl acrylate) ((Number average molecular weight (Mₙ) = 2000-3000 gmol⁻¹, polydispersity index (PDI) < 1.30); Sigma Aldrich (product number: 699764)) was dissolved in 1 mL of methanol at room temperature, to which 2 mL (1.8 g, 29.6 mmol) of ethylenediamine (Sigma Aldrich) was added. (Molecular weights (Mₙ) and polydispersity indexes (PDI = M_{w}/Mₙ) were obtained by aqueous gel permeation chromatography (GPC), performed at room temperature, with 0.2 M NaNO₃ as the eluent. Molecular weights are referenced to polyethylene glycol (PEG) standards.) The solution was stirred at room temperature overnight. The polymer was then precipitated from solution dropwise into 50 mL of diethyl ether. The polymer solid was isolated and washed twice with diethyl ether.

The isolated polymer was dissolved in 5 mL of a carbonate buffer (pH 9.4), to which 3.0 g (8.4 mmol) of diethylene triamine pentaacetic acid dianhydride (Sigma Aldrich) was added, and the solution stirred. The pH of the solution was monitored by a pH meter. The pH of the solution was increased to 8.0 through the addition of a 5M solution of NaOH. A further 2.0 g (5.6 mmol) of DTPA dianhydride was added and the pH again increased to 8.0 though the addition of a 5M solution of NaOH. The solution was then stirred at RT for 2 h.

The reaction solution was transferred into 4 Millipore centrifugal filter units with a 3000 g mol⁻¹ molecular weight cut off. The solution was centrifuged and the polymer solution washed with distilled water and centrifuged a further five times. The polymer solution was collected and freeze-dried overnight to afford the azide-terminated, DTPA substituted polyacrylamide in a 95 mg yield. GPC analysis indicated that the isolated polymer was low molecular weight (*M*ₙ = 5000 g mol⁻¹ and had a narrow PDI (*M_{w}*/*Mₙ* = 1.20).

### Example 5 - Preparation of trans-cyclooctyl (TCO) terminated, 1,4,7,10-tetraazacylcododecane-1,4,7,10- tetraacetic acid (DOTA) substituted polyacrylamide via thiol-ene click chemistry

Thiol-end DOTA containing polymer was prepared by reversible addition-fragmentation chain transfer (RAFT) polymerization according to Majonis et al. (Analytical Chemistry, 2010. 82:8961-8969). First an N, N-dimethylarylamide and N-acryloxysuccinimide random copolymer by RAFT polymerization was synthesised. This copolymer was reacted with tert-butyl 2-aminoethylcarbamate, followed by deprotection with trifluoracetic acid, generating an amino-containing polymer. This polymer was then reacted with activated DOTA using a published procedure to generate thiol-end DOTA containing polymer.

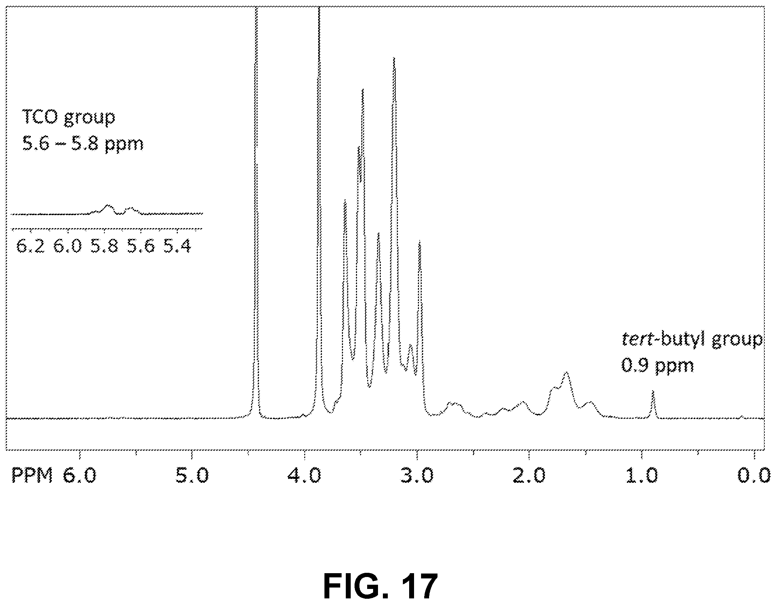

Thiol-terminated 1,4,7,10-tetraazacylcododecane-1,4,7,10- tetraacetic acid (DOTA) substituted polyacrylamide was dissolved in phosphate buffered saline (PBS). Thiol-terminated 1,4,7,10-tetraazacylcododecane-1,4,7,10- tetraacetic acid substituted polyacrylamide can be prepared using methods known in the art, such as those described in Lou et al., 2007, Angew. Chem. Int. Ed., 46:6111. To this solution, TCO-PEG3-maleimide (Click chemistry tools, Scotsdale AZ) was added and the solution stirred at room temperature for 2 h. Polymer was purified by washing through Millipore Amicon Ultra-15 Centrifugal filter units with molecular weight cut-off of 3000 to remove small molecular weight impurities. GPC analysis of the isolated polymer indicated that the isolated polymer has a moderate molecular weight (Mₙ = 11,000 gmol^{- 1}) and a narrow PDI (M_{w}/Mₙ = 1.20) (aqueous GPC). The peak at δ = 0.9 ppm in Figure 17 can be assigned to the protons on the tert-butyl end-group. The broad peak at δ = 1.2 - 2.2 ppm can be assigned to the protons of the methylene groups on the copolymer main chain. The broad peaks at δ = 5.5 - 6.1 ppm are due to protons from the TCO end-group.

### Example 6 - Preparation of azide terminated, 1,4,7,10-tetraazacylcododecane-1,4,7,10-tetraacetic acid (DOTA) substituted polyacrylamide via thiol-ene click chemistry

Azide-end mass tag was prepared by reaction of thiol-end DOTA-containing polymer, prepared as described in the preceding examples, with azido-PEG3-Maleimide at room temperature in PBS buffer for 2 hours. Polymer was purified by washing through Millipore Amicon Ultra-15 Centrifugal filter units with molecular weight cut-off of 3000 to remove small molecular weight impurities.

### Example 7 - Preparation of mass-tagged substrates via copper-free click chemistry

The preparation of DBCO or tetrazine-functionalised substrates (e.g. WGA lectin, antibodies (Abs), phalloidin), through the reaction of primary amines (e.g. from the side chain of a lysine moiety on the WGA) with either DBCO-PEG4-NHS (Sigma Aldrich) or tetrazine-PEG5-NHS (Sigma Aldrich), can be performed at mild pH (6-8) using procedures well known in the art.

The methodology for the complexation of heavy metals onto metal-chelating moieties, such as DTPA and DOTA-substituted polyacrylamide, to form a mass tag is well known in the art.

DBCO-PEG5-NHS or Tetrzine-PEG5-NHS bifunctional linker molecules were used to activate biomolecules with desired number of DBCO or Tetrazine groups, then react the activated biomolecules with azide- or TCO-activated polymer without Cu(I) in aqueous conditions to form a stable DBCO or triazole moiety. In brief, 100 µg of protein was incubated with DBCO-PEG5-NHS at molar equivalent of 10 in PBS buffer for 120 min at room temperature. Then, for biomolecules with larger molecular weight (i.e. Abs and lectins), the unreacted DBCO-PEG5-NHS was removed using the Amicon Ultra-0.5 NMWL 10 kDa Centrifugal Filter (EMD Millipore, Billerica, MA), whereas for small molecule conjugation (e.g. phalloidin) directly proceeded to mass-tagging procedure. A similar procedure was followed for activation of biomolecules with Tetrazine moiety. Following the activation step, the purified DBCO or Tetrazine-functionalized biomolecules were reacted with azide- or TCO- modified metal-chelating polymers. The click reaction was conducted at 37 °C for 60-90 min. The unreacted polymers were removed using the Amicon Ultra-0.5 NMWL 100 kDa Centrifugal Filter (EMD Millipore, Billerica, MA).

For dual-labelled reagent preparation, carrier protein-free purified anti-CD3(UCHT1) were first labelled with Alexa Fluor^{™} 594 at molar equivalents of 1:10, 1:20, and 1:30 using thiol-maleimide chemistry according to the manufacturer's instructions. Then, the purified Alexa Fluor^{™} 594 tagged anti-CD3 mAbs were conjugated with mass tags using the Tetrazine-TCO ligation chemistry. Degree of labeling and concentrations of Ab conjugates were determined photometrically at 280 nm and 594 nm using NanoDrop^{™} 2000/2000c Spectrophotometer (Thermofisher Scientific, Waltham, MA). Then, conjugates were adjusted to concentrations of 0.25 mg/mL using PBS-based Ab stabilizer (Candor Bioscience, Wangen, Germany).

### Example 8 - Staining cells with mass tag-labelled substrates.

Staining of cells for imaging mass cytometry (IMC) can be performed using standard procedures in the art.

### Surface Ab staining of suspension cells

Live cells (2× 10⁶ per tube) were collected from growth media by centrifugation and resuspended in Maxpar^{®} Cell Staining Buffer (Fluidigm, CA, USA). The cells were initially stained with a ¹⁰³Rh-intercalator to a final concentration of 1 µM. After the incubation cells were washed with Maxpar^{®} Cell Staining Buffer and stained with cocktails of mass-tagged Abs mix diluted in Maxpar^{®} Cell Staining Buffer for 30 min at room temperature. Cells were then washed twice in Maxpar^{®} Cell Staining Buffer and stained with Cell-ID^{™} Intercalator-Ir diluted in Maxpar^{®} Fix & Perm Buffer as recommended by the manufacturer (Fluidigm, CA, USA). For WGA staining, cells were incubated with 1µg/ml of ¹⁷⁴Yb-WGA at room temperature for 30 min after the surface Abs staining.

### Staining procedure for adherent cells

HeLa human cervix tumor cell line were seeded in 4-chamber slide (Nunc^{™} Lab-Tek^{™} II CC2^{™}) at 0.25/ml in 1ml growth media per chamber. The cells seat and grow as a monolayer in DMEM, supplemented with 10% FBS (Sigma Aldrich) for 3 days at 37 °C, 5% CO2. The media was aspirated and cells were rinsed in PBS, then stained with surface mass-tagged Abs mix at 1:100 titer in 250 el per chamber in Maxpar^{®} Cell Staining Buffer for 90 min at RT. Excess mass-tagged Abs were washed on the cell surface with PBS, then cells were fixed for 15 min in in 400ul 1.6% formaldehyde/PBS per chamber. After fixation, cells were permeabilized and blocked with 400ul of Perm-S Buffer at RT for 30min and 1% BSA/PBS for 60 min at 37C, respectively. Cells were stained with metal-conjugated Abs to intracellular markers (1:50 in 250ul) in Maxpar^{®} Cell Staining Buffer and phalloidin-¹⁶⁵Ho for overnight at 4°C. Finally, cells were incubated with Cell-ID^{™} Intercalator-Ir at 25 µM (Fluidigm Cat.No. 201192A) for 30 min at room temperature for nuclei staining. The samples were rinsed twice in ddH₂O and air-dried before IMC analysis.

### Staining of tissue sections

Immunohistochemistry (IHC) staining of Frozen and Formalin-Fixed, Paraffin-Embedded (FFPE) tissues with mass-tagged Abs for IMC analysis was performed according to previously published protocol (Chang, et al., 2016. Scientific Reports, 6:36641). Briefly, snap frozen tissue sections from human tonsil were purchased from amsbio LLC (Cambridge, MA). Each slide that contain two sections (cores) were fixed with 4% paraformaldehyde, washed with DPBS, blocked with 3% BSA, and then core one stained with a mix of meta-conjugated Abs and core two stained with dual tagged Abs (metal and fluorophore) for overnight. Slides washed with 0.1% Triton-X in DPBS and DPBS. Core one was stained with 191Ir/193Ir Intercalator. Core two were stained with DAPI solution. Core one washed with ddH₂O, air dried and ablated by IMC. Core two were washed with DPBS covered with mounting media and cover slips, image was taken by Immunofluorescent microscope.

For FFPE tissue sections, before incubating with metal-conjugated Abs, sections were dewaxed, dehydrated, and undergone through heat-induced antigen retrieval step in alkaline solution. After mass-tagged Ab incubation steps, the tissue sections were then stained with WGA-¹⁷⁴Yb conjugates at 1µg/ml and Phalloidin-¹⁶⁵Ho at 75 ng/ml in PBS for 30 min at room temperature. Finally, sections were exposed to Cell-ID^{™} Intercalator-Ir at 25 µM (Fluidigm Cat.No. 201192A) for 30 min at room temperature for nuclei staining. The samples were rinsed twice in distilled water and air-dried before IMC analysis.

### Example 9 - Mean signal intensities detected during IMC of cells stained with labelled substrates prepared by either Maxpar or Azide-DBCO click chemistry

To determine the successful attachment of mass tags to the Ab molecule, ICP-MS was used to measure the number of attached mass tags per Ab. Also, the degree of labeling was optimised by using varied number of DBCO-PEG5-NHS molar equivalents, then monitored the number of mass tags per Ab after reaction and assessed the immune reactivity of the resultant mass-tagged Ab. It was found that the optimal molar equivalent for DBCO-PEG5-NHS to Ab was 10 (~6 DBCO per Ab), which resulted in 3-4 mass tags or 150-200 metal atoms per Ab. A polyclonal Goat anti-Mouse IgG Fc Secondary Abs (GAM) was used as a model Ab molecule for protocol optimization. It was coupled with ¹⁶⁹Tm loaded mass tags using DBCO-Azide reaction. The functionality test result was performed on human Ramos cells and human KG1a cells stained with anti-CD20 (2H7) primary (5 µg/mL) followed by ¹⁶⁹Tm-tagged GAM (1.25 µg/mL), and from the CyTOF analysis result, click chemistry conjugated ¹⁶⁹Tm-GAM was determined to show high sensitivity and low background as compared to Maxpar^{™} labeled ¹⁵⁹Tm-GAM. Then, various monoclonal Abs with IgG and IgM isotypes are also conjugated to mass tags using click chemistry reaction and tested for their functionality in mass cytometry assay. In Figure 18, results from human PBMCs stained with Maxpar^{™} Human PBMC Basic II Phenotyping Panel Kit (Cat. No. 201315) and anti-human CD57 (HCD57)-¹⁷²Yb Ab are shown. Click chemistry conjugated CD4-¹⁴⁵Nd, CD14-¹⁶⁰Gd, and CD16-¹⁶⁵Ho Abs at 1-2 µg/mL and CD57(HC57)-¹⁷²Yb (IgM class) were replaced respective Abs in the basic phenotyping panel to assess the effect of click conjugation in multiplex assay format. The CyTOF analysis result revealed that the new conjugates showed a similar phenotyping capability (Figure 18A and Figure 18B) and superior sensitivity (two to three-fold mean signal increase) and lower background intensity (Figure 18C-E) as compared to commercially available PBMC phenotyping kit. The improved sensitivity of the click chemistry conjugated Abs is mainly attributed from the number of metal atoms attached to each Ab, which is higher than Maxpar^{™} conjugates. In general, the click chemistry conjugation showed the potential to be used as a replacement for low sensitive Maxpar^{™} labeled Abs and generate new Ab based affinity reagents for probing dim markers.

### Example 10 - Mass-tagged WGA conjugates prepared by copper-free click chemistry conjugation of azide-terminated polymer-based mass tags and DBCO-activated biomolecules

Another application area identified for click chemistry is the generation of mass-tagged affinity reagents for glycobiology application. A lectin from *Triticum vulgaris* (Wheat Germ Agglutinin (WGA)) was used as a model molecule for direct conjugation of mass tags using the click chemistry approach. WGA is a lectin that can bind oligosaccharides containing terminal N-acetylglucosamine or chitobiose, structures which are common to many serum and membrane glycoproteins. The receptor sugars to WGA are sialic acid and N-acetylglucosamine residues, which is ubiquitous in plasma membrane of cells. WGA-¹⁷⁴Yb conjugates were tested on suspension assay format to stain plasma membranes on human Ramos, KG1a and CCRF-CEM cells. Ramos, CCRF-CEM, and KG1a cells were stained with CD20-¹⁴⁷Sm, CD34-¹⁴⁸Nd, and CD4-¹⁴⁵Nd Abs in separate tube. Cells were then stained with WGA-¹⁷⁴Yb. In order to evaluate the specificity of WGA staining on cell membrane, Ramos cells were stained with WGA-¹⁷⁴Yb blocked with N-acetylglucosamine (NAG) sugar molecule as a negative control. Part of the stained cells were used for CyTOF analysis and the remaining stained cells are attached on slide to visualize the membrane staining by WGA. Figure 19A shows representative IMC pseudo-color overlay image of human Ramos cells stained with WGA-¹⁷⁴Yb, CD45-¹⁵⁴Sm, and ^{191/193}Ir DNA intercalator. The image shows the WGA (green) stains around the periphery of the cell membrane. Figure 19B and 19C show the staining intensity of mixed Ramos, CCRF-CEM, and KG1a cells analyzed by IMC. The result depicts the specificity of WGA to NAG on plasma membrane of cell. The CyTOF^{®} analysis also confirmed the observation from the IMC result, as expected, and cell size related mean intensity increase was observed.

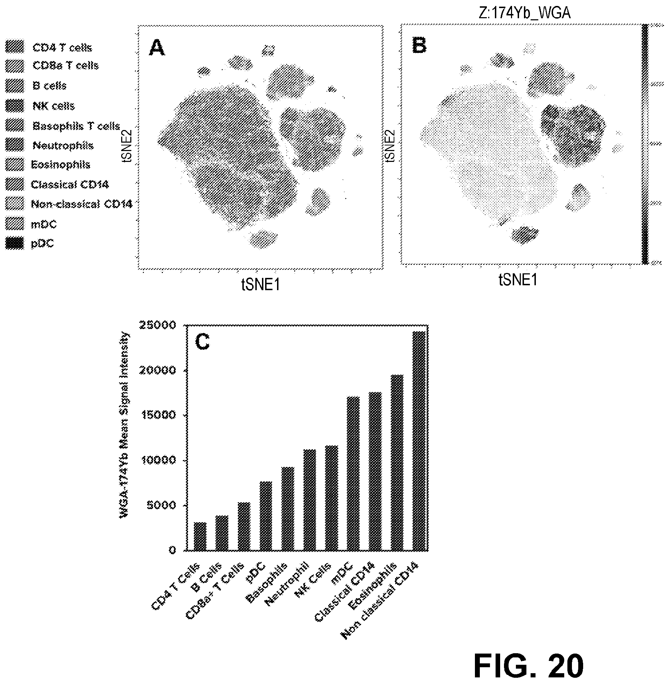

Human whole blood populations using 29 panel Abs against surface markers to identify major immune cell types were also stained, and then cells were stained with ¹⁷⁴Yb-WGA. Figure 20A shows a two-dimensional viSNE representation of human whole blood cell populations showing the locations of major cell populations and Figure 20B shows the distribution of WGA intensity across immune cell subtypes. In agreement with previous report, results showed that the relative WGA staining intensity within specific immune cell subsets were consistent but varied across individuals. The mass-tagged WGA mean intensities for gated cell populations in human whole blood were further quantified for size discrimination, and the observed result (Figure 20C) shows a correlation between cell size and WGA staining mean intensity values for each immune cell subsets, which agrees with the observation by other published data.

IMC images were acquired according to a procedure described in Lou et al., 2007, Angew. Chem. Int. Ed., 46:6111. Briefly, stained and air-dried slides were inserted into the ablation chamber of the Hyperion^{™} Imaging System (Fluidigm), where a pulsed 200 Hz laser is focused to a 1 µm spot and applied over a user-defined area, ablating adjacent spots in 1 µm steps as the slide moves under the laser beam. The plumes of vaporized material are streamed by inert gas with high time fidelity into the inductively coupled plasma ion source for analysis by the mass cytometer. Data processing and visualization were performed using in-house-developed Wolfram Mathematica^{®} (V10.3) algorithms (Chang, et al., 2016. Scientific Reports, 6:36641). Images of each mass channel were reconstructed by plotting the laser shot signals in the order in which they were recorded, line scan by line scan. RGB images were overlaid for desired channel combinations.

For mass cytometry analysis, cells were dispersed in 100 µL of DI water, and an aliquot (10-20 µL) of the standard reference bead stock solution (EQ-4 beads) was added to the cells, then filtered into 5 ml round bottom polystyrene tubes with a 30 µm cell strainer cap to remove possible cell clusters or aggregates. Helios^{®} instrument at Fluidigm Canada (Markham, ON) were used for data acquisition in FCS3.0 file format. The acquired data processed by FlowJo software.

Fluorescent Images were acquired using a Zeiss Axiolmager M2 microscope equipped with both 10x and 20x EC Plan-Neofluar objectives (NA 0.3 and 0.5, respectively) and Zeiss Filter sets. The images were captured with a monochrome Zeiss Axiocam 506 digital camera using Zen Pro software.

### Example 11 - Mass-tagged peptide conjugates prepared by copper-free click chemistry conjugation of azide-terminated polymer-based mass tags and DBCO-activated biomolecules

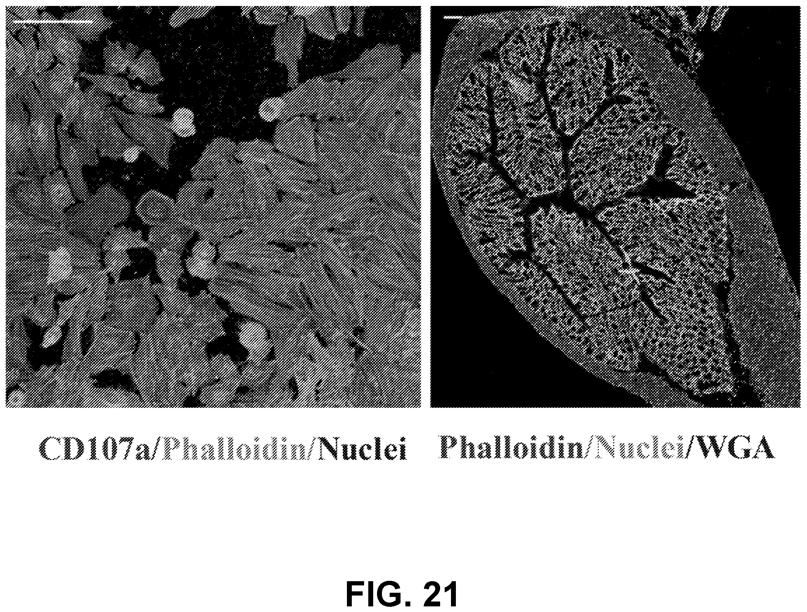

A click chemistry based conjugation strategy was also used to couple small peptide molecules with mass tags. Phalloidin was used as a model peptide molecule for mass cytometry application. Phalloidin is a highly selective bicyclic peptide that is used for staining actin filaments (also known as F-actin) in cells and tissue sections. Developing mass-tagged phalloidin reagent is attractive for visualizing or quantifying actin filaments in formaldehyde-fixed and permeabilized tissue sections, and cells. The mass-tagged phalloidin reagent has been optimized to carry one mass tag per phalloidin molecule that resulted in a bright actin filament staining on IMC images of HeLa cells and FFPE mouse colon sections, which are stained with phalloidin-¹⁶⁵Ho and other mass-tagged Abs (Figure 21).

### Example 12 - Mass-tagged antibody conjugates prepared by copper-free click chemistry conjugation of tetrazine-terminated antibody and TCO-activated polymer-based mass tags

Polymers with TCO-end group and anti-CD3(UCHT1) monoclonal antibody were used as a model system. The antibody was first modified with tetrazine moiety, then coupled with TCO-mass tag to generate mass-tagged antibodies. In order to test the efficiency of this conjugation strategy, azide and career free (LEAF) anti-CD3 Abs were tagged with ¹⁷⁰Er metal using Maxpar, SPAAC, and TCO-Tetrazine conjugation methods. The functionality test result of these three conjugates on human Jurkat and Ramos cells showed comparable sensitivity to and low background signal between the three conjugation chemistries (Figure 22A).

As sodium azide is the most common preservative used for biomolecule storage, it is noteworthy to develop a conjugation strategy that can be performed without the interference of azide molecule. One advantage of the TCO-Tetrazine ligation over the SPAAC conjugation method is its ability to use it to perform conjugation in the presence of azide molecule. Affinity chromatography purified anti-human CD3 Ab containing 0.09% sodium azide and EDTA was used for conjugating with TCO-Tetrazine and Maxpar labeling strategy. From the conjugation experiment, no interference from the azide molecule was observed during the coupling process. The number of mass tags per Ab was similar (3-4 tags per Ab) to previously conjugations. The functionality test on human Jurkat and Ramos cells also showed reproducible result, high positive mean intensity and low background (Figure 22B).

Developing dual-labeled Ab conjugates for cross-platform comparison between fluorescence based systems and mass cytometry has becoming a major interest by various researchers recently. However, the reproducibility of these approaches for different Ab targets could still be a challenge. In this example dual tagged conjugates successfully generated with controlled reaction for fluorophore and Ab tagging using the conventional thiol-maleimide conjugation strategy followed by TCO-tetrazine ligation chemistry for fluorophore and mass-tagging, respectively. One of the challenge that was commonly encountered during the preparation of dual-labelled reagent is the ability to control the balance between the fluorophore and mass-tag brightness when used in fluorescence and mass cytometry based platforms. Three different molar equivalents (10, 20, 30) of AlexaFluor594 dye to the Ab amount were tested, and each yield 4, 5, and 7 dyes per Ab molecule. The click chemistry conjugation also yields approximately 3.5 tags (~ 175 atoms/Ab) per Ab. For testing the functionality of the conjugates, human Jurkat and Ramos cells were stained with the three conjugates. The result revealed that all the click chemistry conjugates showed high positive mean intensity and low background signal compared to the Maxpar control (Figure 22C). One possible application envisaged for dual-labeled conjugates is its potential use for IMC and immunofluorescence systems, either for platform comparison or pre-scanning of tissue sections that needs to be acquired by mass cytometry. Figure 22D and 22E, show representative IMC (Fig. 22D) and IF (Fig. 22E) images of frozen human tonsillar tissue sections labeled with dual-tagged CD3-¹⁷⁰Er-AlexaFluor594 mAb prepared via a two-step (thiol-maleimide conjugation for fluorophore tagging and click chemistry for mass tagging) conjugation method. The result depicts that the distribution of CD3-positive T-cells in IMC image was consistent with what was observed for IF. This result proves the potential of dual tagged Abs for applications that require cross-platform comparison between fluorescence and mass cytometry based platforms and pre-enrichment or rapid scanning using fluorescence microscopy and deep profiling using IMC.

### Example 13 - Mass-tagged antibody conjugates, wherein the mass tag is a metal-binding protein

Metallothionein-1 from rabbit liver (Enzo Life Sciences; ALX-202-072-M001) dissolved in loading buffer is loaded with an appropriate amount of metal ion (Cd or Pt) at 50mM, and washed several times in a 3K cut off spin column with buffer pH 7.5.

Metal-loaded Metallothionein-1 is reacted with Tetrazine-PEG5-NHS ester.

Antibody (e.g. anti-CD20 antibody) is resuspended in buffer pH 7.5 and is reacted with Trans-Cyclooctene-PEG4-NHS ester.

The metal conjugate CD20-metallothionein-1-Cd is used to stain live cells that express the CD20 marker (Ramos) and cells that do not express CD20 (Jurkat).

Cells are stained with CD45 and Intercalator and analyzed by Helios.

### Protocol for conjugation:

1. 1.25 uL of Tetrazine-PEG5-NHS is added to 100 ug of Ab in PBS to a molar equivalent of 10, following by mixing well and incubation at RT for 2 h
2. 2.5 uL of TCO-PEG4-NHS is added to 100 ug metal-binding protein in PBS to a molar equivalent of 20, following by mixing well and incubation at RT for 1 h
3. Ab and metal-binding protein are washed in spin filters; resuspend in PBS and combined in new filter, followed by incubation at 37°C for 1 h.
4. The conjufated Ab-metal-binding protein is then further washed.

## Claims

1. A mass-tagged specific binding pair member (SBP), comprising an SBP and a mass tag, wherein the SBP and the mass tag are joined by a covalent linker, wherein the linker is formed at least in part by a strain-promoted click chemistry reaction product, and wherein the mass tag comprises a polymer comprising a plurality of metal chelating moieties loaded with labeling atoms of the same metal isotope.

2. The mass-tagged SBP of claim 1, wherein the SBP and the mass tag are joined by a linker which comprises the reaction product of a strained cycloalkyne and an azide, for example DBCO and an azide.

3. The mass-tagged SBP of claim 2, wherein the SBP and the mass tag are joined by a linker comprising a triazole, optionally wherein the triazole group in the linker is part of a multi-ring structure.

4. The mass-tagged SBP of claim 3, wherein the multi-ring structure comprises a dibenzocyclooctene group, optionally wherein:
(i) the triazole group is separated from the SBP by the dibenzocyclooctene group; or
(ii) the dibenzocyclooctene group is separated from the SBP by the triazole group.

5. The mass-tagged SBP of claim 1, wherein the SBP and the mass tag are joined by a linker which comprises the reaction product of a strained cycloalkene and a tetrazine, for example TCO and a tetrazine.

6. The mass-tagged SBP of claim 5, wherein the SBP and the mass tag are joined by a linker comprising a pyridazine, optionally wherein the pyridazine group in the linker is part of a multi-ring structure.

7. The mass-tagged SBP of claim 6, wherein the multi-ring structure comprises a cyclooctane group, optionally wherein:
(i) the pyridazine group is separated from the SBP by the cyclooctane group; or
(ii) the cyclooctane group is separated from the SBP by the pyridazine group.

8. The mass-tagged SBP of claim 1, wherein the SBP and the mass tag are joined by a linker which comprises the reaction product of a strained cycloalkyne and a nitrone, for example DBCO and a nitrone.

9. The mass-tagged SBP of claim 8, wherein the SBP and the mass tag are joined by a linker comprising an isoxazole, optionally wherein the isoxazole group in the linker is part of a multi-ring structure.

10. The mass-tagged SBP of claim 3, wherein the multi-ring structure comprises a dibenzocyclooctene group, optionally wherein:
(i) the isoxazole group is separated from the SBP by the dibenzocyclooctene group; or
(ii) the dibenzocyclooctene group is separated from the SBP by the isoxazole group.

11. The mass-tagged SBP of any one of claims 1-10, wherein the linker component comprises at least one spacer, optionally wherein the spacer is a polyethylene glycol (PEG) spacer, a poly(N-vinylpyrolide) (PVP) spacer, a polyglycerol (PG) spacer, poly(N-(2-hydroxylpropyl)methacrylamide) spacer, or a polyoxazoline (POZ, such as polymethyloxazoline, polyethyloxazoline or polypropyloxazoline) or a C5-C20 noncyclic alkyl spacer, optionally wherein the spacer is a PEG spacer having from 3-12 ethylene glycol units.

12. A kit comprising two or more mass-tagged SBPs according to any one of claims 1-10, optionally wherein the kit comprises, at least 3, at least 4, at least 5, at least 6, at least 7, at least 8, at least 9, at least 10, at least 20, at least 30, at least 40, at least 50, or at least 100 of the mass-tagged SBPs according to any one of claims 1-10, and wherein each mass tag comprises a different isotope.

13. A method of making the mass-tagged SBP of any one of claims 1 to 10, comprising the steps of:
(i) providing the SBP and a mass tag comprising a polymer that comprises a plurality of metal chelating moieties loaded with labeling atoms of the same metal isotope; and
(ii) conjugating the SBP to said mass tag wherein at least one step in the conjugation comprises a strain-promoted click chemistry reaction.

## Patentansprüche

1. Massen-markiertes spezifisches Bindungspaarelement (SBP), umfassend ein SBP und einen Massenmarker, wobei das SBP und der Massenmarker durch einen kovalenten Linker verbunden sind, wobei der Linker zumindest teilweise durch ein spannungsgetriebenes Click-Chemie-Reaktionsprodukt gebildet ist, und wobei der Massenmarker ein Polymer umfasst, das eine Mehrzahl von Metallchelatiernden Einheiten umfasst, die mit Markierungsatomen desselben Metallisotops beladen sind.

2. Massen-markiertes SBP nach Anspruch 1, wobei das SBP und der Massenmarker durch einen Linker verbunden sind, der das Reaktionsprodukt eines gespannten Cycloalkins und eines Azids, beispielsweise DBCO und eines Azids, umfasst.

3. Massen-markiertes SBP nach Anspruch 2, wobei das SBP und der Massenmarker durch einen Linker verbunden sind, der ein Triazol umfasst, gegebenenfalls wobei die Triazolgruppe in dem Linker Teil einer Mehrringstruktur ist.

4. Massen-markiertes SBP nach Anspruch 3, wobei die Mehrringstruktur eine Dibenzocyclooctengruppe umfasst, gegebenenfalls wobei:
(i) die Triazolgruppe durch die Dibenzocyclooctengruppe von dem SBP getrennt ist; oder
(ii) die Dibenzocyclooctengruppe durch die Triazolgruppe von dem SBP getrennt ist.

5. Massen-markiertes SBP nach Anspruch 1, wobei das SBP und der Massenmarker durch einen Linker verbunden sind, der das Reaktionsprodukt eines gespannten Cycloalkens und eines Tetrazins, beispielsweise TCO und eines Tetrazins, umfasst.

6. Massen-markiertes SBP nach Anspruch 5, wobei das SBP und der Massenmarker durch einen Linker verbunden sind, der ein Pyridazin umfasst, gegebenenfalls wobei die Pyridazin-Gruppe in dem Linker Teil einer Mehrringstruktur ist.

7. Massen-markiertes SBP nach Anspruch 6, wobei die Mehrringstruktur eine Cyclooctangruppe umfasst, gegebenenfalls wobei:
(i) die Pyridazingruppe durch die Cyclooctangruppe von dem SBP getrennt ist; oder
(ii) die Cyclooctangrupe durch die Pyridazingruppe von dem SBP getrennt ist.

8. Massen-markiertes SBP nach Anspruch 1, wobei das SBP und der Massenmarker durch einen Linker verbunden sind, der das Reaktionsprodukt eines gespannten Cycloalkins und eines Nitrons umfasst, beispielsweise DBCO und eines Nitrons.

9. Massen-markiertes SBP nach Anspruch 8, wobei das SBP und der Massenmarker durch einen Linker verbunden sind, der ein Isoxazol umfasst, gegebenenfalls wobei die Isoxazolgruppe in dem Linker Teil einer Mehrringstruktur ist.

10. Massen-markiertes SBP nach Anspruch 3, wobei die Mehrringstruktur eine Dibenzocyclooctengruppe umfasst, gegebenenfalls wobei:
(i) die Isoxazolgruppe durch die Dibenzocyclooctengruppe von dem SBP getrennt ist; oder
(ii) die Dibenzocyclooctengruppe durch die Isoxazolgruppe von dem SBP getrennt ist.

11. Massenmarkiertes SBP nach einem der Ansprüche 1 bis 10, wobei die Linkerkomponente mindestens einen Spacer umfasst, gegebenenfalls wobei der Spacer ein Polyethylenglykol (PEG)-Spacer, ein Poly(N-vinylpyrolid) (PVP)-Spacer, ein Polyglycerin (PG)-Spacer, ein Poly(N-(2-hydroxylpropyl)methacrylamid)-Spacer oder ein Polyoxazolin (POZ, wie Polymethyloxazolin, Polyethyloxazolin oder Polypropyloxazolin) oder ein nicht-cyclischer C5-C20-Alkylspacer ist, gegebenenfalls wobei der Spacer ein PEG-Spacer mit 3 bis 12 Ethylenglykoleinheiten ist.

12. Kit, umfassend zwei oder mehr massenmarkierte SBPs nach einem der Ansprüche 1 bis 10, wobei das Kit gegebenenfalls mindestens 3, mindestens 4, mindestens 5, mindestens 6, mindestens 7, mindestens 8, mindestens 9, mindestens 10, mindestens 20, mindestens 30, mindestens 40, mindestens 50 oder mindestens 100 der massenmarkierten SBPs nach einem der Ansprüche 1 bis 10 umfasst, und wobei jeder Massenmarker ein unterschiedliches Isotop umfasst.

13. Verfahren zur Herstellung des massenmarkierten SBP nach einem der Ansprüche 1 bis 10, umfassend die Schritte:
(i) Bereitstellen des SBP und eines Massenmarkers, der ein Polymer umfasst, das eine Mehrzahl von Metallchelatierenden Einheiten umfasst, die mit Markierungsatomen desselben Metallisotops beladen sind; und
(ii) Konjugieren des SBP mit dem Massenmarker, wobei mindestens ein Schritt bei der Konjugation eine spannungsgetriebene Click-Chemie-Reaktion umfasst.

## Revendications

1. Elément de paire de liaison spécifique marqué en masse (SBP), comprenant un SBP et un marqueur de masse, dans lequel le SBP et le marqueur de masse sont joints par un lieur covalent, dans lequel le lieur est formé au moins en partie par un produit de réaction de chimie clic favorisée par contrainte, et dans lequel le marqueur de masse comprend un polymère comprenant une pluralité de groupements chélatants de métaux chargés avec des atomes de marquage du même isotope métallique.

2. SBP marqué en masse selon la revendication 1, dans lequel le SBP et le marqueur en masse sont joints par un lieur qui comprend le produit de réaction d'un cycloalcyne contraint et d'un azoture, par exemple DBCO et un azoture.

3. SBP marqué en masse selon la revendication 2, dans lequel le SBP et le marqueur en masse sont joints par un lieur comprenant un triazole, éventuellement dans lequel le groupe triazole dans le lieur fait partie d'une structure multicyclique.

4. SBP marqué en masse selon la revendication 3, dans lequel la structure multicyclique comprend un groupe dibenzocyclooctène, éventuellement dans lequel :
(i) le groupe triazole est séparé du SBP par le groupe dibenzocyclooctène ; ou
(ii) le groupe dibenzocyclooctène est séparé du SBP par le groupe triazole.

5. SBP marqué en masse selon la revendication 1, dans lequel le SBP et le marqueur en masse sont joints par un lieur qui comprend le produit de réaction d'un cycloalcène contraint et d'une tétrazine, par exemple TCO et une tétrazine.

6. SBP marqué en masse selon la revendication 5, dans lequel le SBP et le marqueur en masse sont joints par un lieur comprenant une pyridazine, éventuellement dans lequel le groupe pyridazine dans le lieur fait partie d'une structure multicyclique.

7. SBP marqué en masse selon la revendication 6, dans lequel la structure multicyclique comprend un groupe cyclooctane, éventuellement dans lequel :
(i) le groupe pyridazine est séparé du SBP par le groupe cyclooctane ; ou
(ii) le groupe cyclooctane est séparé du SBP par le groupe pyridazine.

8. SBP marqué en masse selon la revendication 1, dans lequel le SBP et le marqueur en masse sont joints par un lieur qui comprend le produit de réaction d'un cycloalcyne contraint et d'une nitrone, par exemple DBCO et une nitrone.

9. SBP marqué en masse selon la revendication 8, dans lequel le SBP et le marqueur en masse sont joints par un lieur comprenant un isoxazole, éventuellement dans lequel le groupe isoxazole dans le lieur fait partie d'une structure multicyclique.

10. SBP marqué en masse selon la revendication 3, dans lequel la structure multicyclique comprend un groupe dibenzocyclooctène, éventuellement dans lequel :
(i) le groupe isoxazole est séparé du SBP par le groupe dibenzocyclooctène ; ou
(ii) le groupe dibenzocyclooctène est séparé du SBP par le groupe isoxazole.

11. SBP marqué en masse selon l'une quelconque des revendications 1 à 10, dans lequel le composant lieur comprend au moins un espaceur, éventuellement dans lequel l'espaceur est un espaceur de polyéthylène glycol (PEG), un espaceur de poly(N-vinylpyrolide) (PVP), un espaceur de polyglycérol (PG), un espaceur de poly(N-(2-hydroxylpropyl)méthacrylamide), ou une polyoxazoline (POZ, telle qu'une polyméthyloxazoline, polyéthyloxazoline ou polypropyloxazoline) ou un espaceur alkyle en C5-C20 non cyclique, éventuellement dans lequel l'espaceur est un espaceur PEG ayant de 3 à 12 motifs d'éthylène glycol.

12. Kit comprenant deux SBP marqués en masse ou plus selon l'une quelconque des revendications 1 à 10, éventuellement dans lequel le kit comprend au moins 3, au moins 4, au moins 5, au moins 6, au moins 7, au moins 8, au moins 9, au moins 10, au moins 20, au moins 30, au moins 40, au moins 50 ou au moins 100 des SBP marqués en masse selon l'une quelconque des revendications 1 à 10, et dans lequel chaque marqueur de masse comprend un isotope différent.

13. Procédé de fabrication du SBP marqué en masse selon l'une quelconque des revendications 1 à 10, comprenant les étapes de :
(i) fourniture du SBP et d'un marqueur de masse comprenant un polymère qui comprend une pluralité de groupements chélatants de métaux chargés avec des atomes de marquage du même isotope métallique ; et
(ii) conjugaison du SBP audit marqueur de masse, dans lequel au moins une étape dans la conjugaison comprend une réaction de chimie clic favorisée par contrainte.
